# EUROPEAN PATENT APPLICATION

(11) **EP 2 388 265 A1**
(43) Date of publication of application: **23.11.2011**
(21) Application number: 11177505.2
(22) Date of filing: 21.02.2003
(51) Int. Cl.: C07H 21/04, C07K 14/00, C12N 15/63, C12N 15/85, C12N 1/21, C12N 1/19, C12N 15/00, A61K 38/00, G01N 33/53, C12Q 1/68

(54) **Compositions and methods for the treatment of immune related diseases**

(30) Priority: 22.02.2002 US 359461 P
(62) Divisional of application: 03711174.7
(71) Applicant: Genentech, Inc., South San Francisco CA 94080-4990 (US)
(72) Inventor: Bodary, Sarah C., Menlo Park, CA 94025-5914 (US); Clark, Hilary, San Francisco, CA 94131 (US); Hunte, Brisdell, San Francisco, CA 94110 (US); Jackman, Janet K., Half Moon Bay, CA 94019 (US); Schoenfeld, Jill R., Ashland, OR 97520 (US); Williams, P. Mickey, Emerald Hills, CA 94062 (US); Wood, William, Cupertino, CA 95014 (US); Wu, Thomas D., San Francisco, CA 94110-5720 (US)
(74) Representative: Woolley, Lindsey Claire

(57) **Abstract**

The present invention relates to compositions containing novel proteins and methods of using those compositions for the diagnosis and treatment of immune related diseases.

## Description

### Field of the Invention

The present invention relates to compositions and methods useful for the diagnosis and treatment of immune related diseases.

### Background of the Invention

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive-stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

Many immune related diseases are known and have been extensively studied. Such diseases include immune-mediated inflammatory diseases, non-immune-mediated inflammatory diseases, infectious diseases, immunodeficiency diseases, neoplasia, etc.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, *etc.* The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, i.e., lymphokines, which play a central role in the activation of B cells, cytotoxic T cells and a variety of other cells which participate in the immune response.

Immune related diseases could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

CD4+ T cells are known to be important regulators of inflammation. Herein, CD4+ T cells were activated and the profile of genes differentially expressed upon activation was analyzed. As such, the activation specific genes may be potential therapeutic targets. *In vivo* co-stimulation is necessary for a productive immune proliferative response. The list of costimulatory molecules is quite extensive and it is still unclear just which co-stimulatory molecules play critical roles in different types and stages of inflammation. In this application the focus is on genes which are specifically upregulated by stimulation with ICAM, anti-CD28 or ICAM/anti-CD28 in combination and may be useful in targeting inflammatory processes which are associated with these different molecules.

### Summary of the Invention

### A. Embodiments

The present invention concerns compositions and methods useful for the diagnosis and treatment of immune related disease in mammals, including humans. The present invention is based on the identification of proteins (including agonist and antagonist antibodies) which are a result of stimulation of the immune response in mammals. Immune related diseases can be treated by suppressing or enhancing the immune response. Molecules that enhance the immune response stimulate or potentiate the immune response to an antigen. Molecules which stimulate the immune response can be used therapeutically where enhancement of the immune response would be beneficial. Alternatively, molecules that suppress the immune response attenuate or reduce the immune response to an antigen (*e.g*., neutralizing antibodies) can be used therapeutically where attenuation of the immune response would be beneficial (*e.g*., inflammation). Accordingly, the PRO polypeptides, agonists and antagonists thereof are also useful to prepare medicines and medicaments for the treatment of immune-related and inflammatory diseases. In a specific aspect, such medicines and medicaments comprise a therapeutically effective amount of a PRO polypeptide, agonist or antagonist thereof with a pharmaceutically acceptable carrier. Preferably, the admixture is sterile.

In a further embodiment, the invention concerns a method of identifying agonists or antagonists to a PRO polypeptide which comprises contacting the PRO polypeptide with a candidate molecule and monitoring a biological activity mediated by said PRO polypeptide. Preferably, the PRO polypeptide is a native sequence PRO polypeptide. In a specific aspect, the PRO agonist or antagonist is an anti-PRO antibody.

In another embodiment, the invention concerns a composition of matter comprising a PRO polypeptide or an agonist or antagonist antibody which binds the polypeptide in admixture with a carrier or excipient. In one aspect, the composition comprises a therapeutically effective amount of the polypeptide or antibody. In another aspect, when the composition comprises an immune stimulating molecule, the composition is useful for: (a) increasing infiltration of inflammatory cells into a tissue of a mammal in need thereof, (b) stimulating or enhancing an immune response in a mammal in need thereof, (c) increasing the proliferation of T-lymphocytes in a mammal in need thereof in response to an antigen, (d) stimulating the activity of T-lymphocytes or (e) increasing the vascular permeability. In a further aspect, when the composition comprises an immune inhibiting molecule, the composition is useful for: (a) decreasing infiltration of inflammatory cells into a tissue of a mammal in need thereof, (b) inhibiting or reducing an immune response in a mammal in need thereof, (c) decreasing the activity of T-lymphocytes or (d) decreasing the proliferation of T-lymphocytes in a mammal in need thereof in response to an antigen. In another aspect, the composition comprises a further active ingredient, which may, for example, be a further antibody or a cytotoxic or chemotherapeutic agent. Preferably, the composition is sterile.

In another embodiment, the invention concerns a method of treating an immune related disorder in a mammal in need thereof, comprising administering to the mammal an effective amount of a PRO polypeptide, an agonist thereof, or an antagonist thereto. In a preferred aspect, the immune related disorder is selected from the group consisting of: systemic lupus erythematosis, rheumatoid arthritis, osteoarthritis, juvenile chronic arthritis, spondyloarthropathies, systemic sclerosis, idiopathic inflammatory myopathies, Sjögren's syndrome, systemic vasculitis, sarcoidosis, autoimmune hemolytic anemia, autoimmune thrombocytopenia, thyroiditis, diabetes mellitus, immune-mediated renal disease, demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious, autoimmune chronic active hepatitis, primary biliary cirrhosis, granulomatous hepatitis, and sclerosing cholangitis, inflammatory bowel disease, gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

In another embodiment, the invention provides an antibody which specifically binds to any of the above or below described polypeptides. Optionally, the antibody is a monoclonal antibody, humanized antibody, antibody fragment or single-chain antibody. In one aspect, the present invention concerns an isolated antibody which binds a PRO polypeptide. In another aspect, the antibody mimics the activity of a PRO polypeptide (an agonist antibody) or conversely the antibody inhibits or neutralizes the activity of a PRO polypeptide (an antagonist antibody). In another aspect, the antibody is a monoclonal antibody, which preferably has nonhuman complementarity determining region (CDR) residues and human framework region (FR) residues. The antibody may be labeled and may be immobilized on a solid support. In a further aspect, the antibody is an antibody fragment, a monoclonal antibody, a single-chain antibody, or an anti-idiotypic antibody.

In yet another embodiment, the present invention provides a composition comprising an anti-PRO antibody in admixture with a pharmaceutically acceptable carrier. In one aspect, the composition comprises a therapeutically effective amount of the antibody. Preferably, the composition is sterile. The composition may be administered in the form of a liquid pharmaceutical formulation, which may be preserved to achieve extended storage stability. Alternatively, the antibody is a monoclonal antibody, an antibody fragment, a humanized antibody, or a single-chain antibody.

In a further embodiment, the invention concerns an article of manufacture, comprising:
(a) a composition of matter comprising a PRO polypeptide or agonist or antagonist thereof;
(b) a container containing said composition; and
(c) a label affixed to said container, or a package insert included in said container referring to the use of said PRO polypeptide or agonist or antagonist thereof in the treatment of an immune related disease. The composition may comprise a therapeutically effective amount of the PRO polypeptide or the agonist or antagonist thereof.

In yet another embodiment, the present invention concerns a method of diagnosing an immune related disease in a mammal, comprising detecting the level of expression of a gene encoding a PRO polypeptide (a) in a test sample of tissue cells obtained from the mammal, and (b) in a control sample of known normal tissue cells of the same cell type, wherein a higher or lower expression level in the test sample as compared to the control sample indicates the presence of immune related disease in the mammal from which the test tissue cells were obtained.

In another embodiment, the present invention concerns a method of diagnosing an immune disease in a mammal, comprising (a) contacting an anti-PRO antibody with a test sample of tissue cells obtained from the mammal, and (b) detecting the formation of a complex between the antibody and a PRO polypeptide, in the test sample; wherein the formation of said complex is indicative of the presence or absence of said disease. The detection may be qualitative or quantitative, and may be performed in comparison with monitoring the complex formation in a control sample of known normal tissue cells of the same cell type. A larger quantity of complexes formed in the test sample indicates the presence or absence of an immune disease in the mammal from which the test tissue cells were obtained. The antibody preferably carries a detectable label. Complex formation can be monitored, for example, by light microscopy, flow cytometry, fluorimetry, or other techniques known in the art. The test sample is usually obtained from an individual suspected of having a deficiency or abnormality of the immune system.

In another embodiment, the invention provides a method for determining the presence of a PRO polypeptide in a sample comprising exposing a test sample of cells suspected of containing the PRO polypeptide to an anti-PRO antibody and determining the binding of said antibody to said cell sample. In a specific aspect, the sample comprises a cell suspected of containing the PRO polypeptide and the antibody binds to the cell. The antibody is preferably detectably labeled and/or bound to a solid support.

In another embodiment, the present invention concerns an immune-related disease diagnostic kit, comprising an anti-PRO antibody and a carrier in suitable packaging. The kit preferably contains instructions for using the antibody to detect the presence of the PRO polypeptide. Preferably the carrier is pharmaceutically acceptable.

In another embodiment, the present invention concerns a diagnostic kit, containing an anti-PRO antibody in suitable packaging. The kit preferably contains instructions for using the antibody to detect the PRO polypeptide.

In another embodiment, the invention provides a method of diagnosing an immune-related disease in a mammal which comprises detecting the presence or absence or a PRO polypeptide in a test sample of tissue cells obtained from said mammal, wherein the presence or absence of the PRO polypeptide in said test sample is indicative of the presence of an immune-related disease in said mammal.

In another embodiment, the present invention concerns a method for identifying an agonist of a PRO polypeptide comprising:
(a) contacting cells and a test compound to be screened under conditions suitable for the induction of a cellular response normally induced by a PRO polypeptide; and
(b) determining the induction of said cellular response to determine if the test compound is an effective agonist, wherein the induction of said cellular response is indicative of said test compound being an effective agonist.

In another embodiment, the invention concerns a method for identifying a compound capable of inhibiting the activity of a PRO polypeptide comprising contacting a candidate compound with a PRO polypeptide under conditions and for a time sufficient to allow these two components to interact and determining whether the activity of the PRO polypeptide is inhibited. In a specific aspect, either the candidate compound or the PRO polypeptide is immobilized on a solid support. In another aspect, the non-immobilized component carries a detectable label. In a preferred aspect, this method comprises the steps of:
(a) contacting cells and a test compound to be screened in the presence of a PRO polypeptide under conditions suitable for the induction of a cellular response normally induced by a PRO polypeptide; and
(b) determining the induction of said cellular response to determine if the test compound is an effective antagonist.

In another embodiment, the invention provides a method for identifying a compound that inhibits the expression of a PRO polypeptide in cells that normally express the polypeptide, wherein the method comprises contacting the cells with a test compound and determining whether the expression of the PRO polypeptide is inhibited. In a preferred aspect, this method comprises the steps of:
(a) contacting cells and a test compound to be screened under conditions suitable for allowing expression of the PRO polypeptide; and
(b) determining the inhibition of expression of said polypeptide.

In yet another embodiment, the present invention concerns a method for treating an immune-related disorder in a mammal that suffers therefrom comprising administering to the mammal a nucleic acid molecule that codes for either (a) a PRO polypeptide, (b) an agonist of a PRO polypeptide or (c) an antagonist of a PRO polypeptide, wherein said agonist or antagonist may be an anti-PRO antibody. In a preferred embodiment, the mammal is human. In another preferred embodiment, the nucleic acid is administered via *ex vivo* gene therapy. In a further preferred embodiment, the nucleic acid is comprised within a vector, more preferably an adenoviral, adeno-associated viral, lentiviral or retroviral vector.

In yet another aspect, the invention provides a recombinant viral particle comprising a viral vector consisting essentially of a promoter, nucleic acid encoding (a) a PRO polypeptide, (b) an agonist polypeptide of a PRO polypeptide, or (c) an antagonist polypeptide of a PRO polypeptide, and a signal sequence for cellular secretion of the polypeptide, wherein the viral vector is in association with viral structural proteins. Preferably, the signal sequence is from a mammal, such as from a native PRO polypeptide.

In a still further embodiment, the invention concerns an *ex vivo* producer cell comprising a nucleic acid construct that expresses retroviral structural proteins and also comprises a retroviral vector consisting essentially of a promoter, nucleic acid encoding (a) a PRO polypeptide, (b) an agonist polypeptide of a PRO polypeptide or (c) an antagonist polypeptide of a PRO polypeptide, and a signal sequence for cellular secretion of the polypeptide, wherein said producer cell packages the retroviral vector in association with the structural proteins to produce recombinant retroviral particles.

In a still further embodiment, the invention provides a method of increasing the activity of T-lymphocytes in a mammal comprising administering to said mammal (a) a PRO polypeptide, (b) an agonist of a PRO polypeptide, or (c) an antagonist of a PRO polypeptide, wherein the activity of T-lymphocytes in the mammal is increased.

In a still further embodiment, the invention provides a method of decreasing the activity of T-lymphocytes in a mammal comprising administering to said mammal (a) a PRO polypeptide, (b) an agonist of a PRO polypeptide, or (c) an antagonist of a PRO polypeptide, wherein the activity of T-lymphocytes in the mammal is decreased.

In a still further embodiment, the invention provides a method of increasing the proliferation of T-lymphocytes in a mammal comprising administering to said mammal (a) a PRO polypeptide, (b) an agonist of a PRO polypeptide, or (c) an antagonist of a PRO polypeptide, wherein the proliferation of T-lymphocytes in the mammal is increased.

In a still further embodiment, the invention provides a method of decreasing the proliferation of T-lymphocytes in a mammal comprising administering to said mammal (a) a PRO polypeptide, (b) an agonist of a PRO polypeptide, or (c) an antagonist of a PRO polypeptide, wherein the proliferation of T-lymphocytes in the mammal is decreased.

### B. Additional Embodiments

In other embodiments of the present invention, the invention provides vectors comprising DNA encoding any of the herein described polypeptides. Host cell comprising any such vector are also provided. By way of example, the host cells may be CHO cells, E. *coli*, or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

In other embodiments, the invention provides an isolated nucleic acid molecule comprising a nucleotide sequence that encodes a PRO polypeptide.

In one aspect, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs deposited with the ATCC as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides isolated PRO polypeptide encoded by any of the isolated nucleic acid sequences herein above identified.

In a certain aspect, the invention concerns an isolated PRO polypeptide, comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs deposited with the ATCC as disclosed herein.

In a specific aspect, the invention provides an isolated PRO polypeptide without the N-terminal signal sequence and/or the initiating methionine and is encoded by a nucleotide sequence that encodes such an amino acid sequence as herein before described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist or antagonist thereof as herein before described, or an anti-PRO antibody, for the preparation of a medicament useful in the treatment of a condition which is responsive to the PRO polypeptide, an agonist or antagonist thereof or an anti-PRO antibody.

### BRIEF DESCRIPTION OF THE DRAWINGS

Figure 1 shows a nucleotide sequence (SEQ ID NO:1) of a native sequence PR069457 cDNA, wherein SEQ ID NO:1 is a clone designated herein as "DNA287163".
Figure 2 shows the amino acid sequence (SEQ ID NO:2) derived from the coding sequence of SEQ ID NO:1 shown in Figure 1.
Figure 3 shows a nucleotide sequence (SEQ ID NO:3) of a native sequence PR069458 cDNA, wherein SEQ ID NO:3 is a clone designated herein as "DNA287164".
Figure 4 shows the amino acid sequence (SEQ ID NO:4) derived from the coding sequence of SEQ ID NO:3 shown in Figure 3.
Figure 5 shows a nucleotide sequence (SEQ ID NO:5) of a native sequence PR052268 cDNA, wherein SEQ ID NO:5 is a clone designated herein as "DNA287165".
Figure 6 shows the amino acid sequence (SEQ ID NO:6) derived from the coding sequence of SEQ ID NO:5 shown in Figure 5.
Figure 7 shows a nucleotide sequence (SEQ ID NO:7) of a native sequence PR069459 cDNA, wherein SEQ ID NO:7 is a clone designated herein as "DNA287166".
Figure 8 shows the amino acid sequence (SEQ ID NO:8) derived from the coding sequence of SEQ ID NO:7 shown in Figure 7.
Figure 9 shows a nucleotide sequence (SEQ ID NO:9) of a native sequence PR062927 cDNA, wherein SEQ ID NO:9 is a clone designated herein as "DNA275240".
Figure 10 shows the amino acid sequence (SEQ ID NO:10) derived from the coding sequence of SEQ ID NO:9 shown in Figure 9.
Figure 11 shows a nucleotide sequence (SEQ ID NO:11) of a native sequence PR059136 cDNA, wherein SEQ ID NO:11 is a clone designated herein as "DNA287167".
Figure 12 shows the amino acid sequence (SEQ ID NO:12) derived from the coding sequence of SEQ ID NO:11 shown in Figure 11.
Figure 13 shows a nucleotide sequence (SEQ ID NO:13) of a native sequence PR037121 cDNA, wherein SEQ ID NO:13 is a clone designated herein as "DNA226658".
Figure 14 shows the amino acid sequence (SEQ ID NO:14) derived from the coding sequence of SEQ ID NO:14 shown in Figure 14.
Figure 15 shows a nucleotide sequence (SEQ ID NO:15) of a native sequence PR069460 cDNA, wherein SEQ ID NO:15 is a clone designated herein as "DNA287168".
Figure 16 shows the amino acid sequence (SEQ ID NO:16) derived from the coding sequence of SEQ ID NO:15 shown in Figure 15.
Figure 17 shows a nucleotide sequence (SEQ ID NO:17) of a native sequence PR060475 cDNA, wherein SEQ ID NO:17 is a clone designated herein as "DNA272213".
Figure 18 shows the amino acid sequence (SEQ ID NO:18) derived from the coding sequence of SEQ ID NO:17 shown in Figure 17.
Figure 19 shows a nucleotide sequence (SEQ ID NO:19) of a native sequence PR034451 cDNA, wherein SEQ ID NO:19 is a clone designated herein as "DNA218655".
Figure 20 shows the amino acid sequence (SEQ ID NO:20) derived from the coding sequence of SEQ ID NO: 19 shown in Figure 19.
Figure 21 shows a nucleotide sequence (SEQ ID NO:21) of a native sequence PR038070 cDNA, wherein SEQ ID NO:21 is a clone designated herein as "DNA227607".
Figure 22 shows the amino acid sequence (SEQ ID NO:22) derived from the coding sequence of SEQ ID NO:21 shown in Figure 21.
Figure 23 shows a nucleotide sequence (SEQ ID NO:23) of a native sequence PR023756 cDNA, wherein SEQ ID NO:23 is a clone designated herein as "DNA194378".
Figure 24 shows the amino acid sequence (SEQ ID NO:24) derived from the coding sequence of SEQ ID NO:23 shown in Figure 23.
Figure 25 shows a nucleotide sequence (SEQ ID NO:25) of a native sequence PRO10404 cDNA, wherein SEQ ID NO:25 is a clone designated herein as "DNA287169".
Figure 26 shows the amino acid sequence (SEQ ID NO:26) derived from the coding sequence of SEQ ID NO:25 shown in Figure 25.
Figure 27 shows a nucleotide sequence (SEQ ID NO:27) of a native sequence PR069461 cDNA, wherein SEQ ID NO:27 is a clone designated herein as "DNA288240".
Figure 28 shows the amino acid sequence (SEQ ID NO:28) derived from the coding sequence of SEQ ID NO:27 shown in Figure 27.
Figure 29 shows a nucleotide sequence (SEQ ID NO:29) of a native sequence PR070006 cDNA, wherein SEQ ID NO:29 is a clone designated herein as "DNA288241".
Figure 30 shows the amino acid sequence (SEQ ID NO:30) derived from the coding sequence of SEQ ID NO:29 shown in Figure 29.
Figure 31 shows a nucleotide sequence (SEQ ID NO:31) of a native sequence PR069462 cDNA, wherein SEQ ID NO:31 is a clone designated herein as "DNA287171".
Figure 32 shows the amino acid sequence (SEQ ID NO:32) derived from the coding sequence of SEQ ID NO:31 shown in Figure 31.
Figure 33 shows a nucleotide sequence (SEQ ID NO:33) of a native sequence PR02081 cDNA, wherein SEQ ID NO:33 is a clone designated herein as "DNA287620".
Figure 34 shows the amino acid sequence (SEQ ID NO:34) derived from the coding sequence of SEQ ID NO:33 shown in Figure 33.
Figure 35A-B shows a nucleotide sequence (SEQ ID NO:35A-B) of a native sequence PR070007 cDNA, wherein SEQ ID NO:35A-B is a clone designated herein as "DNA288242".
Figure 36 shows the amino acid sequence (SEQ ID NO:36) derived from the coding sequence of SEQ ID NO:35A-B shown in Figure 35A-B.
Figure 37 shows a nucleotide sequence (SEQ ID NO:37) of a native sequence PR069463 cDNA, wherein SEQ ID NO:37 is a clone designated herein as "DNA287173".
Figure 38 shows the amino acid sequence (SEQ ID NO:38) derived from the coding sequence of SEQ ID NO:37 shown in Figure 37.
Figure 39 shows a nucleotide sequence (SEQ ID NO:39) of a native sequence PR062908 cDNA, wherein SEQ ID NO:39 is a clone designated herein as "DNA275214".
Figure 40 shows the amino acid sequence (SEQ ID NO:40) derived from the coding sequence of SEQ ID NO:39 shown in Figure 39.
Figure 41 shows a nucleotide sequence (SEQ ID NO:41) of a native sequence PR069464 cDNA, wherein SEQ ID NO:41 is a clone designated herein as "DNA287174".
Figure 42 shows the amino acid sequence (SEQ ID NO:42) derived from the coding sequence of SEQ ID NO:41 shown in Figure 41
Figure 43 shows a nucleotide sequence (SEQ ID NO:43) of a native sequence PR052804 cDNA, wherein SEQ ID NO:43 is a clone designated herein as "DNA287175".
Figure 44 shows the amino acid sequence (SEQ ID NO:44) derived from the coding sequence of SEQ ID NO:43 shown in Figure 43.
Figure 45 shows a nucleotide sequence (SEQ ID NO:45) of a native sequence PRO60438 cDNA, wherein SEQ ID NO:45 is a clone designated herein as "DNA272171".
Figure 46 shows the amino acid sequence (SEQ ID NO:46) derived from the coding sequence of SEQ ID NO:45 shown in Figure 45.
Figure 47 shows a nucleotide sequence (SEQ ID NO:47) of a native sequence PR069465 cDNA, wherein SEQ ID NO:47 is a clone designated herein as "DNA287176".
Figure 48 shows the amino acid sequence (SEQ ID NO:48) derived from the coding sequence of SEQ ID NO:47 shown in Figure 47.
Figure 49 shows a nucleotide sequence (SEQ ID NO:49) of a native sequence PR037421 cDNA, wherein SEQ ID NO:49 is a clone designated herein as "DNA226958".
Figure 50 shows the amino acid sequence (SEQ ID NO:50) derived from the coding sequence of SEQ ID NO:49 shown in Figure 49.
Figure 51 shows a nucleotide sequence (SEQ ID NO:51) of a native sequence PR037596 cDNA, wherein SEQ ID NO:51 is a clone designated herein as "DNA227133".
Figure 52 shows the amino acid sequence (SEQ ID NO:52) derived from the coding sequence of SEQ ID NO:51 shown in Figure 51.
Figure 53 shows a nucleotide sequence (SEQ ID NO:53) of a native sequence PR036124 cDNA, wherein SEQ ID NO:53 is a clone designated herein as "DNA225661".
Figure 54 shows the amino acid sequence (SEQ ID NO:54) derived from the coding sequence of SEQ ID NO:53 shown in Figure 53.
Figure 55 shows a nucleotide sequence (SEQ ID NO:55) of a native sequence PR069466 cDNA, wherein SEQ ID NO:55 is a clone designated herein as "DNA287177".
Figure 56 shows the amino acid sequence (SEQ ID NO:56) derived from the coding sequence of SEQ ID NO:55 shown in Figure 55.
Figure 57 shows a nucleotide sequence (SEQ ID NO:57) of a native sequence PRO60499 cDNA, wherein SEQ ID NO:57 is a clone designated herein as "DNA272237".
Figure 58 shows the amino acid sequence (SEQ ID NO:58) derived from the coding sequence of SEQ ID NO:57 shown in Figure 57.
Figure 59 shows a nucleotide sequence (SEQ ID NO:59) of a native sequence PRO69467 cDNA, wherein SEQ ID NO:59 is a clone designated herein as "DNA287178".
Figure 60 shows the amino acid sequence (SEQ ID NO:60) derived from the coding sequence of SEQ ID NO:59 shown in Figure 59.
Figure 61 shows a nucleotide sequence (SEQ ID NO:61) of a native sequence PRO61824 cDNA, wherein SEQ ID NO:61 is a clone designated herein as "DNA273865".
Figure 62 shows the amino acid sequence (SEQ ID NO:62) derived from the coding sequence of SEQ ID NO:61 shown in Figure 61.
Figure 63 shows a nucleotide sequence (SEQ ID NO:63) of a native sequence PR069468 cDNA, wherein SEQ ID NO:63 is a clone designated herein as "DNA287179".
Figure 64 shows the amino acid sequence (SEQ ID NO:64) derived from the coding sequence of SEQ ID NO:63 shown in Figure 63.
Figure 65 shows a nucleotide sequence (SEQ ID NO:65) of a native sequence PRO21341 cDNA, wherein SEQ ID NO:65 is a clone designated herein as "DNA287180".
Figure 66 shows the amino acid sequence (SEQ ID NO:66) derived from the coding sequence of SEQ ID NO:65 shown in Figure 65.
Figure 67A-B shows a nucleotide sequence (SEQ ID NO:67A-B) of a native sequence PR038213 cDNA, wherein SEQ ID NO:67A-B is a clone designated herein as "DNA227750".
Figure 68 shows the amino acid sequence (SEQ ID NO:68) derived from the coding sequence of SEQ ID NO:67A-B shown in Figure 67A-B.
Figure 69 shows a nucleotide sequence (SEQ ID NO:69) of a native sequence PR069469 cDNA, wherein SEQ ID NO:69 is a clone designated herein as "DNA287181".
Figure 70 shows the amino acid sequence (SEQ ID NO:70) derived from the coding sequence of SEQ ID NO:69 shown in Figure 69.
Figure 71 shows a nucleotide sequence (SEQ ID NO:71) of a native sequence PR037172 cDNA, wherein SEQ ID NO:71 is a clone designated herein as "DNA226709".
Figure 72 shows the amino acid sequence (SEQ ID NO:72) derived from the coding sequence of SEQ ID NO:71 shown in Figure 71.
Figure 73 shows a nucleotide sequence (SEQ ID NO:73) of a native sequence PRO35991 cDNA, wherein SEQ ID NO:73 is a clone designated herein as "DNA225528".
Figure 74 shows the amino acid sequence (SEQ ID NO:74) derived from the coding sequence of SEQ ID NO:73 shown in Figure 73.
Figure 75A-B shows a nucleotide sequence (SEQ ID NO:75A-B) of a native sequence PR036905 cDNA, wherein SEQ ID NO:75A-B is a clone designated herein as "DNA226442".
Figure 76 shows the amino acid sequence (SEQ ID NO:76) derived from the coding sequence of SEQ ID NO:75A-B shown in Figure 75A-B.
Figure 77 shows a nucleotide sequence (SEQ ID NO:77) of a native sequence PR069470 cDNA, wherein SEQ ID NO:77 is a clone designated herein as "DNA287182".
Figure 78 shows the amino acid sequence (SEQ ID NO:78) derived from the coding sequence of SEQ ID NO:77 shown in Figure 77.
Figure 79 shows a nucleotide sequence (SEQ ID NO:79) of a native sequence PR036451 cDNA, wherein SEQ ID NO:79 is a clone designated herein as "DNA288243".
Figure 80 shows the amino acid sequence (SEQ ID NO:80) derived from the coding sequence of SEQ ID NO:79 shown in Figure 79.
Figure 81 shows a nucleotide sequence (SEQ ID NO:81) of a native sequence PR069471 cDNA, wherein SEQ ID NO:81 is a clone designated herein as "DNA287184".
Figure 82 shows the amino acid sequence (SEQ ID NO:82) derived from the coding sequence of SEQ ID NO:81 shown in Figure 81.
Figure 83 shows a nucleotide sequence (SEQ ID NO:83) of a native sequence PR037492 cDNA, wherein SEQ ID NO:83 is a clone designated herein as "DNA227029".
Figure 84 shows the amino acid sequence (SEQ ID NO:84) derived from the coding sequence of SEQ ID NO:83 shown in Figure 83.
Figure 85A-B shows a nucleotide sequence (SEQ ID NO:85A-B) of a native sequence PRO70008 cDNA, wherein SEQ ID NO:85A-B is a clone designated herein as "DNA288244".
Figure 86 shows the amino acid sequence (SEQ ID NO:86) derived from the coding sequence of SEQ ID NO:85A-B shown in Figure 85A-B.
Figure 87 shows a nucleotide sequence (SEQ ID NO:87) of a native sequence PR069472 cDNA, wherein SEQ ID NO:87 is a clone designated herein as "DNA287186".
Figure 88 shows the amino acid sequence (SEQ ID NO:88) derived from the coding sequence of SEQ ID NO:87 shown in Figure 87.
Figure 89 shows a nucleotide sequence (SEQ ID NO:89) of a native sequence PR069473 cDNA, wherein SEQ ID NO:89 is a clone designated herein as "DNA287187".
Figure 90 shows the amino acid sequence (SEQ ID NO:90) derived from the coding sequence of SEQ ID NO:89 shown in Figure 89.
Figure 91 shows a nucleotide sequence (SEQ ID NO:91) of a native sequence PR036996 cDNA, wherein SEQ ID NO:91 is a clone designated herein as "DNA226533".
Figure 92 shows the amino acid sequence (SEQ ID NO:92) derived from the coding sequence of SEQ ID NO:91 shown in Figure 91.
Figure 93 shows a nucleotide sequence (SEQ ID NO:93) of a native sequence PR022613 cDNA, wherein SEQ ID NO:93 is a clone designated herein as "DNA189698".

Figure 94 shows the amino acid sequence (SEQ ID NO:94) derived from the coding sequence of SEQ ID NO:93 shown in Figure 93.
Figure 95 shows a nucleotide sequence (SEQ ID NO:95) of a native sequence PR069475 cDNA, wherein SEQ ID NO:95 is a clone designated herein as "DNA287189".
Figure 96 shows the amino acid sequence (SEQ ID NO:96) derived from the coding sequence of SEQ ID NO:95 shown in Figure 95.
Figure 97 shows a nucleotide sequence (SEQ ID NO:97) of a native sequence PR061755 cDNA, wherein SEQ ID NO:97 is a clone designated herein as "DNA273794".
Figure 98 shows the amino acid sequence (SEQ ID NO:98) derived from the coding sequence of SEQ ID NO:97 shown in Figure 97.
Figure 99 shows a nucleotide sequence (SEQ ID NO:99) of a native sequence PR070009 cDNA, wherein SEQ ID NO:99 is a clone designated herein as "DNA288245".
Figure 100 shows the amino acid sequence (SEQ ID NO:100) derived from the coding sequence of SEQ ID NO:99 shown in Figure 99.
Figure 101 shows a nucleotide sequence (SEQ ID NO:101) of a native sequence PR069476 cDNA, wherein SEQ ID NO:101 is a clone designated herein as "DNA287190".
Figure 102 shows the amino acid sequence (SEQ ID NO:102) derived from the coding sequence of SEQ ID NO:101 shown in Figure 101.
Figure 103 shows a nucleotide sequence (SEQ ID NO:103) of a native sequence PR04881 cDNA, wherein SEQ ID NO:103 is a clone designated herein as "DNA103554".
Figure 104 shows the amino acid sequence (SEQ ID NO:104) derived from the coding sequence of SEQ ID NO:103 shown in Figure 103.
Figure 105A-B shows a nucleotide sequence (SEQ ID NO:105A-B) of a native sequence PRO12876 cDNA, wherein SEQ ID NO:105A-B is a clone designated herein as "DNA151420".
Figure 106 shows the amino acid sequence (SEQ ID NO:106) derived from the coding sequence of SEQ ID NO:105A-B shown in Figure 105A-B.
Figure 107 shows a nucleotide sequence (SEQ ID NO:107) of a native sequence PR070010 cDNA, wherein SEQ ID NO:107 is a clone designated herein as "DNA288246".
Figure 108 shows the amino acid sequence (SEQ ID NO:108) derived from the coding sequence of SEQ ID NO: 107 shown in Figure 107.
Figure 109 shows a nucleotide sequence (SEQ ID NO:109) of a native sequence PR037534 cDNA, wherein SEQ ID NO:109 is a clone designated herein as "DNA227071".
Figure 110 shows the amino acid sequence (SEQ ID NO:110) derived from the coding sequence of SEQ ID NO:109 shown in Figure 109.
Figure 111A-B shows a nucleotide sequence (SEQ ID NO:111A-B) of a native sequence PR021928 cDNA, wherein SEQ ID NO:111A-B is a clone designated herein as "DNA188400".
Figure 112 shows the amino acid sequence (SEQ ID NO:112) derived from the coding sequence of SEQ ID NO: 111A-B shown in Figure 111A-B.
Figure 113A-B shows a nucleotide sequence (SEQ ID NO:113A-B) of a native sequence PR069478 cDNA, wherein SEQ ID NO:113A-B is a clone designated herein as "DNA287192".
Figure 114 shows the amino acid sequence (SEQ ID NO:114) derived from the coding sequence of SEQ ID NO:113A-B shown in Figure 113A-B.
Figure 115A-B shows a nucleotide sequence (SEQ ID NO:115A-B) of a native sequence PRO69479 cDNA, wherein SEQ ID NO:115A-B is a clone designated herein as "DNA287193".
Figure 116 shows the amino acid sequence (SEQ ID NO:116) derived from the coding sequence of SEQ ID NO:115A-B shown in Figure 115A-B.
Figure 117 shows a nucleotide sequence (SEQ ID NO:117) of a native sequence PR069480 cDNA, wherein SEQ ID NO:117 is a clone designated herein as "DNA287194".
Figure 118 shows the amino acid sequence (SEQ ID NO:118) derived from the coding sequence of SEQ ID NO:117 shown in Figure 117.
Figure 119 shows a nucleotide sequence (SEQ ID NO:119) of a native sequence PR069481 cDNA, wherein SEQ ID NO:119 is a clone designated herein as "DNA287195".
Figure 120 shows the amino acid sequence (SEQ ID NO:120) derived from the coding sequence of SEQ ID NO:119 shown in Figure 119.
Figure 121 shows a nucleotide sequence (SEQ ID NO:121) of a native sequence PR069482 cDNA, wherein SEQ ID NO:121 is a clone designated herein as "DNA287196".
Figure 122 shows the amino acid sequence (SEQ ID NO:122) derived from the coding sequence of SEQ ID NO:121 shown in Figure 121.
Figure 123 shows a nucleotide sequence (SEQ ID NO:123) of a native sequence PR069483 cDNA, wherein SEQ ID NO:123 is a clone designated herein as "DNA287197".
Figure 124 shows the amino acid sequence (SEQ ID NO:124) derived from the coding sequence of SEQ ID NO:123 shown in Figure 123.
Figure 125 shows a nucleotide sequence (SEQ ID NO:125) of a native sequence PRO38642 cDNA, wherein SEQ ID NO:125 is a clone designated herein as "DNA228179".
Figure 126 shows the amino acid sequence (SEQ ID NO:126) derived from the coding sequence of SEQ ID NO:125 shown in Figure 125.
Figure 127 shows a nucleotide sequence (SEQ ID NO:127) of a native sequence PR069484 cDNA, wherein SEQ ID NO:127 is a clone designated herein as "DNA287198".
Figure 128 shows the amino acid sequence (SEQ ID NO:128) derived from the coding sequence of SEQ ID NO:127 shown in Figure 127.
Figure 129 shows a nucleotide sequence (SEQ ID NO:129) of a native sequence PRO66269 cDNA, wherein SEQ ID NO:129 is a clone designated herein as "DNA287199".
Figure 130 shows the amino acid sequence (SEQ ID NO:130) derived from the coding sequence of SEQ ID NO:129 shown in Figure 129.
Figure 131 shows a nucleotide sequence (SEQ ID NO:131) of a native sequence PRO1723 cDNA, wherein SEQ ID NO:131 is a clone designated herein as "DNA82376".
Figure 132 shows the amino acid sequence (SEQ ID NO:132) derived from the coding sequence of SEQ ID NO:131 shown in Figure 131.
Figure 133 shows a nucleotide sequence (SEQ ID NO:133) of a native sequence PRO22297 cDNA, wherein SEQ ID NO:133 is a clone designated herein as "DNA287623".
Figure 134 shows the amino acid sequence (SEQ ID NO:134) derived from the coding sequence of SEQ ID NO:133 shown in Figure 133.
Figure 135 shows a nucleotide sequence (SEQ ID NO:135) of a native sequence PR061349 cDNA, wherein SEQ ID NO:135 is a clone designated herein as "DNA273346".
Figure 136 shows the amino acid sequence (SEQ ID NO:136) derived from the coding sequence of SEQ ID NO:135 shown in Figure 135.
Figure 137 shows a nucleotide sequence (SEQ ID NO:137) of a native sequence PR069485 cDNA, wherein SEQ ID NO:137 is a clone designated herein as "DNA287201".
Figure 138 shows the amino acid sequence (SEQ ID NO:138) derived from the coding sequence of SEQ ID NO:137 shown in Figure 137.
Figure 139 shows a nucleotide sequence (SEQ ID NO:139) of a native sequence PRO69486 cDNA, wherein SEQ ID NO:139 is a clone designated herein as "DNA287202".
Figure 140 shows the amino acid sequence (SEQ ID NO:140) derived from the coding sequence of SEQ ID NO:139 shown in Figure 139.
Figure 141 shows a nucleotide sequence (SEQ ID NO:141) of a native sequence PR069487 cDNA, wherein SEQ ID NO:141 is a clone designated herein as "DNA287203".
Figure 142 shows the amino acid sequence (SEQ ID NO:142) derived from the coding sequence of SEQ ID NO:141 shown in Figure 141.
Figure 143 shows a nucleotide sequence (SEQ ID NO:143) of a native sequence PR036963 cDNA, wherein SEQ ID NO:143 is a clone designated herein as "DNA226500".
Figure 144 shows the amino acid sequence (SEQ ID NO:144) derived from the coding sequence of SEQ ID NO:143 shown in Figure 143.
Figure 145 shows a nucleotide sequence (SEQ ID NO:145) of a native sequence PR023814 cDNA, wherein SEQ ID NO:145 is a clone designated herein as "DNA287204".
Figure 146 shows the amino acid sequence (SEQ ID NO:146) derived from the coding sequence of SEQ ID NO:145 shown in Figure 145.
Figure 147 shows a nucleotide sequence (SEQ ID NO:147) of a native sequence PRO57980 cDNA, wherein SEQ ID NO:147 is a clone designated herein as "DNA287205".
Figure 148 shows the amino acid sequence (SEQ ID NO:148) derived from the coding sequence of SEQ ID NO:147 shown in Figure 147.
Figure 149 shows a nucleotide sequence (SEQ ID NO:149) of a native sequence PR020128 cDNA, wherein SEQ ID NO:149 is a clone designated herein as "DNA171400".
Figure 150 shows the amino acid sequence (SEQ ID NO:150) derived from the coding sequence of SEQ ID NO:149 shown in Figure 149.
Figure 151 shows a nucleotide sequence (SEQ ID NO:151) of a native sequence PR04551 cDNA, wherein SEQ ID NO:151 is a clone designated herein as "DNA103221".
Figure 152 shows the amino acid sequence (SEQ ID NO:152) derived from the coding sequence of SEQ ID NO:151 shown in Figure 151.
Figure 153 shows a nucleotide sequence (SEQ ID NO:153) of a native sequence PR069488 cDNA, wherein SEQ ID NO:153 is a clone designated herein as "DNA287206".
Figure 154 shows the amino acid sequence (SEQ ID NO:154) derived from the coding sequence of SEQ ID NO:153 shown in Figure 153.
Figure 155 shows a nucleotide sequence (SEQ ID NO:155) of a native sequence PR039268 cDNA, wherein SEQ ID NO:155 is a clone designated herein as "DNA287207".
Figure 156 shows the amino acid sequence (SEQ ID NO:156) derived from the coding sequence of SEQ ID NO:155 shown in Figure 155.
Figure 157 shows a nucleotide sequence (SEQ ID NO:157) of a native sequence PR069489 cDNA, wherein SEQ ID NO:157 is a clone designated herein as "DNA287208".
Figure 158 shows the amino acid sequence (SEQ ID NO:158) derived from the coding sequence of SEQ ID NO:157 shown in Figure 157.
Figure 159 shows a nucleotide sequence (SEQ ID NO:159) of a native sequence PR069490 cDNA, wherein SEQ ID NO: 159 is a clone designated herein as "DNA287209".
Figure 160 shows the amino acid sequence (SEQ ID NO:160) derived from the coding sequence of SEQ ID NO:159 shown in Figure 159.
Figure 161 shows a nucleotide sequence (SEQ ID NO:161) of a native sequence PRO69491 cDNA, wherein SEQ ID NO:161 is a clone designated herein as "DNA287625".
Figure 162 shows the amino acid sequence (SEQ ID NO:162) derived from the coding sequence of SEQ ID NO:161 shown in Figure 161.
Figure 163 shows a nucleotide sequence (SEQ ID NO:163) of a native sequence PRO69492 cDNA, wherein SEQ ID NO:163 is a clone designated herein as "DNA287211".
Figure 164 shows the amino acid sequence (SEQ ID NO:164) derived from the coding sequence of SEQ ID NO:163 shown in Figure 163.
Figure 165 shows a nucleotide sequence (SEQ ID NO:165) of a native sequence PRO37713 cDNA, wherein SEQ ID NO:165 is a clone designated herein as "DNA227250".
Figure 166 shows the amino acid sequence (SEQ ID NO:166) derived from the coding sequence of SEQ ID NO:165 shown in Figure 165.
Figure 167 shows a nucleotide sequence (SEQ ID NO:167) of a native sequence PRO58993cDNA, wherein SEQ ID NO:167 is a clone designated herein as "DNA287212".
Figure 168 shows the amino acid sequence (SEQ ID NO:168) derived from the coding sequence of SEQ ID NO:167 shown in Figure 167.
Figure 169 shows a nucleotide sequence (SEQ ID NO:169) of a native sequence PR069493 cDNA, wherein SEQ ID NO:169 is a clone designated herein as "DNA287213".
Figure 170 shows the amino acid sequence (SEQ ID NO:170) derived from the coding sequence of SEQ ID NO:169 shown in Figure 169.
Figure 171 shows a nucleotide sequence (SEQ ID NO:171) of a native sequence PR069494 cDNA, wherein SEQ ID NO:171 is a clone designated herein as "DNA287214".
Figure 172 shows the amino acid sequence (SEQ ID NO:172) derived from the coding sequence of SEQ ID NO:171 shown in Figure 171.
Figure 173 shows a nucleotide sequence (SEQ ID NO:173) of a native sequence PR069495 cDNA, wherein SEQ ID NO:173 is a clone designated herein as "DNA287215".
Figure 174 shows the amino acid sequence (SEQ ID NO: 174) derived from the coding sequence of SEQ ID NO: 173 shown in Figure 173.
Figure 175 shows a nucleotide sequence (SEQ ID N0:175) of a native sequence PRO70011 cDNA, wherein SEQ ID NO: 175 is a clone designated herein as "DNA288247".
Figure 176 shows the amino acid sequence (SEQ ID NO: 176) derived from the coding sequence of SEQ ID NO: 175 shown in Figure 175.
Figure 177 shows a nucleotide sequence (SEQ ID NO: 177) of a native sequence PR062861 cDNA, wherein SEQ ID NO: 177 is a clone designated herein as "DNA275157".
Figure 178 shows the amino acid sequence (SEQ ID NO:178) derived from the coding sequence of SEQ ID NO: 177 shown in Figure 177.
Figure 179 shows a nucleotide sequence (SEQ ID NO: 179) of a native sequence PR036640 cDNA, wherein SEQ ID NO: 179 is a clone designated herein as "DNA226177".
Figure 180 shows the amino acid sequence (SEQ ID NO: 180) derived from the coding sequence of SEQ ID NO: 179 shown in Figure 179.
Figure 181A-B shows a nucleotide sequence (SEQ ID NO:181A-B) of a native sequence PRO36766 cDNA, wherein SEQ ID NO:181A-B is a clone designated herein as "DNA287217".
Figure 182 shows the amino acid sequence (SEQ ID NO:182) derived from the coding sequence of SEQ ID NO:181A-B shown in Figure 3181A-B.
Figure 183 shows a nucleotide sequence (SEQ ID NO:183) of a native sequence PR069497 cDNA, wherein SEQ ID NO: 183 is a clone designated herein as "DNA287218".
Figure 184 shows the amino acid sequence (SEQ ID NO: 194) derived from the coding sequence of SEQ ID NO:183 shown in Figure 183.
Figure 185 shows a nucleotide sequence (SEQ ID NO:185) of a native sequence PR069498 cDNA, wherein SEQ ID NO:185 is a clone designated herein as "DNA287219".
Figure 186 shows the amino acid sequence (SEQ ID NO: 186) derived from the coding sequence of SEQ ID NO: 185 shown in Figure 185.
Figure 187 shows a nucleotide sequence (SEQ ID NO: 187) of a native sequence PR069499 cDNA, wherein SEQ ID NO: 187 is a clone designated herein as "DNA287220".
Figure 188 shows the amino acid sequence (SEQ ID NO: 188) derived from the coding sequence of SEQ ID NO: 187 shown in Figure 187.
Figure 189 shows a nucleotide sequence (SEQ ID NO: 189) of a native sequence PR069500 cDNA, wherein SEQ ID N0:189 is a clone designated herein as "DNA297221".
Figure 190 shows the amino acid sequence (SEQ ID NO: 190) derived from the coding sequence of SEQ ID NO: 189 shown in Figure 189.
Figure 191 shows a nucleotide sequence (SEQ ID NO: 191) of a native sequence PRO69501 cDNA, wherein SEQ ID NO: 191 is a clone designated herein as "DNA287222".
Figure 192 shows the amino acid sequence (SEQ ID NO: 192) derived from the coding sequence of SEQ ID NO: 191 shown in Figure 191.
Figure 193 shows a nucleotide sequence (SEQ ID NO:193) of a native sequence PR070012 cDNA, wherein SEQ ID NO: 193 is a clone designated herein as "DNA288248".

Figure 194 shows the amino acid sequence (SEQ ID NO: 194) derived from the coding sequence of SEQ ID NO:193 shown in Figure 193.
Figure 195 shows a nucleotide sequence (SEQ ID NO: 195) of a native sequence PR069503 cDNA, wherein SEQ ID NO: 195 is a clone designated herein as "DNA287224".
Figure 196 shows the amino acid sequence (SEQ ID NO: 196) derived from the coding sequence of SEQ ID NO:195 shown in Figure 195.
Figure 197 shows a nucleotide sequence (SEQ ID NO:197) of a native sequence PR069474 cDNA, wherein SEQ ID NO: 197 is a clone designated herein as "DNA287188".
Figure 198 shows the amino acid sequence (SEQ ID NO: 198) derived from the coding sequence of SEQ ID NO:197 shown in Figure 197.
Figure 199 shows a nucleotide sequence (SEQ ID NO:199) of a native sequence PR069505 cDNA, wherein SEQ ID NO:199 is a clone designated herein as "DNA287226".
Figure 200 shows the amino acid sequence (SEQ ID NO:200) derived from the coding sequence of SEQ ID NO:199 shown in Figure 199.
Figure 201 shows a nucleotide sequence (SEQ ID NO:201) of a native sequence PR069506 cDNA, wherein SEQ ID NO:201 is a clone designated herein as "DNA287227".
Figure 202 shows the amino acid sequence (SEQ ID NO:202) derived from the coding sequence of SEQ ID NO:201 shown in Figure 201.
Figure 203 shows a nucleotide sequence (SEQ ID NO:203) of a native sequence PRO69507 cDNA, wherein SEQ ID NO:203 is a clone designated herein as "DNA288249".
Figure 204 shows the amino acid sequence (SEQ ID NO:204) derived from the coding sequence of SEQ ID NO: 203 shown in Figure 203.
Figure 205 shows a nucleotide sequence (SEQ ID NO:205) of a native sequence PR051301 cDNA, wherein SEQ ID NO:205 is a clone designated herein as "DNA256257".
Figure 206 shows the amino acid sequence (SEQ ID NO:206) derived from the coding sequence of SEQ ID NO: 205 shown in Figure 205.
Figure 207 shows a nucleotide sequence (SEQ ID NO:207) of a native sequence PR069508 cDNA, wherein SEQ ID NO:207 is a clone designated herein as "DNA287229".
Figure 208 shows the amino acid sequence (SEQ ID NO:208) derived from the coding sequence of SEQ ID NO: 207 shown in Figure 207.
Figure 209 shows a nucleotide sequence (SEQ ID NO:209) of a native sequence PRO69509 cDNA, wherein SEQ ID NO:209 is a clone designated herein as "DNA287230".
Figure 210 shows the amino acid sequence (SEQ ID NO:210) derived from the coding sequence of SEQ ID NO:209 shown in Figure 209.
Figure 211 shows a nucleotide sequence (SEQ ID NO:211) of a native sequence PR069510 cDNA, wherein SEQ ID NO:211 is a clone designated herein as "DNA287231".
Figure 212 shows the amino acid sequence (SEQ ID NO:212) derived from the coding sequence of SEQ ID NO: 211 shown in Figure 211.
Figure 213 shows a nucleotide sequence (SEQ ID NO:213) of a native sequence PRO69511 cDNA, wherein SEQ ID NO:213 is a clone designated herein as "DNA287232".
Figure 214 shows the amino acid sequence (SEQ ID NO:214) derived from the coding sequence of SEQ ID NO:213 shown in Figure 213.
Figure 215 shows a nucleotide sequence (SEQ ID NO:215) of a native sequence PR051309 cDNA, wherein SEQ ID NO:215 is a clone designated herein as "DNA256265".
Figure 216 shows the amino acid sequence (SEQ ID NO:216) derived from the coding sequence of SEQ ID NO:215 shown in Figure 215.
Figure 217A-B shows a nucleotide sequence (SEQ ID NO:217A-B) of a native sequence PR050578 cDNA, wherein SEQ ID NO:217A-B is a clone designated herein as "DNA255513".
Figure 218 shows the amino acid sequence (SEQ ID NO:218) derived from the coding sequence of SEQ ID NO:217A-B shown in Figure 217A-B.
Figure 219A-B shows a nucleotide sequence (SEQ ID NO:219A-B) of a native sequence PR069512 cDNA, wherein SEQ ID NO:219A-B is a clone designated herein as "DNA287233".
Figure 220 shows the amino acid sequence (SEQ ID NO:220) derived from the coding sequence of SEQ ID NO:219A-B shown in Figure 219A-B.
Figure 221 shows a nucleotide sequence (SEQ ID NO:221) of a native sequence PR069513 cDNA, wherein SEQ ID NO:221 is a clone designated herein as "DNA287234".
Figure 222 shows the amino acid sequence (SEQ ID NO:222) derived from the coding sequence of SEQ ID NO:221 shown in Figure 221.
Figure 223 shows a nucleotide sequence (SEQ ID NO:223) of a native sequence PR069514 cDNA, wherein SEQ ID NO:223 is a clone designated herein as "DNA287235".
Figure 224 shows the amino acid sequence (SEQ ID NO:224) derived from the coding sequence of SEQ ID NO:223 shown in Figure 223.
Figure 225A-B shows a nucleotide sequence (SEQ ID NO:225A-B) of a native sequence PR010607 cDNA, wherein SEQ ID NO:225A-B is a clone designated herein as "DNA287236".
Figure 226 shows the amino acid sequence (SEQ ID NO:226) derived from the coding sequence of SEQ ID NO:225A-B shown in Figure 225A-B.
Figure 227A-B shows a nucleotide sequence (SEQ ID NO:227A-B) of a native sequence PR061705 cDNA, wherein SEQ ID NO:227A-B is a clone designated herein as "DNA273742".
Figure 228 shows the amino acid sequence (SEQ ID NO:228) derived from the coding sequence of SEQ ID NO:227A-B shown in Figure 227A-B.
Figure 229 shows a nucleotide sequence (SEQ ID NO:229) of a native sequence PR049214 cDNA, wherein SEQ ID NO:229 is a clone designated herein as "DNA253811".
Figure 230 shows the amino acid sequence (SEQ ID NO:230) derived from the coding sequence of SEQ ID NO:229 shown in Figure 229.
Figure 231 shows a nucleotide sequence (SEQ ID NO:231) of a native sequence PRO39648 cDNA, wherein SEQ ID NO:231 is a clone designated herein as "DNA287237".
Figure 232 shows the amino acid sequence (SEQ ID NO:232) derived from the coding sequence of SEQ ID NO:231 shown in Figure 231.
Figure 233 shows a nucleotide sequence (SEQ ID NO:233) of a native sequence PR069515 cDNA, wherein SEQ ID NO:233 is a clone designated herein as "DNA287238".
Figure 234 shows the amino acid sequence (SEQ ID NO:234) derived from the coding sequence of SEQ ID NO:233 shown in Figure 233.
Figure 235 shows a nucleotide sequence (SEQ ID NO:235) of a native sequence PRO38497 cDNA, wherein SEQ ID NO:235 is a clone designated herein as "DNA287239".
Figure 236 shows the amino acid sequence (SEQ ID NO:236) derived from the coding sequence of SEQ ID NO:235 shown in Figure 235.
Figure 237 shows a nucleotide sequence (SEQ ID NO:237) of a native sequence PR029371 cDNA, wherein SEQ ID NO:237 is a clone designated herein as "DNA287240".
Figure 238 shows the amino acid sequence (SEQ ID NO:238) derived from the coding sequence of SEQ ID NO:237 shown in Figure 237.
Figure 239 shows a nucleotide sequence (SEQ ID NO:239) of a native sequence PR070013 cDNA, wherein SEQ ID NO:239 is a clone designated herein as "DNA288250".
Figure 240 shows the amino acid sequence (SEQ ID NO:240) derived from the coding sequence of SEQ ID NO:239 shown in Figure 239.
Figure 241 shows a nucleotide sequence (SEQ ID NO:241) of a native sequence PR069516 cDNA, wherein SEQ ID NO:241 is a clone designated herein as "DNA287241".
Figure 242 shows the amino acid sequence (SEQ ID NO:242) derived from the coding sequence of SEQ ID NO:241 shown in Figure 241.
Figure 243 shows a nucleotide sequence (SEQ ID NO:243) of a native sequence PR069517 cDNA, wherein SEQ ID NO:243 is a clone designated herein as "DNA287242".
Figure 244 shows the amino acid sequence (SEQ ID NO:244) derived from the coding sequence of SEQ ID NO:243 shown in Figure 243.
Figure 245 shows a nucleotide sequence (SEQ ID NO:245) of a native sequence PRO6951 cDNA, wherein SEQ ID NO:245 is a clone designated herein as "DNA287243".
Figure 246 shows the amino acid sequence (SEQ ID NO:246) derived from the coding sequence of SEQ ID NO:245 shown in Figure 245.
Figure 247 shows a nucleotide sequence (SEQ ID NO:247) of a native sequence PR070014 cDNA, wherein SEQ ID NO:247 is a clone designated herein as "DNA288251".
Figure 248 shows the amino acid sequence (SEQ ID NO:248) derived from the coding sequence of SEQ ID NO:247 shown in Figure 247.
Figure 249 shows a nucleotide sequence (SEQ ID NO:249) of a native sequence PR069520 cDNA, wherein SEQ ID NO:249 is a clone designated herein as "DNA287245".
Figure 250 shows the amino acid sequence (SEQ ID NO:250) derived from the coding sequence of SEQ ID NO:249 shown in Figure 249.
Figure 251 shows a nucleotide sequence (SEQ ID NO:251) of a native sequence PRO69521 cDNA, wherein SEQ ID NO:251 is a clone designated herein as "DNA287246".
Figure 252 shows the amino acid sequence (SEQ ID NO:252) derived from the coding sequence of SEQ ID NO:251 shown in Figure 251.
Figure 253 shows a nucleotide sequence (SEQ ID NO:253) of a native sequence PRO69522 cDNA, wherein SEQ ID NO:253 is a clone designated herein as "DNA287247".
Figure 254 shows the amino acid sequence (SEQ ID NO:254) derived from the coding sequence of SEQ ID NO:253 shown in Figure 253.
Figure 255 shows a nucleotide sequence (SEQ ID NO:255) of a native sequence PR069523 cDNA, wherein SEQ ID NO:255 is a clone designated herein as "DNA287628".
Figure 256 shows the amino acid sequence (SEQ ID NO:256) derived from the coding sequence of SEQ ID NO:255 shown in Figure 255.
Figure 257 shows a nucleotide sequence (SEQ ID NO:257) of a native sequence PR060513 cDNA, wherein SEQ ID NO:257 is a clone designated herein as "DNA272251".
Figure 258 shows the amino acid sequence (SEQ ID NO:258) derived from the coding sequence of SEQ ID NO:257 shown in Figure 257.
Figure 259 shows a nucleotide sequence (SEQ ID NO:259) of a native sequence PR02512 cDNA, wherein SEQ ID NO:259 is a clone designated herein as "DNA288252".
Figure 260 shows the amino acid sequence (SEQ ID NO:260) derived from the coding sequence of SEQ ID NO:259 shown in Figure 259.
Figure 261 shows a nucleotide sequence (SEQ ID NO:261) of a native sequence PR069524 cDNA, wherein SEQ ID NO:261 is a clone designated herein as "DNA287250".
Figure 262 shows the amino acid sequence (SEQ ID NO:262) derived from the coding sequence of SEQ ID NO:261 shown in Figure 261.
Figure 263 shows a nucleotide sequence (SEQ ID NO:263) of a native sequence PRO12569 cDNA, wherein SEQ ID NO:263 is a clone designated herein as "DNA150989".
Figure 264 shows the amino acid sequence (SEQ ID NO:264) derived from the coding sequence of SEQ ID NO:263 shown in Figure 263.
Figure 265 shows a nucleotide sequence (SEQ ID NO:265) of a native sequence PR069525 cDNA, wherein SEQ ID NO:265 is a clone designated herein as "DNA287251".
Figure 266 shows the amino acid sequence (SEQ ID NO:266) derived from the coding sequence of SEQ ID NO: 265 shown in Figure 265.
Figure 267 shows a nucleotide sequence (SEQ ID NO:267) of a native sequence PRO69526 cDNA, wherein SEQ ID NO:267 is a clone designated herein as "DNA287252".
Figure 268 shows the amino acid sequence (SEQ ID NO:268) derived from the coding sequence of SEQ ID NO:267 shown in Figure 267.
Figure 269 shows a nucleotide sequence (SEQ ID NO:269) of a native sequence PR069527 cDNA, wherein SEQ ID NO:269 is a clone designated herein as "DNA287253".
Figure 270 shows the amino acid sequence (SEQ ID NO:270) derived from the coding sequence of SEQ ID NO:269 shown in Figure 269.
Figure 271 shows a nucleotide sequence (SEQ ID NO:271) of a native sequence PR069528 cDNA, wherein SEQ ID NO:271 is a clone designated herein as "DNA287254".
Figure 272 shows the amino acid sequence (SEQ ID NO:272) derived from the coding sequence of SEQ ID NO:271 shown in Figure 271.
Figure 273 shows a nucleotide sequence (SEQ ID NO:273) of a native sequence PR069529 cDNA, wherein SEQ ID NO:273 is a clone designated herein as "DNA287255".
Figure 274 shows the amino acid sequence (SEQ ID NO:274) derived from the coding sequence of SEQ ID NO:273 shown in Figure 273.
Figure 275 shows a nucleotide sequence (SEQ ID NO:275) of a native sequence PR012166 cDNA, wherein SEQ ID NO:275 is a clone designated herein as "DNA151021".
Figure 276 shows the amino acid sequence (SEQ ID NO:276) derived from the coding sequence of SEQ ID NO:275 shown in Figure 275.
Figure 277 shows a nucleotide sequence (SEQ ID NO:277) of a native sequence PR02154 cDNA, wherein SEQ ID NO:277 is a clone designated herein as "DNA287630".
Figure 278 shows the amino acid sequence (SEQ ID NO:278) derived from the coding sequence of SEQ ID NO:277 shown in Figure 277.
Figure 279 shows a nucleotide sequence (SEQ ID NO:279) of a native sequence PRO69530 cDNA, wherein SEQ ID NO:279 is a clone designated herein as "DNA287257".
Figure 280 shows the amino acid sequence (SEQ ID NO:280) derived from the coding sequence of SEQ ID NO:279 shown in Figure 279.
Figure 281 shows a nucleotide sequence (SEQ ID NO:281) of a native sequence PR051916 cDNA, wherein SEQ ID NO:281 is a clone designated herein as "DNA257326".
Figure 282 shows the amino acid sequence (SEQ ID NO:282) derived from the coding sequence of SEQ ID NO:281 shown in Figure 281.
Figure 283 shows a nucleotide sequence (SEQ ID NO:283) of a native sequence PRO52174 cDNA, wherein SEQ ID NO:283 is a clone designated herein as "DNA287258".
Figure 284 shows the amino acid sequence (SEQ ID NO:284) derived from the coding sequence of SEQ ID NO:283 shown in Figure 283.
Figure 285 shows a nucleotide sequence (SEQ ID NO:285) of a native sequence PR06953l cDNA, wherein SEQ ID NO:285 is a clone designated herein as "DNA287259".
Figure 286 shows the amino acid sequence (SEQ ID NO:286) derived from the coding sequence of SEQ ID NO:285 shown in Figure 285.
Figure 287 shows a nucleotide sequence (SEQ ID NO:287) of a native sequence PR069532 cDNA, wherein SEQ ID NO:287 is a clone designated herein as "DNA287260".
Figure 288 shows the amino acid sequence (SEQ ID NO:288) derived from the coding sequence of SEQ ID NO:287 shown in Figure 287.
Figure 289 shows a nucleotide sequence (SEQ ID NO:289) of a native sequence PR069533 cDNA, wherein SEQ ID NO:289 is a clone designated herein as "DNA287261".
Figure 290 shows the amino acid sequence (SEQ ID NO:290) derived from the coding sequence of SEQ ID NO:289 shown in Figure 289.
Figure 291 shows a nucleotide sequence (SEQ ID NO:291) of a native sequence PR069534 cDNA, wherein SEQ ID NO:291 is a clone designated herein as "DNA287262".
Figure 292 shows the amino acid sequence (SEQ ID NO:292) derived from the coding sequence of SEQ ID NO:291 shown in Figure 291.
Figure 293 shows a nucleotide sequence (SEQ ID NO:293) of a native sequence PR054728 cDNA, wherein SEQ ID NO:293 is a clone designated herein as "DNA260982".

Figure 294 shows the amino acid sequence (SEQ ID NO:294) derived from the coding sequence of SEQ ID NO:293 shown in Figure 293.
Figure 295 shows a nucleotide sequence (SEQ ID NO:295) of a native sequence PR070015 cDNA, wherein SEQ ID NO:295 is a clone designated herein as "DNA288253".
Figure 296 shows the amino acid sequence (SEQ ID NO:296) derived from the coding sequence of SEQ ID NO:295 shown in Figure 295.
Figure 297 shows a nucleotide sequence (SEQ ID NO:297) of a native sequence PR069536 cDNA, wherein SEQ ID NO:297 is a clone designated herein as "DNA288254".
Figure 298 shows the amino acid sequence (SEQ ID NO:298) derived from the coding sequence of SEQ ID NO:297 shown in Figure 297.
Figure 299 shows a nucleotide sequence (SEQ ID NO:299) of a native sequence PR069537 cDNA, wherein SEQ ID NO:299 is a clone designated herein as "DNA287265".
Figure 300 shows the amino acid sequence (SEQ ID NO:300) derived from the coding sequence of SEQ ID NO:299 shown in Figure 299.
Figure 301 shows a nucleotide sequence (SEQ ID NO:301) of a native sequence PR037498 cDNA, wherein SEQ ID NO:301 is a clone designated herein as "DNA227035".
Figure 302 shows the amino acid sequence (SEQ ID NO:302) derived from the coding sequence of SEQ ID NO:301 shown in Figure 301.
Figure 303A-B shows a nucleotide sequence (SEQ ID NO:303A-B) of a native sequence PRO22175 cDNA, wherein SEQ ID NO:303A-B is a clone designated herein as "DNA189214".
Figure 304 shows the amino acid sequence (SEQ ID NO:304) derived from the coding sequence of SEQ ID NO:303A-B shown in Figure 303A-B.
Figure 305 shows a nucleotide sequence (SEQ ID NO:305) of a native sequence PR069538 cDNA, wherein SEQ ID NO:305 is a clone designated herein as "DNA287266".
Figure 306 shows the amino acid sequence (SEQ ID NO:306) derived from the coding sequence of SEQ ID NO:305 shown in Figure 305.
Figure 307 shows a nucleotide sequence (SEQ ID NO:307) of a native sequence PR037015 cDNA, wherein SEQ ID NO:307 is a clone designated herein as "DNA287267".
Figure 308 shows the amino acid sequence (SEQ ID NO:308) derived from the coding sequence of SEQ ID NO:307 shown in Figure 307.
Figure 309 shows a nucleotide sequence (SEQ ID NO:309) of a native sequence PRO12187 cDNA, wherein SEQ ID NO:309 is a clone designated herein as "DNA151799".
Figure 310 shows the amino acid sequence (SEQ ID NO:310) derived from the coding sequence of SEQ ID NO:309 shown in Figure 309.
Figure 311 shows a nucleotide sequence (SEQ ID NO:3 11) of a native sequence PRO69539 cDNA, wherein SEQ ID NO:311 is a clone designated herein as "DNA287268".
Figure 312 shows the amino acid sequence (SEQ ID NO:312) derived from the coding sequence of SEQ ID NO:311 shown in Figure 311.
Figure 313 shows a nucleotide sequence (SEQ ID NO:313) of a native sequence PR069880 cDNA, wherein SEQ ID NO:313 is a clone designated herein as "DNA287632".
Figure 314 shows the amino acid sequence (SEQ ID NO:314) derived from the coding sequence of SEQ ID NO:313 shown in Figure 313.
Figure 315 shows a nucleotide sequence (SEQ ID NO:315) of a native sequence PRO69541 cDNA, wherein SEQ ID NO:315 is a clone designated herein as "DNA287270".
Figure 316 shows the amino acid sequence (SEQ ID NO:316) derived from the coding sequence of SEQ ID NO:315 shown in Figure 315.
Figure 317 shows a nucleotide sequence (SEQ ID NO:317) of a native sequence PR069542 cDNA, wherein SEQ ID NO:317 is a clone designated herein as "DNA287271".
Figure 318 shows the amino acid sequence (SEQ ID NO:319) derived from the coding sequence of SEQ ID NO:317 shown in Figure 317.
Figure 319 shows a nucleotide sequence (SEQ ID NO:319) of a native sequence PRO69543 cDNA, wherein SEQ ID NO:319 is a clone designated herein as "DNA287272".
Figure 320 shows the amino acid sequence (SEQ ID NO:320) derived from the coding sequence of SEQ ID NO:319 shown in Figure 319.
Figure 321 shows a nucleotide sequence (SEQ ID NO:321) of a native sequence PRO70016 cDNA, wherein SEQ ID NO:321 is a clone designated herein as "DNA288255".
Figure 322 shows the amino acid sequence (SEQ ID NO:322) derived from the coding sequence of SEQ ID NO:321 shown in Figure 321.
Figure 323A-B shows a nucleotide sequence (SEQ ID NO:323A-B) of a native sequence PR069545 cDNA, wherein SEQ ID NO:323A-B is a clone designated herein as "DNA287273".
Figure 324 shows the amino acid sequence (SEQ ID NO:324) derived from the coding sequence of SEQ ID NO:323A-B shown in Figure 323A-B.
Figure 325 shows a nucleotide sequence (SEQ ID NO:325) of a native sequence PR050197 cDNA, wherein SEQ ID NO:325 is a clone designated herein as "DNA255115".
Figure 326 shows the amino acid sequence (SEQ ID NO:326) derived from the coding sequence of SEQ ID NO:325 shown in Figure 325.
Figure 327 shows a nucleotide sequence (SEQ ID NO:327) of a native sequence PRO69546 cDNA, wherein SEQ ID NO:327 is a clone designated herein as "DNA287274".
Figure 328 shows the amino acid sequence (SEQ ID NO:328) derived from the coding sequence of SEQ ID NO:327 shown in Figure 327.
Figure 329 shows a nucleotide sequence (SEQ ID NO:329) of a native sequence PR069547 cDNA, wherein SEQ ID NO:329 is a clone designated herein as "DNA287275".
Figure 330 shows the amino acid sequence (SEQ ID NO:330) derived from the coding sequence of SEQ ID NO:329 shown in Figure 329.
Figure 331 shows a nucleotide sequence (SEQ ID NO:331) of a native sequence PR069548 cDNA, wherein SEQ ID NO:331 is a clone designated herein as "DNA287276".
Figure 332 shows the amino acid sequence (SEQ ID NO:332) derived from the coding sequence of SEQ ID NO:331 shown in Figure 331.
Figure 333 shows a nucleotide sequence (SEQ ID NO:333) of a native sequence PRO69549 cDNA, wherein SEQ ID NO:333 is a clone designated herein as "DNA287277".
Figure 334 shows the amino acid sequence (SEQ ID NO:334) derived from the coding sequence of SEQ ID NO:333 shown in Figure 333.
Figure 335 shows a nucleotide sequence (SEQ ID NO:335) of a native sequence PR069550 cDNA, wherein SEQ ID NO:335 is a clone designated herein as "DNA287278".
Figure 336 shows the amino acid sequence (SEQ ID NO:336) derived from the coding sequence of SEQ ID NO:335 shown in Figure 335.
Figure 337 shows a nucleotide sequence (SEQ ID NO:337) of a native sequence PR069551 cDNA, wherein SEQ ID NO:337 is a clone designated herein as "DNA287279".
Figure 338 shows the amino acid sequence (SEQ ID NO:339) derived from the coding sequence of SEQ ID NO:337 shown in Figure 337.
Figure 339 shows a nucleotide sequence (SEQ ID NO:339) of a native sequence PR069552 cDNA, wherein SEQ ID NO:339 is a clone designated herein as "DNA287280".
Figure 340 shows the amino acid sequence (SEQ ID NO:340) derived from the coding sequence of SEQ ID NO:339 shown in Figure 339.
Figure 341 shows a nucleotide sequence (SEQ ID NO:341) of a native sequence PRO37460 cDNA, wherein SEQ ID NO:341 is a clone designated herein as "DNA226997".
Figure 342 shows the amino acid sequence (SEQ ID NO:342) derived from the coding sequence of SEQ ID NO:341 shown in Figure 341.
Figure 343 shows a nucleotide sequence (SEQ ID NO:343) of a native sequence PR042223 cDNA, wherein SEQ ID NO:343 is a clone designated herein as "DNA242927".
Figure 344 shows the amino acid sequence (SEQ ID NO:344) derived from the coding sequence of SEQ ID NO:343 shown in Figure 343.
Figure 345A-B shows a nucleotide sequence (SEQ ID NO:345A-B) of a native sequence PR069553 cDNA, wherein SEQ ID NO:345A-B is a clone designated herein as "DNA287281".
Figure 346 shows the amino acid sequence (SEQ ID NO:346) derived from the coding sequence of SEQ ID NO:345A-B shown in Figure 345A-B.
Figure 347 shows a nucleotide sequence (SEQ ID NO:347) of a native sequence PR069554 cDNA, wherein SEQ ID NO:347 is a clone designated herein as "DNA287282".
Figure 348 shows the amino acid sequence (SEQ ID NO:348) derived from the coding sequence of SEQ ID NO:347 shown in Figure 347.
Figure 349 shows a nucleotide sequence (SEQ ID NO:349) of a native sequence PR069555 cDNA, wherein SEQ ID NO:349 is a clone designated herein as "DNA287283".
Figure 350 shows the amino acid sequence (SEQ ID NO:350) derived from the coding sequence of SEQ ID NO:349 shown in Figure 349.
Figure 351 shows a nucleotide sequence (SEQ ID NO:351) of a native sequence PRO61014 cDNA, wherein SEQ ID NO:351 is a clone designated herein as "DNA272930".
Figure 352 shows the amino acid sequence (SEQ ID NO:352) derived from the coding sequence of SEQ ID NO:351 shown in Figure 351.
Figure 353 shows a nucleotide sequence (SEQ ID NO:353) of a native sequence PR059915 cDNA, wherein SEQ ID NO:353 is a clone designated herein as "DNA287284".
Figure 354 shows the amino acid sequence (SEQ ID NO:354) derived from the coding sequence of SEQ ID NO:353 shown in Figure 353.
Figure 355A-B shows a nucleotide sequence (SEQ ID NO:355A-B) of a native sequence PR037891 cDNA, wherein SEQ ID NO:355A-B is a clone designated herein as "DNA227428".
Figure 356 shows the amino acid sequence (SEQ ID NO:356) derived from the coding sequence of SEQ ID NO:355A-B shown in Figure 355A-B.
Figure 357 shows a nucleotide sequence (SEQ ID NO:357) of a native sequence PR069556 cDNA, wherein SEQ ID NO:357 is a clone designated herein as "DNA287285".
Figure 358 shows the amino acid sequence (SEQ ID NO:358) derived from the coding sequence of SEQ ID NO:357 shown in Figure 357.
Figure 359 shows a nucleotide sequence (SEQ ID NO:359) of a native sequence PR012875 cDNA, wherein SEQ ID NO:359 is a clone designated herein as "DNA151237".
Figure 360 shows the amino acid sequence (SEQ ID NO:360) derived from the coding sequence of SEQ ID NO:359 shown in Figure 359.
Figure 361 shows a nucleotide sequence (SEQ ID NO:361) of a native sequence PR070017 cDNA, wherein SEQ ID NO:361 is a clone designated herein as "DNA288256".
Figure 362 shows the amino acid sequence (SEQ ID NO:362) derived from the coding sequence of SEQ ID NO:361 shown in Figure 361.
Figure 363 shows a nucleotide sequence (SEQ ID NO:363) of a native sequence PRO70018 cDNA, wherein SEQ ID NO:363 is a clone designated herein as "DNA288257".
Figure 364 shows the amino acid sequence (SEQ ID NO:364) derived from the coding sequence of SEQ ID NO:363 shown in Figure 363.
Figure 365 shows a nucleotide sequence (SEQ ID NO:365) of a native sequence PR04426 cDNA, wherein SEQ ID NO:365 is a clone designated herein as "DNA287287".
Figure 366 shows the amino acid sequence (SEQ ID NO:366) derived from the coding sequence of SEQ ID NO:365 shown in Figure 365.
Figure 367 shows a nucleotide sequence (SEQ ID NO:367) of a native sequence PR069558 cDNA, wherein SEQ ID NO:367 is a clone designated herein as "DNA287288".
Figure 368 shows the amino acid sequence (SEQ ID NO:368) derived from the coding sequence of SEQ ID NO:367 shown in Figure 367.
Figure 369 shows a nucleotide sequence (SEQ ID NO:369) of a native sequence PR069559 cDNA, wherein SEQ ID NO:369 is a clone designated herein as "DNA287289".
Figure 370 shows the amino acid sequence (SEQ ID NO:370) derived from the coding sequence of SEQ ID NO:369 shown in Figure 369.
Figure 371 shows a nucleotide sequence (SEQ ID NO:371) of a native sequence PR037676 cDNA, wherein SEQ ID NO:371 is a clone designated herein as "DNA227213".
Figure 372 shows the amino acid sequence (SEQ ID NO:372) derived from the coding sequence of SEQ ID NO:371 shown in Figure 371.
Figure 373 shows a nucleotide sequence (SEQ ID NO:373) of a native sequence PR069560 cDNA, wherein SEQ ID NO:373 is a clone designated herein as "DNA287290".
Figure 374 shows the amino acid sequence (SEQ ID NO:374) derived from the coding sequence of SEQ ID NO:373 shown in Figure 373.
Figure 375 shows a nucleotide sequence (SEQ ID NO:375) of a native sequence PR069561 cDNA, wherein SEQ ID NO:375 is a clone designated herein as "DNA287291".
Figure 376 shows the amino acid sequence (SEQ ID NO:376) derived from the coding sequence of SEQ ID NO:375 shown in Figure 375.
Figure 377 shows a nucleotide sequence (SEQ ID NO:377) of a native sequence PR069562 cDNA, wherein SEQ ID NO:377 is a clone designated herein as "DNA287292".
Figure 378 shows the amino acid sequence (SEQ ID NO:378) derived from the coding sequence of SEQ ID NO:377 shown in Figure 377.
Figure 379 shows a nucleotide sequence (SEQ ID NO:379) of a native sequence PR063204 cDNA, wherein SEQ ID NO:379 is a clone designated herein as "DNA287293".
Figure 380 shows the amino acid sequence (SEQ ID NO:380) derived from the coding sequence of SEQ ID NO:379 shown in Figure 379.
Figure 381 shows a nucleotide sequence (SEQ ID NO:381) of a native sequence PR070019 cDNA, wherein SEQ ID NO:381 is a clone designated herein as "DNA288258".
Figure 382 shows the amino acid sequence (SEQ ID NO:382) derived from the coding sequence of SEQ ID NO: 3 81 shown in Figure 381.
Figure 383 shows a nucleotide sequence (SEQ ID NO:383) of a native sequence PR069564 cDNA, wherein SEQ ID NO:383 is a clone designated herein as "DNA287295" .
Figure 384 shows the amino acid sequence (SEQ ID NO:384) derived from the coding sequence of SEQ ID NO:383 shown in Figure 383.
Figure 385 shows a nucleotide sequence (SEQ ID NO:385) of a native sequence PR062830 cDNA, wherein SEQ ID NO:385 is a clone designated herein as "DNA287296".
Figure 386 shows the amino acid sequence (SEQ ID NO:386) derived from the coding sequence of SEQ ID NO:385 shown in Figure 385.
Figure 387 shows a nucleotide sequence (SEQ ID NO:387) of a native sequence PR069565 cDNA, wherein SEQ ID NO:387 is a clone designated herein as "DNA287297".
Figure 388 shows the amino acid sequence (SEQ ID NO:388) derived from the coding sequence of SEQ ID NO:387 shown in Figure 387.
Figure 389 shows a nucleotide sequence (SEQ ID NO:389) of a native sequence PR069566 cDNA, wherein SEQ ID NO:389 is a clone designated herein as "DNA287298".
Figure 390 shows the amino acid sequence (SEQ ID NO:390) derived from the coding sequence of SEQ ID NO:389 shown in Figure 389.
Figure 391 shows a nucleotide sequence (SEQ ID NO:391) of a native sequence PR069567 cDNA, wherein SEQ ID NO:391 is a clone designated herein as "DNA287299".
Figure 392 shows the amino acid sequence (SEQ ID NO:392) derived from the coding sequence of SEQ ID NO:391 shown in Figure 391.
Figure 393 shows a nucleotide sequence (SEQ ID NO:393) of a native sequence PRO49675 cDNA, wherein SEQ ID NO:393 is a clone designated herein as "DNA254572".

Figure 394 shows the amino acid sequence (SEQ ID NO:394) derived from the coding sequence of SEQ ID NO:393 shown in Figure 393.
Figure 395 shows a nucleotide sequence (SEQ ID NO:395) of a native sequence PR069568 cDNA, wherein SEQ ID NO:395 is a clone designated herein as "DNA287300".
Figure 396 shows the amino acid sequence (SEQ ID NO:396) derived from the coding sequence of SEQ ID NO:395 shown in Figure 395.
Figure 397 shows a nucleotide sequence (SEQ ID NO:397) of a native sequence PR02013 cDNA, wherein SEQ ID NO:397 is a clone designated herein as "DNA75526".
Figure 398 shows the amino acid sequence (SEQ ID NO:398) derived from the coding sequence of SEQ ID NO:397 shown in Figure 397.
Figure 399 shows a nucleotide sequence (SEQ ID NO:399) of a native sequence PR069569 cDNA, wherein SEQ ID NO:399 is a clone designated herein as "DNA287302".
Figure 400 shows the amino acid sequence (SEQ ID NO:400) derived from the coding sequence of SEQ ID NO:399 shown in Figure 399.
Figure 401 shows a nucleotide sequence (SEQ ID NO:401) of a native sequence PR069570 cDNA, wherein SEQ ID NO:401 is a clone designated herein as "DNA287303".
Figure 402 shows the amino acid sequence (SEQ ID NO:402) derived from the coding sequence of SEQ ID NO:401 shown in Figure 401.
Figure 403 shows a nucleotide sequence (SEQ ID NO:403) of a native sequence PR069571 cDNA, wherein SEQ ID NO:403 is a clone designated herein as "DNA287304".
Figure 404 shows the amino acid sequence (SEQ ID NO:404) derived from the coding sequence of SEQ ID NO:403 shown in Figure 403.
Figure 405A-B shows a nucleotide sequence (SEQ ID NO:405A-B) of a native sequence PR036403 cDNA, wherein SEQ ID NO:405A-B is a clone designated herein as "DNA225940".
Figure 406 shows the amino acid sequence (SEQ ID NO:406) derived from the coding sequence of SEQ ID NO:405A-B shown in Figure 405A-B.
Figure 407 shows a nucleotide sequence (SEQ ID NO:407) of a native sequence PR04676 cDNA, wherein SEQ ID NO:407 is a clone designated herein as "DNA288259".
Figure 408 shows the amino acid sequence (SEQ ID NO:408) derived from the coding sequence of SEQ ID NO:407 shown in Figure 407.
Figure 409 shows a nucleotide sequence (SEQ ID NO:409) of a native sequence PR037657 cDNA, wherein SEQ ID NO:409 is a clone designated herein as "DNA227194".
Figure 410 shows the amino acid sequence (SEQ ID NO:410) derived from the coding sequence of SEQ ID NO:409 shown in Figure 409.
Figure 411 shows a nucleotide sequence (SEQ ID NO:411) of a native sequence PR062097 cDNA, wherein SEQ ID NO:411 is a clone designated herein as "DNA274167".
Figure 412 shows the amino acid sequence (SEQ ID NO:412) derived from the coding sequence of SEQ ID NO:411 shown in Figure 411.
Figure 413 shows a nucleotide sequence (SEQ ID NO:413) of a native sequence PR038081 cDNA, wherein SEQ ID NO:413 is a clone designated herein as "DNA227618".
Figure 414 shows the amino acid sequence (SEQ ID NO:414) derived from the coding sequence of SEQ ID NO:413 shown in Figure 413.
Figure 415 shows a nucleotide sequence (SEQ ID NO:415) of a native sequence PRO69572 cDNA, wherein SEQ ID NO:415 is a clone designated herein as "DNA287306".
Figure 416 shows the amino acid sequence (SEQ ID NO:416) derived from the coding sequence of SEQ ID NO:415 shown in Figure 415.
Figure 417 shows a nucleotide sequence (SEQ ID NO:417) of a native sequence PR069573 cDNA, wherein SEQ ID NO:417 is a clone designated herein as "DNA287307".
Figure 418 shows the amino acid sequence (SEQ ID NO:418) derived from the coding sequence of SEQ ID NO:417 shown in Figure 417.
Figure 419 shows a nucleotide sequence (SEQ ID NO:419) of a native sequence PRO69574 cDNA. wherein SEQ ID NO:419 is a clone designated herein as "DNA287308".
Figure 420 shows the amino acid sequence (SEQ ID NO:420) derived from the coding sequence of SEQ ID NO:419 shown in Figure 419.
Figure 421 shows a nucleotide sequence (SEQ ID NO:421) of a native sequence PRO69883 cDNA, wherein SEQ ID NO:421 is a clone designated herein as "DNA287635".
Figure 422 shows the amino acid sequence (SEQ ID NO:422) derived from the coding sequence of SEQ ID NO:421 shown in Figure 421.
Figure 423 shows a nucleotide sequence (SEQ ID NO:423) of a native sequence PR069576 cDNA, wherein SEQ ID NO:423 is a clone designated herein as "DNA287310".
Figure 424 shows the amino acid sequence (SEQ ID NO:424) derived from the coding sequence of SEQ ID NO:423 shown in Figure 423.
Figure 425 shows a nucleotide sequence (SEQ ID NO:425) of a native sequence PR037584 cDNA, wherein SEQ ID NO:425 is a clone designated herein as "DNA227121".
Figure 426 shows the amino acid sequence (SEQ ID NO:426) derived from the coding sequence of SEQ ID NO:425 shown in Figure 425.
Figure 427 shows a nucleotide sequence (SEQ ID NO:427) of a native sequence PRO11603 cDNA, wherein SEQ ID NO:427 is a clone designated herein as "DNA151007".
Figure 428 shows the amino acid sequence (SEQ ID NO:428) derived from the coding sequence of SEQ ID NO:427 shown in Figure 427.
Figure 429 shows a nucleotide sequence (SEQ ID NO:429) of a native sequence PR070020 cDNA, wherein SEQ ID NO:429 is a clone designated herein as "DNA288260".
Figure 430 shows the amino acid sequence (SEQ ID NO:430) derived from the coding sequence of SEQ ID NO:429 shown in Figure 429.
Figure 431 shows a nucleotide sequence (SEQ ID NO:431) of a native sequence PRO51695 cDNA, wherein SEQ ID NO:431 is a clone designated herein as "DNA256762".
Figure 432 shows the amino acid sequence (SEQ ID NO:432) derived from the coding sequence of SEQ ID NO:431 shown in Figure 431.
Figure 433 shows a nucleotide sequence (SEQ ID NO:433) of a native sequence PR069579 cDNA, wherein SEQ ID NO:433 is a clone designated herein as "DNA287314".
Figure 434 shows the amino acid sequence (SEQ ID NO:434) derived from the coding sequence of SEQ ID NO:433 shown in Figure 433.
Figure 435 shows a nucleotide sequence (SEQ ID NO:435) of a native sequence PR069580 cDNA, wherein SEQ ID NO:435 is a clone designated herein as "DNA287315".
Figure 436 shows the amino acid sequence (SEQ ID NO:436) derived from the coding sequence of SEQ ID NO:435 shown in Figure 435.
Figure 437 shows a nucleotide sequence (SEQ ID NO:437) of a native sequence PR069581 cDNA, wherein SEQ ID NO:437 is a clone designated herein as "DNA287316".
Figure 438 shows the amino acid sequence (SEQ ID NO:438) derived from the coding sequence of SEQ ID NO:437 shown in Figure 437.
Figure 439 shows a nucleotide sequence (SEQ ID NO:439) of a native sequence PR069582 cDNA, wherein SEQ ID NO:439 is a clone designated herein as "DNA287317".
Figure 440 shows the amino acid sequence (SEQ ID NO:440) derived from the coding sequence of SEQ ID NO:439 shown in Figure 439.
Figure 441 shows a nucleotide sequence (SEQ ID NO:441) of a native sequence PR069583 cDNA, wherein SEQ ID NO:441 is a clone designated herein as "DNA287318".
Figure 442 shows the amino acid sequence (SEQ ID NO:442) derived from the coding sequence of SEQ ID NO:441 shown in Figure 441.
Figure 443 shows a nucleotide sequence (SEQ ID NO:443) of a native sequence PR069584 cDNA, wherein SEQ ID NO:443 is a clone designated herein as "DNA287319".
Figure 444 shows the amino acid sequence (SEQ ID NO:444) derived from the coding sequence of SEQ ID NO:443 shown in Figure 443.
Figure 445 shows a nucleotide sequence (SEQ ID NO:445) of a native sequence PRO69585 cDNA, wherein SEQ ID NO:445 is a clone designated herein as "DNA287320".
Figure 446 shows the amino acid sequence (SEQ ID NO:446) derived from the coding sequence of SEQ ID NO:445 shown in Figure 445.
Figure 447 shows a nucleotide sequence (SEQ ID NO:447) of a native sequence PR069586 cDNA, wherein SEQ ID NO:447 is a clone designated herein as "DNA287321".
Figure 448 shows the amino acid sequence (SEQ ID NO:448) derived from the coding sequence of SEQ ID NO:447 shown in Figure 447.
Figure 449 shows a nucleotide sequence (SEQ ID NO:449) of a native sequence PR069587 cDNA, wherein SEQ ID NO:449 is a clone designated herein as "DNA287322".
Figure 450 shows the amino acid sequence (SEQ ID NO:450) derived from the coding sequence of SEQ ID NO:449 shown in Figure 449.
Figure 451 shows a nucleotide sequence (SEQ ID NO:451) of a native sequence PRO69588 cDNA, wherein SEQ ID NO:451 is a clone designated herein as "DNA287323".
Figure 452 shows the amino acid sequence (SEQ ID NO:452) derived from the coding sequence of SEQ ID NO:451 shown in Figure 451.
Figure 453 shows a nucleotide sequence (SEQ ID NO:453) of a native sequence PR069589 cDNA, wherein SEQ ID NO:453 is a clone designated herein as "DNA287637".
Figure 454 shows the amino acid sequence (SEQ ID NO:454) derived from the coding sequence of SEQ ID NO:453 shown in Figure 453.
Figure 455A-B shows a nucleotide sequence (SEQ ID NO:455A-B) of a native sequence PRO70021 cDNA, wherein SEQ ID NO:455A-B is a clone designated herein as "DNA288261".
Figure 456 shows the amino acid sequence (SEQ ID NO:456) derived from the coding sequence of SEQ ID NO:455A-B shown in Figure 455A-B.
Figure 457 shows a nucleotide sequence (SEQ ID NO:457) of a native sequence PR069590 cDNA, wherein SEQ ID NO:457 is a clone designated herein as "DNA288262".
Figure 458 shows the amino acid sequence (SEQ ID NO:458) derived from the coding sequence of SEQ ID NO:457 shown in Figure 457.
Figure 459 shows a nucleotide sequence (SEQ ID NO:459) of a native sequence PR070022 cDNA, wherein SEQ ID NO:459 is a clone designated herein as "DNA288263".
Figure 460 shows the amino acid sequence (SEQ ID NO:460) derived from the coding sequence of SEQ ID NO:459 shown in Figure 459.
Figure 461A-B shows a nucleotide sequence (SEQ ID NO:461A-B) of a native sequence PR069592 cDNA, wherein SEQ ID NO:461A-B is a clone designated herein as "DNA287327".
Figure 462 shows the amino acid sequence (SEQ ID NO:462) derived from the coding sequence of SEQ ID NO:461A-B shown in Figure 461A-B.
Figure 463 shows a nucleotide sequence (SEQ ID NO:463) of a native sequence PR037029 cDNA, wherein SEQ ID NO:463 is a clone designated herein as "DNA287328".
Figure 464 shows the amino acid sequence (SEQ ID NO:464) derived from the coding sequence of SEQ ID NO:463 shown in Figure 463.
Figure 465 shows a nucleotide sequence (SEQ ID NO:465) of a native sequence PR069593 cDNA, wherein SEQ ID NO:465 is a clone designated herein as "DNA287329".
Figure 466 shows the amino acid sequence (SEQ ID NO:466) derived from the coding sequence of SEQ ID NO:465 shown in Figure 465.
Figure 467A-B shows a nucleotide sequence (SEQ ID NO:467A-B) of a native sequence PR069594 cDNA, wherein SEQ ID NO:467A-B is a clone designated herein as "DNA287330".
Figure 468 shows the amino acid sequence (SEQ ID NO:468) derived from the coding sequence of SEQ ID NO:467A-B shown in Figure 467A-B.
Figure 469 shows a nucleotide sequence (SEQ ID NO:469) of a native sequence PR069595 cDNA, wherein SEQ ID NO:469 is a clone designated herein as "DNA287331".
Figure 470 shows the amino acid sequence (SEQ ID NO:470) derived from the coding sequence of SEQ ID NO:469 shown in Figure 469.
Figure 471 shows a nucleotide sequence (SEQ ID NO:471) of a native sequence PR01207 cDNA, wherein SEQ ID NO:471 is a clone designated herein as "DNA66480".
Figure 472 shows the amino acid sequence (SEQ ID NO:472) derived from the coding sequence of SEQ ID NO:471 shown in Figure 471.
Figure 473 shows a nucleotide sequence (SEQ ID NO:473) of a native sequence PRO69596 cDNA, wherein SEQ ID NO:473 is a clone designated herein as "DNA287332".
Figure 474 shows the amino acid sequence (SEQ ID NO:474) derived from the coding sequence of SEQ ID NO:473 shown in Figure 473.
Figure 475 shows a nucleotide sequence (SEQ ID NO:475) of a native sequence PR069597 cDNA, wherein SEQ ID NO:475 is a clone designated herein as "DNA287333".
Figure 476 shows the amino acid sequence (SEQ ID NO:476) derived from the coding sequence of SEQ ID NO:475 shown in Figure 475.
Figure 477 shows a nucleotide sequence (SEQ ID NO:477) of a native sequence PRO51139 cDNA, wherein SEQ ID NO:477 is a clone designated herein as "DNA256089".
Figure 478 shows the amino acid sequence (SEQ ID NO:478) derived from the coding sequence of SEQ ID NO:477 shown in Figure 477.
Figure 479 shows a nucleotide sequence (SEQ ID NO:479) of a native sequence PR062545 cDNA, wherein SEQ ID NO: 479 is a clone designated herein as "DNA274778".
Figure 480 shows the amino acid sequence (SEQ ID NO:480) derived from the coding sequence of SEQ ID NO:479 shown in Figure 479.
Figure 481 shows a nucleotide sequence (SEQ ID NO:481) of a native sequence PR03615 cDNA, wherein SEQ ID NO:481 is a clone designated herein as "DNA287334".
Figure 482 shows the amino acid sequence (SEQ ID NO:482) derived from the coding sequence of SEQ ID NO:481 shown in Figure 481.
Figure 483 shows a nucleotide sequence (SEQ ID NO:483) of a native sequence PR038036 cDNA, wherein SEQ ID NO:483 is a clone designated herein as "DNA227573".
Figure 484 shows the amino acid sequence (SEQ ID NO:484) derived from the coding sequence of SEQ ID NO:483 shown in Figure 483.
Figure 485 shows a nucleotide sequence (SEQ ID NO:485) of a native sequence PR069598 cDNA, wherein SEQ ID NO:485 is a clone designated herein as "DNA287335".
Figure 486 shows the amino acid sequence (SEQ ID NO:486) derived from the coding sequence of SEQ ID NO:485 shown in Figure 485.
Figure 487 shows a nucleotide sequence (SEQ ID NO:487) of a native sequence PR04701 cDNA, wherein SEQ ID NO:487 is a clone designated herein as "DNA103371".
Figure 488 shows the amino acid sequence (SEQ ID NO:488) derived from the coding sequence of SEQ ID NO:487 shown in Figure 487.
Figure 489 shows a nucleotide sequence (SEQ ID NO:489) of a native sequence PR069599 cDNA, wherein SEQ ID NO:489 is a clone designated herein as "DNA287336".
Figure 490 shows the amino acid sequence (SEQ ID NO:490) derived from the coding sequence of SEQ ID NO:489 shown in Figure 489.
Figure 491 shows a nucleotide sequence (SEQ ID NO:491) of a native sequence PR069600 cDNA, wherein SEQ ID NO:491 is a clone designated herein as "DNA287337".
Figure 492 shows the amino acid sequence (SEQ ID NO:492) derived from the coding sequence of SEQ ID NO:491 shown in Figure 491.
Figure 493 shows a nucleotide sequence (SEQ ID NO:493) of a native sequence PR069601 cDNA, wherein SEQ ID NO:493 is a clone designated herein as "DNA287338".

Figure 494 shows the amino acid sequence (SEQ ID NO:494) derived from the coding sequence of SEQ ID NO:493 shown in Figure 493.
Figure 495 shows a nucleotide sequence (SEQ ID NO:495) of a native sequence PR069887 cDNA, wherein SEQ ID NO:495 is a clone designated herein as "DNA287640".
Figure 496 shows the amino acid sequence (SEQ ID NO:496) derived from the coding sequence of SEQ ID NO:495 shown in Figure 495.
Figure 497 shows a nucleotide sequence (SEQ ID NO:497) of a native sequence PR069603 cDNA, wherein SEQ ID NO:497 is a clone designated herein as "DNA287340".
Figure 498 shows the amino acid sequence (SEQ ID NO:498) derived from the coding sequence of SEQ ID NO:497 shown in Figure 497.
Figure 499 shows a nucleotide sequence (SEQ ID NO:499) of a native sequence PR069604 cDNA, wherein SEQ ID NO:499 is a clone designated herein as "DNA287341".
Figure 500 shows the amino acid sequence (SEQ ID NO:500) derived from the coding sequence of SEQ ID NO:499 shown in Figure 499.
Figure 501 shows a nucleotide sequence (SEQ ID NO:501) of a native sequence PR070023 cDNA, wherein SEQ ID NO:501 is a clone designated herein as "DNA288264".
Figure 502 shows the amino acid sequence (SEQ ID NO:502) derived from the coding sequence of SEQ ID NO:501 shown in Figure 501.
Figure 503 shows a nucleotide sequence (SEQ ID NO:503) of a native sequence PR069606 cDNA, wherein SEQ ID NO:503 is a clone designated herein as "DNA287343".
Figure 504 shows the amino acid sequence (SEQ ID NO:504) derived from the coding sequence of SEQ ID NO:503 shown in Figure 503.
Figure 505 shows a nucleotide sequence (SEQ ID NO:505) of a native sequence PR069607 cDNA, wherein SEQ ID NO:505 is a clone designated herein as "DNA287344".
Figure 506 shows the amino acid sequence (SEQ ID NO:506) derived from the coding sequence of SEQ ID NO:505 shown in Figure 505.
Figure 507 shows a nucleotide sequence (SEQ ID NO:507) of a native sequence PR069608 cDNA, wherein SEQ ID NO:507 is a clone designated herein as "DNA287345".
Figure 508 shows the amino acid sequence (SEQ ID NO:508) derived from the coding sequence of SEQ ID NO:507 shown in Figure 507.
Figure 509 shows a nucleotide sequence (SEQ ID NO:509) of a native sequence PRO69609 cDNA, wherein SEQ ID NO:509 is a clone designated herein as "DNA287346".
Figure 510 shows the amino acid sequence (SEQ ID NO:510) derived from the coding sequence of SEQ ID NO:509 shown in Figure 509.
Figure 511 shows a nucleotide sequence (SEQ ID NO:511) of a native sequence PRO69610 cDNA, wherein SEQ ID NO:511 is a clone designated herein as "DNA287347".
Figure 512 shows the amino acid sequence (SEQ ID NO:512) derived from the coding sequence of SEQ ID NO:511 shown in Figure 511.
Figure 513 shows a nucleotide sequence (SEQ ID NO:513) of a native sequence PR09902 cDNA, wherein SEQ ID NO:513 is a clone designated herein as "DNA287642".
Figure 514 shows the amino acid sequence (SEQ ID NO:514) derived from the coding sequence of SEQ ID NO:513 shown in Figure 513.
Figure 515 shows a nucleotide sequence (SEQ ID NO:515) of a native sequence PR069611 cDNA, wherein SEQ ID NO:515 is a clone designated herein as "DNA287349".
Figure 516 shows the amino acid sequence (SEQ ID NO:516) derived from the coding sequence of SEQ ID NO:515 shown in Figure 515.
Figure 517 shows a nucleotide sequence (SEQ ID NO:517) of a native sequence PRO69612 cDNA, wherein SEQ ID NO:517 is a clone designated herein as "DNA287350".
Figure 518 shows the amino acid sequence (SEQ ID NO:518 derived from the coding sequence of SEQ ID NO:517 shown in Figure 517.
Figure 519 shows a nucleotide sequence (SEQ ID NO:519) of a native sequence PR069613 cDNA, wherein SEQ ID NO:519 is a clone designated herein as "DNA287351".
Figure 520 shows the amino acid sequence (SEQ ID NO:520) derived from the coding sequence of SEQ ID NO:519 shown in Figure 519.
Figure 521 shows a nucleotide sequence (SEQ ID NO:521) of a native sequence PR069614 cDNA, wherein SEQ ID NO:521 is a clone designated herein as "DNA287352".
Figure 522 shows the amino acid sequence (SEQ ID NO:522) derived from the coding sequence of SEQ ID NO:521 shown in Figure 521.
Figure 523 shows a nucleotide sequence (SEQ ID NO:523) of a native sequence PR069615 cDNA, wherein SEQ ID NO:523 is a clone designated herein as "DNA287643".
Figure 524 shows the amino acid sequence (SEQ ID NO:524) derived from the coding sequence of SEQ ID NO:523 shown in Figure 523.
Figure 525 shows a nucleotide sequence (SEQ ID NO:525) of a native sequence PRO70024 cDNA, wherein SEQ ID NO:525 is a clone designated herein as "DNA288265".
Figure 526 shows the amino acid sequence (SEQ ID NO:526) derived from the coding sequence of SEQ ID NO:525 shown in Figure 525.
Figure 527 shows a nucleotide sequence (SEQ ID NO:527) of a native sequence PR069616 cDNA, wherein SEQ ID NO:527 is a clone designated herein as "DNA287354".
Figure 528 shows the amino acid sequence (SEQ ID NO:528) derived from the coding sequence of SEQ ID NO:527 shown in Figure 527.
Figure 529 shows a nucleotide sequence (SEQ ID NO:529) of a native sequence PRO49619 cDNA, wherein SEQ ID NO:529 is a clone designated herein as "DNA254512".
Figure 530 shows the amino acid sequence (SEQ ID NO:530) derived from the coding sequence of SEQ ID NO:529 shown in Figure 529.
Figure 531 shows a nucleotide sequence (SEQ ID NO:531) of a native sequence PR069617 cDNA, wherein SEQ ID NO:531 is a clone designated herein as "DNA287355".
Figure 532 shows the amino acid sequence (SEQ ID NO:532) derived from the coding sequence of SEQ ID NO:531 shown in Figure 531.
Figure 533 shows a nucleotide sequence (SEQ ID NO:533) of a native sequence PRO69618 cDNA, wherein SEQ ID NO:533 is a clone designated herein as "DNA287356".
Figure 534 shows the amino acid sequence (SEQ ID NO:534) derived from the coding sequence of SEQ ID NO:533 shown in Figure 533.
Figure 535 shows a nucleotide sequence (SEQ ID NO:535) of a native sequence PR038040 cDNA, wherein SEQ ID NO:535 is a clone designated herein as "DNA227577".
Figure 536 shows the amino acid sequence (SEQ ID NO:536) derived from the coding sequence of SEQ ID NO:535 shown in Figure 535.
Figure 537 shows a nucleotide sequence (SEQ ID NO:537) of a native sequence PR069619 cDNA, wherein SEQ ID NO:537 is a clone designated herein as "DNA287357".
Figure 538 shows the amino acid sequence (SEQ ID NO:538) derived from the coding sequence of SEQ ID NO:537 shown in Figure 537.
Figure 539 shows a nucleotide sequence (SEQ ID NO:539) of a native sequence PR069620 cDNA, wherein SEQ ID NO:539 is a clone designated herein as "DNA287358".
Figure 540 shows the amino acid sequence (SEQ ID NO:540) derived from the coding sequence of SEQ ID NO:539 shown in Figure 539.
Figure 541 shows a nucleotide sequence (SEQ ID NO:541) of a native sequence PR069621 cDNA, wherein SEQ ID NO:541 is a clone designated herein as "DNA287359".
Figure 542 shows the amino acid sequence (SEQ ID NO:542) derived from the coding sequence of SEQ ID NO:541 shown in Figure 541.
Figure 543A-B shows a nucleotide sequence (SEQ ID NO:543A-B) of a native sequence PRO69622 cDNA, wherein SEQ ID NO:543A-B is a clone designated herein as "DNA287360".
Figure 544 shows the amino acid sequence (SEQ ID NO:544) derived from the coding sequence of SEQ ID NO:543A-B shown in Figure 543A-B.
Figure 545 shows a nucleotide sequence (SEQ ID NO:545) of a native sequence PR04401 cDNA, wherein SEQ ID NO:545 is a clone designated herein as "DNA287362".
Figure 546 shows the amino acid sequence (SEQ ID NO:546) derived from the coding sequence of SEQ ID NO:545 shown in Figure 545.
Figure 547 shows a nucleotide sequence (SEQ ID NO:547) of a native sequence PR070025 cDNA, wherein SEQ ID NO:547 is a clone designated herein as "DNA288266".
Figure 548 shows the amino acid sequence (SEQ ID NO:548) derived from the coding sequence of SEQ ID NO:547 shown in Figure 547.
Figure 549 shows a nucleotide sequence (SEQ ID NO:549) of a native sequence PR069625 cDNA, wherein SEQ ID NO:549 is a clone designated herein as "DNA287364".
Figure 550 shows the amino acid sequence (SEQ ID NO:550) derived from the coding sequence of SEQ ID NO:549 shown in Figure 549.
Figure 551 shows a nucleotide sequence (SEQ ID NO:551) of a native sequence PRO12025 cDNA, wherein SEQ ID NO:551 is a clone designated herein as "DNA288267".
Figure 552 shows the amino acid sequence (SEQ ID NO:552) derived from the coding sequence of SEQ ID NO:551 shown in Figure 551.
Figure 553 shows a nucleotide sequence (SEQ ID NO:553) of a native sequence PR070026 cDNA, wherein SEQ ID NO:553 is a clone designated herein as "DNA288268".
Figure 554 shows the amino acid sequence (SEQ ID NO:554) derived from the coding sequence of SEQ ID NO:553 shown in Figure 553.
Figure 555 shows a nucleotide sequence (SEQ ID NO:555) of a native sequence PR069627 cDNA, wherein SEQ ID NO:555 is a clone designated herein as "DNA287367".
Figure 556 shows the amino acid sequence (SEQ ID NO:556) derived from the coding sequence of SEQ ID NO:555 shown in Figure 555.
Figure 557 shows a nucleotide sequence (SEQ ID NO:557) of a native sequence PR069628 cDNA, wherein SEQ ID NO:557 is a clone designated herein as "DNA287368".
Figure 558 shows the amino acid sequence (SEQ ID NO:558) derived from the coding sequence of SEQ ID NO:557 shown in Figure 557.
Figure 559 shows a nucleotide sequence (SEQ ID NO:559) of a native sequence PR022637 cDNA, wherein SEQ ID NO:559 is a clone designated herein as "DNA189703".
Figure 560 shows the amino acid sequence (SEQ ID NO:560) derived from the coding sequence of SEQ ID NO:559 shown in Figure 559.
Figure 561A-B shows a nucleotide sequence (SEQ ID NO:561A-B) of a native sequence PR069629 cDNA, wherein SEQ ID NO:561A-B is a clone designated herein as "DNA287369".
Figure 562 shows the amino acid sequence (SEQ ID NO:562) derived from the coding sequence of SEQ ID NO:561A-B shown in Figure 561A-B.
Figure 563 shows a nucleotide sequence (SEQ ID NO:563) of a native sequence PR070027 cDNA, wherein SEQ ID NO:563 is a clone designated herein as "DNA288269".
Figure 564 shows the amino acid sequence (SEQ ID NO:564) derived from the coding sequence of SEQ ID NO:563 shown in Figure 563.
Figure 565 shows a nucleotide sequence (SEQ ID NO:565) of a native sequence PR070028 cDNA, wherein SEQ ID NO:565 is a clone designated herein as "DNA288270".
Figure 566 shows the amino acid sequence (SEQ ID NO:566) derived from the coding sequence of SEQ ID NO:565 shown in Figure 565.
Figure 567 shows a nucleotide sequence (SEQ ID NO:567) of a native sequence PR069632 cDNA, wherein SEQ ID NO:567 is a clone designated herein as "DNA287372".
Figure 568 shows the amino acid sequence (SEQ ID NO:568) derived from the coding sequence of SEQ ID NO:567 shown in Figure 567.
Figure 569 shows a nucleotide sequence (SEQ ID NO:569) of a native sequence PR069634 cDNA, wherein SEQ ID NO:569 is a clone designated herein as "DNA287374".
Figure 570 shows the amino acid sequence (SEQ ID NO:570) derived from the coding sequence of SEQ ID NO:569 shown in Figure 569.
Figure 571 shows a nucleotide sequence (SEQ ID NO:571) of a native sequence PR036857 cDNA, wherein SEQ ID NO:571 is a clone designated herein as "DNA226394".
Figure 572 shows the amino acid sequence (SEQ ID NO:572) derived from the coding sequence of SEQ ID NO:571 shown in Figure 571.
Figure 573 shows a nucleotide sequence (SEQ ID NO:573) of a native sequence PR069893 cDNA, wherein SEQ ID NO:573 is a clone designated herein as "DNA287648".
Figure 574 shows the amino acid sequence (SEQ ID NO:574) derived from the coding sequence of SEQ ID NO:573 shown in Figure 573.
Figure 575 shows a nucleotide sequence (SEQ ID NO:575) of a native sequence PRO69635 cDNA, wherein SEQ ID NO:575 is a clone designated herein as "DNA287375".
Figure 576 shows the amino acid sequence (SEQ ID NO:576) derived from the coding sequence of SEQ ID NO:575 shown in Figure 575.
Figure 577 shows a nucleotide sequence (SEQ ID NO:577) of a native sequence PR06180 cDNA, wherein SEQ ID NO:577 is a clone designated herein as "DNA287376".
Figure 578 shows the amino acid sequence (SEQ ID NO:578) derived from the coding sequence of SEQ ID NO:577 shown in Figure 577.
Figure 579 shows a nucleotide sequence (SEQ ID NO:579) of a native sequence PR069637 cDNA, wherein SEQ ID NO:579 is a clone designated herein as "DNA287378".
Figure 580 shows the amino acid sequence (SEQ ID NO:580) derived from the coding sequence of SEQ ID NO:579 shown in Figure 579.
Figure 581 shows a nucleotide sequence (SEQ ID NO:581) of a native sequence PRO69638 cDNA, wherein SEQ ID NO:581 is a clone designated herein as "DNA287379".
Figure 582 shows the amino acid sequence (SEQ ID NO:582) derived from the coding sequence of SEQ ID NO:581 shown in Figure 581.
Figure 583 shows a nucleotide sequence (SEQ ID NO:583) of a native sequence PR069639 cDNA, wherein SEQ ID NO:583 is a clone designated herein as "DNA287380".
Figure 584 shows the amino acid sequence (SEQ ID NO:584) derived from the coding sequence of SEQ ID NO:583 shown in Figure 583.
Figure 585 shows a nucleotide sequence (SEQ ID NO:585) of a native sequence PRO69640 cDNA, wherein SEQ ID NO:585 is a clone designated herein as "DNA287381".
Figure 586 shows the amino acid sequence (SEQ ID NO:586) derived from the coding sequence of SEQ ID NO:585 shown in Figure 585.
Figure 587 shows a nucleotide sequence (SEQ ID NO:587) of a native sequence PRO69641 cDNA, wherein SEQ ID NO:587 is a clone designated herein as "DNA287382".
Figure 588 shows the amino acid sequence (SEQ ID NO:588) derived from the coding sequence of SEQ ID NO:587 shown in Figure 587.
Figure 589 shows a nucleotide sequence (SEQ ID NO:589) of a native sequence PR062766 cDNA, wherein SEQ ID NO:589 is a clone designated herein as "DNA275043".
Figure 590 shows the amino acid sequence (SEQ ID NO:590) derived from the coding sequence of SEQ ID NO:599 shown in Figure 589.
Figure 591 shows a nucleotide sequence (SEQ ID NO:591) of a native sequence PR053782 cDNA, wherein SEQ ID NO:591 is a clone designated herein as "DNA287383".
Figure 592 shows the amino acid sequence (SEQ ID NO:592) derived from the coding sequence of SEQ ID NO:591 shown in Figure 591.
Figure 593 shows a nucleotide sequence (SEQ ID NO:593) of a native sequence PR061472 cDNA, wherein SEQ ID NO:593 is a clone designated herein as "DNA273489".
Figure 594 shows the amino acid sequence (SEQ ID NO:594) derived from the coding sequence of SEQ ID NO:593 shown in Figure 593.
Figure 595 shows a nucleotide sequence (SEQ ID NO:595) of a native sequence PR038179 cDNA, wherein SEQ ID NO:595 is a clone designated herein as "DNA227716".
Figure 596 shows the amino acid sequence (SEQ ID NO:596) derived from the coding sequence of SEQ ID NO:595 shown in Figure 595.
Figure 597 shows a nucleotide sequence (SEQ ID NO:597) of a native sequence PR069642 cDNA, wherein SEQ ID NO:597 is a clone designated herein as "DNA287384".
Figure 598 shows the amino acid sequence (SEQ ID NO:598) derived from the coding sequence of SEQ ID NO:597 shown in Figure 597.
Figure 599 shows a nucleotide sequence (SEQ ID NO:599) of a native sequence PR069643 cDNA, wherein SEQ ID NO:599 is a clone designated herein as "DNA287385".
Figure 600 shows the amino acid sequence (SEQ ID NO:600) derived from the coding sequence of SEQ ID NO:599 shown in Figure 599.
Figure 601 shows a nucleotide sequence (SEQ ID NO:601) of a native sequence PR069644 cDNA, wherein SEQ ID NO:601 is a clone designated herein as "DNA287386".
Figure 602 shows the amino acid sequence (SEQ ID NO:602) derived from the coding sequence of SEQ ID NO:601 shown in Figure 601.
Figure 603 shows a nucleotide sequence (SEQ ID NO:603) of a native sequence PRO69645 cDNA, wherein SEQ ID NO:603 is a clone designated herein as "DNA287387".
Figure 604 shows the amino acid sequence (SEQ ID NO:604) derived from the coding sequence of SEQ ID NO:603 shown in Figure 603.
Figure 605 shows a nucleotide sequence (SEQ ID NO:605) of a native sequence PRO11608 cDNA, wherein SEQ ID NO:605 is a clone designated herein as "DNA151077".
Figure 606 shows the amino acid sequence (SEQ ID NO:606) derived from the coding sequence of SEQ ID NO:605 shown in Figure 605.
Figure 607 shows a nucleotide sequence (SEQ ID NO:607) of a native sequence PR069646 cDNA, wherein SEQ ID NO:607 is a clone designated herein as "DNA287388".
Figure 608 shows the amino acid sequence (SEQ ID NO:608) derived from the coding sequence of SEQ ID NO:607 shown in Figure 607.
Figure 609 shows a nucleotide sequence (SEQ ID NO:609) of a native sequence PR059825 cDNA, wherein SEQ ID NO:609 is a clone designated herein as "DNA271536".
Figure 610 shows the amino acid sequence (SEQ ID NO:610) derived from the coding sequence of SEQ ID NO:609 shown in Figure 609.
Figure 611 shows a nucleotide sequence (SEQ ID NO:611) of a native sequence PR069647 cDNA, wherein SEQ ID NO:611 is a clone designated herein as "DNA287389".
Figure 612 shows the amino acid sequence (SEQ ID NO:612) derived from the coding sequence of SEQ ID NO:611 shown in Figure 611.
Figure 613 shows a nucleotide sequence (SEQ ID NO:613) of a native sequence PRO69648 cDNA, wherein SEQ ID NO:613 is a clone designated herein as "DNA287390".
Figure 614 shows the amino acid sequence (SEQ ID NO:614) derived from the coding sequence of SEQ ID NO:613 shown in Figure 613.
Figure 615 shows a nucleotide sequence (SEQ ID NO:615) of a native sequence PR070029 cDNA, wherein SEQ ID NO:615 is a clone designated herein as "DNA288271".
Figure 616 shows the amino acid sequence (SEQ ID NO:616) derived from the coding sequence of SEQ ID NO:615 shown in Figure 615.
Figure 617 shows a nucleotide sequence (SEQ ID NO:617) of a native sequence PRO1213 cDNA, wherein SEQ ID NO:617 is a clone designated herein as "DNA66487".
Figure 618 shows the amino acid sequence (SEQ ID NO:618) derived from the coding sequence of SEQ ID NO:617 shown in Figure 617.
Figure 619 shows a nucleotide sequence (SEQ ID NO:619) of a native sequence PR070030 cDNA, wherein SEQ ID NO:619 is a clone designated herein as "DNA288272".
Figure 620 shows the amino acid sequence (SEQ ID NO:620) derived from the coding sequence of SEQ ID NO:619 shown in Figure 619.
Figure 621 shows a nucleotide sequence (SEQ ID NO:621) of a native sequence PR050195 cDNA, wherein SEQ ID NO:621 is a clone designated herein as "DNA255113".
Figure 622 shows the amino acid sequence (SEQ ID NO:622) derived from the coding sequence of SEQ ID NO:621 shown in Figure 621.
Figure 623 shows a nucleotide sequence (SEQ ID NO:623) of a native sequence PRO69651 cDNA, wherein SEQ ID NO:623 is a clone designated herein as "DNA287393".
Figure 624 shows the amino acid sequence (SEQ ID NO:624) derived from the coding sequence of SEQ ID NO:623 shown in Figure 623.
Figure 625A-B shows a nucleotide sequence (SEQ ID NO:625A-B) of a native sequence PR037538 cDNA, wherein SEQ ID NO:625A-B is a clone designated herein as "DNA227075".
Figure 626 shows the amino acid sequence (SEQ ID NO:626) derived from the coding sequence of SEQ ID NO:625A-B shown in Figure 625A-B.
Figure 627 shows a nucleotide sequence (SEQ ID NO:627) of a native sequence PR069652 cDNA, wherein SEQ ID NO:627 is a clone designated herein as "DNA287394".
Figure 628 shows the amino acid sequence (SEQ ID NO:628) derived from the coding sequence of SEQ ID NO:627 shown in Figure 627.
Figure 629 shows a nucleotide sequence (SEQ ID NO:629) of a native sequence PR059210 cDNA, wherein SEQ ID NO:629 is a clone designated herein as "DNA270875".
Figure 630 shows the amino acid sequence (SEQ ID NO:630) derived from the coding sequence of SEQ ID NO:629 shown in Figure 629.
Figure 631 shows a nucleotide sequence (SEQ ID NO:631) of a native sequence PR023374 cDNA, wherein SEQ ID NO:631 is a clone designated herein as "DNA193967".
Figure 632 shows the amino acid sequence (SEQ ID NO:632) derived from the coding sequence of SEQ ID NO:631 shown in Figure 631.
Figure 633 shows a nucleotide sequence (SEQ ID NO:633) of a native sequence PR024844 cDNA, wherein SEQ ID NO:633 is a clone designated herein as "DNA288273".
Figure 634 shows the amino acid sequence (SEQ ID NO:634) derived from the coding sequence of SEQ ID NO:633 shown in Figure 633.
Figure 635 shows a nucleotide sequence (SEQ ID NO:635) of a native sequence PR070031 cDNA, wherein SEQ ID NO:635 is a clone designated herein as "DNA288274".
Figure 636 shows the amino acid sequence (SEQ ID NO:636) derived from the coding sequence of SEQ ID NO:635 shown in Figure 635.
Figure 637 shows a nucleotide sequence (SEQ ID NO:637) of a native sequence PR069653 cDNA, wherein SEQ ID NO:637 is a clone designated herein as "DNA287396".
Figure 638 shows the amino acid sequence (SEQ ID NO:638) derived from the coding sequence of SEQ ID NO:637 shown in Figure 637.
Figure 639 shows a nucleotide sequence (SEQ ID NO:639) of a native sequence PR069654 cDNA, wherein SEQ ID NO:639 is a clone designated herein as "DNA287397".
Figure 640 shows the amino acid sequence (SEQ ID NO:640) derived from the coding sequence of SEQ ID NO:639 shown in Figure 639.
Figure 641 shows a nucleotide sequence (SEQ ID NO:641) of a native sequence PR069655 cDNA, wherein SEQ ID NO:641 is a clone designated herein as "DNA287398".
Figure 642 shows the amino acid sequence (SEQ ID NO:642) derived from the coding sequence of SEQ ID NO:641 shown in Figure 641.
Figure 643 shows a nucleotide sequence (SEQ ID NO:643) of a native sequence PR069656 cDNA, wherein SEQ ID NO:643 is a clone designated herein as "DNA287399".
Figure 644 shows the amino acid sequence (SEQ ID NO:644) derived from the coding sequence of SEQ ID NO:643 shown in Figure 643.
Figure 645 shows a nucleotide sequence (SEQ ID NO:645) of a native sequence PRO70032 cDNA, wherein SEQ ID NO:645 is a clone designated herein as "DNA288275".
Figure 646 shows the amino acid sequence (SEQ ID NO:646) derived from the coding sequence of SEQ ID NO:645 shown in Figure 645.
Figure 647 shows a nucleotide sequence (SEQ ID NO:647) of a native sequence PR069659 cDNA, wherein SEQ ID NO:647 is a clone designated herein as "DNA287402".
Figure 648 shows the amino acid sequence (SEQ ID NO:648) derived from the coding sequence of SEQ ID NO:647 shown in Figure 647.
Figure 649 shows a nucleotide sequence (SEQ ID NO:649) of a native sequence PR069660 cDNA, wherein SEQ ID NO:649 is a clone designated herein as "DNA287403".
Figure 650 shows the amino acid sequence (SEQ ID NO:650) derived from the coding sequence of SEQ ID NO:649 shown in Figure 649.
Figure 651A-B shows a nucleotide sequence (SEQ ID NO:651A-B) of a native sequence PR058054 cDNA, wherein SEQ ID NO:651A-B is a clone designated herein as "DNA269642".
Figure 652 shows the amino acid sequence (SEQ ID NO:652) derived from the coding sequence of SEQ ID NO:651A-B shown in Figure 651A-B.
Figure 653 shows a nucleotide sequence (SEQ ID NO:653) of a native sequence PR069661 cDNA, wherein SEQ ID NO:653 is a clone designated herein as "DNA287404".
Figure 654 shows the amino acid sequence (SEQ ID NO:654) derived from the coding sequence of SEQ ID NO:653 shown in Figure 653.
Figure 655 shows a nucleotide sequence (SEQ ID NO:655) of a native sequence PR069662 cDNA, wherein SEQ ID NO:655 is a clone designated herein as "DNA287405".
Figure 656 shows the amino acid sequence (SEQ ID NO:656) derived from the coding sequence of SEQ ID NO:655 shown in Figure 655.
Figure 657 shows a nucleotide sequence (SEQ ID NO:657) of a native sequence PR069898 cDNA, wherein SEQ ID NO:657 is a clone designated herein as "DNA287653".
Figure 658 shows the amino acid sequence (SEQ ID NO:658) derived from the coding sequence of SEQ ID NO:657 shown in Figure 657.
Figure 659 shows a nucleotide sequence (SEQ ID NO:659) of a native sequence PRO69664 cDNA, wherein SEQ ID NO:659 is a clone designated herein as "DNA287407".
Figure 660 shows the amino acid sequence (SEQ ID NO:660) derived from the coding sequence of SEQ ID NO:659 shown in Figure 659.
Figure 661 shows a nucleotide sequence (SEQ ID NO:661) of a native sequence PR069665 cDNA, wherein SEQ ID NO:661 is a clone designated herein as "DNA287408".
Figure 662 shows the amino acid sequence (SEQ ID NO:662) derived from the coding sequence of SEQ ID NO:661 shown in Figure 661.
Figure 663 shows a nucleotide sequence (SEQ ID NO:663) of a native sequence PR069666 cDNA, wherein SEQ ID NO:663 is a clone designated herein as "DNA287409".
Figure 664 shows the amino acid sequence (SEQ ID NO:664) derived from the coding sequence of SEQ ID NO:663 shown in Figure 663.
Figure 665 shows a nucleotide sequence (SEQ ID NO:665) of a native sequence PR069667 cDNA, wherein SEQ ID NO:665 is a clone designated herein as "DNA287410".
Figure 666 shows the amino acid sequence (SEQ ID NO:666) derived from the coding sequence of SEQ ID NO:665 shown in Figure 665.
Figure 667 shows a nucleotide sequence (SEQ ID NO:667) of a native sequence PR069669 cDNA, wherein SEQ ID NO:667 is a clone designated herein as "DNA287412".
Figure 668 shows the amino acid sequence (SEQ ID NO:668) derived from the coding sequence of SEQ ID NO:667 shown in Figure 667.
Figure 669 shows a nucleotide sequence (SEQ ID NO:669) of a native sequence PRO69671 cDNA, wherein SEQ ID NO:669 is a clone designated herein as "DNA287414".
Figure 670 shows the amino acid sequence (SEQ ID NO:670) derived from the coding sequence of SEQ ID NO:669 shown in Figure 669.
Figure 671 shows a nucleotide sequence (SEQ ID NO:671) of a native sequence PR069672 cDNA, wherein SEQ ID NO:671 is a clone designated herein as "DNA287415".
Figure 672 shows the amino acid sequence (SEQ ID NO:672) derived from the coding sequence of SEQ ID NO:671 shown in Figure 671.
Figure 673A-B shows a nucleotide sequence (SEQ ID NO:673A-B) of a native sequence PR058204 cDNA, wherein SEQ ID NO:673A-B is a clone designated herein as "DNA269799".
Figure 674 shows the amino acid sequence (SEQ ID NO:674) derived from the coding sequence of SEQ ID NO:673A-B shown in Figure 673A-B.
Figure 675 shows a nucleotide sequence (SEQ ID NO:675) of a native sequence PRO49419 cDNA, wherein SEQ ID NO:675 is a clone designated herein as "DNA254308".
Figure 676 shows the amino acid sequence (SEQ ID NO:676) derived from the coding sequence of SEQ ID NO:675 shown in Figure 675.
Figure 677 shows a nucleotide sequence (SEQ ID NO:677) of a native sequence PR069673 cDNA, wherein SEQ ID NO:677 is a clone designated herein as "DNA287416".
Figure 678 shows the amino acid sequence (SEQ ID NO:678) derived from the coding sequence of SEQ ID NO:677 shown in Figure 677.
Figure 679 shows a nucleotide sequence (SEQ ID NO:679) of a native sequence PR069674 cDNA, wherein SEQ ID NO:679 is a clone designated herein as "DNA287417".
Figure 680 shows the amino acid sequence (SEQ ID NO:680) derived from the coding sequence of SEQ ID NO:679 shown in Figure 679.
Figure 681 shows a nucleotide sequence (SEQ ID NO:681) of a native sequence PR049810 cDNA, wherein SEQ ID NO:681 is a clone designated herein as "DNA254710".
Figure 682 shows the amino acid sequence (SEQ ID NO:682) derived from the coding sequence of SEQ ID NO:681 shown in Figure 681.
Figure 683 shows a nucleotide sequence (SEQ ID NO:683) of a native sequence PR070033 cDNA, wherein SEQ ID NO:683 is a clone designated herein as "DNA288276".
Figure 684 shows the amino acid sequence (SEQ ID NO:684) derived from the coding sequence of SEQ ID NO:683 shown in Figure 683.
Figure 685 shows a nucleotide sequence (SEQ ID NO:685) of a native sequence PR069676 cDNA, wherein SEQ ID NO:695 is a clone designated herein as "DNA287419".
Figure 686 shows the amino acid sequence (SEQ ID NO:686) derived from the coding sequence of SEQ ID NO:685 shown in Figure 685.
Figure 687 shows a nucleotide sequence (SEQ ID NO:687) of a native sequence PR058076 cDNA, wherein SEQ ID NO:687 is a clone designated herein as "DNA269665".
Figure 688 shows the amino acid sequence (SEQ ID NO:688) derived from the coding sequence of SEQ ID NO:687 shown in Figure 687.
Figure 689 shows a nucleotide sequence (SEQ ID NO:689) of a native sequence PR069677 cDNA, wherein SEQ ID NO:689 is a clone designated herein as "DNA287420".
Figure 690 shows the amino acid sequence (SEQ ID NO:690) derived from the coding sequence of SEQ ID NO:689 shown in Figure 689.
Figure 691 shows a nucleotide sequence (SEQ ID NO:691) of a native sequence PR069678 cDNA, wherein SEQ ID NO:691 is a clone designated herein as "DNA287421".
Figure 692 shows the amino acid sequence (SEQ ID NO:692) derived from the coding sequence of SEQ ID NO:691 shown in Figure 691.
Figure 693 shows a nucleotide sequence (SEQ ID NO:693) of a native sequence PR069679 cDNA, wherein SEQ ID NO:693 is a clone designated herein as "DNA287422".
Figure 694 shows the amino acid sequence (SEQ ID NO:694) derived from the coding sequence of SEQ ID NO:693 shown in Figure 693.
Figure 695 shows a nucleotide sequence (SEQ ID NO:695) of a native sequence PRO1718 cDNA, wherein SEQ ID NO:695 is a clone designated herein as "DNA82362".
Figure 696 shows the amino acid sequence (SEQ ID NO:696) derived from the coding sequence of SEQ ID NO:695 shown in Figure 695.
Figure 697 shows a nucleotide sequence (SEQ ID NO:697) of a native sequence PRO51161 cDNA, wherein SEQ ID NO:697 is a clone designated herein as "DNA256112".
Figure 698 shows the amino acid sequence (SEQ ID NO:698) derived from the coding sequence of SEQ ID NO:697 shown in Figure 697.
Figure 699 shows a nucleotide sequence (SEQ ID NO:699) of a native sequence PRO69680 cDNA, wherein SEQ ID NO:699 is a clone designated herein as "DNA287423".
Figure 700 shows the amino acid sequence (SEQ ID NO:700) derived from the coding sequence of SEQ ID NO:699 shown in Figure 699.
Figure 701 shows a nucleotide sequence (SEQ ID NO:701) of a native sequence PRO59281 cDNA, wherein SEQ ID NO:701 is a clone designated herein as "DNA270950".
Figure 702 shows the amino acid sequence (SEQ ID NO:702) derived from the coding sequence of SEQ ID NO:701 shown in Figure 701.
Figure 703 shows a nucleotide sequence (SEQ ID NO:703) of a native sequence PR036102 cDNA, wherein SEQ ID NO:703 is a clone designated herein as "DNA225639".
Figure 704 shows the amino acid sequence (SEQ ID NO:704) derived from the coding sequence of SEQ ID NO:703 shown in Figure 703.
Figure 705 shows a nucleotide sequence (SEQ ID NO:705) of a native sequence PR061799 cDNA, wherein SEQ ID NO:705 is a clone designated herein as "DNA273839".
Figure 706 shows the amino acid sequence (SEQ ID NO:706) derived from the coding sequence of SEQ ID NO:705 shown in Figure 705.
Figure 707 shows a nucleotide sequence (SEQ ID NO:707) of a native sequence PRO69681 cDNA, wherein SEQ ID NO:707 is a clone designated herein as "DNA287424 ".
Figure 708 shows the amino acid sequence (SEQ ID NO:708) derived from the coding sequence of SEQ ID NO:707 shown in Figure 707.
Figure 709 shows a nucleotide sequence (SEQ ID NO:709) of a native sequence PRO69682 cDNA, wherein SEQ ID NO:709 is a clone designated herein as "DNA287425".
Figure 710 shows the amino acid sequence (SEQ ID NO:710) derived from the coding sequence of SEQ ID NO:710 shown in Figure 710.
Figure 711 shows a nucleotide sequence (SEQ ID NO:711) of a native sequence PR069901 cDNA, wherein SEQ ID NO:711 is a clone designated herein as "DNA287656".
Figure 712 shows the amino acid sequence (SEQ ID NO:712) derived from the coding sequence of SEQ ID NO:711 shown in Figure 711.
Figure 713 shows a nucleotide sequence (SEQ ID NO:713) of a native sequence PRO69684 cDNA, wherein SEQ ID NO:713 is a clone designated herein as "DNA287427".
Figure 714 shows the amino acid sequence (SEQ ID NO:714) derived from the coding sequence of SEQ ID NO:713 shown in Figure 713.
Figure 715 shows a nucleotide sequence (SEQ ID NO:715) of a native sequence PR069685 cDNA, wherein SEQ ID NO:715 is a clone designated herein as "DNA287428".
Figure 716 shows the amino acid sequence (SEQ ID NO:716) derived from the coding sequence of SEQ ID NO:715 shown in Figure 715.
Figure 717 shows a nucleotide sequence (SEQ ID NO:717) of a native sequence PRO69686 cDNA, wherein SEQ ID NO:717 is a clone designated herein as "DNA287429".
Figure 718 shows the amino acid sequence (SEQ ID NO:718) derived from the coding sequence of SEQ ID NO:717 shown in Figure 717.
Figure 719 shows a nucleotide sequence (SEQ ID NO:719) of a native sequence PR069687 cDNA, wherein SEQ ID NO:719 is a clone designated herein as "DNA287430".
Figure 720 shows the amino acid sequence (SEQ ID NO:720) derived from the coding sequence of SEQ ID NO:719 shown in Figure 719.
Figure 721 shows a nucleotide sequence (SEQ ID NO:721) of a native sequence PR038469 cDNA, wherein SEQ ID NO:721 is a clone designated herein as "DNA228006".
Figure 722 shows the amino acid sequence (SEQ ID NO:722) derived from the coding sequence of SEQ ID NO:721 shown in Figure 721.
Figure 723 shows a nucleotide sequence (SEQ ID NO:723) of a native sequence PR069688 cDNA, wherein SEQ ID NO:723 is a clone designated herein as "DNA287657".
Figure 724 shows the amino acid sequence (SEQ ID NO:724) derived from the coding sequence of SEQ ID NO:723 shown in Figure 723.
Figure 725 shows a nucleotide sequence (SEQ ID NO:725) of a native sequence PR070034 cDNA, wherein SEQ ID NO:725 is a clone designated herein as "DNA288277".
Figure 726 shows the amino acid sequence (SEQ ID NO:726) derived from the coding sequence of SEQ ID NO:725 shown in Figure 725.
Figure 727 shows a nucleotide sequence (SEQ ID NO:727) of a native sequence PR059354 cDNA, wherein SEQ ID NO:727 is a clone designated herein as "DNA271026".
Figure 728 shows the amino acid sequence (SEQ ID NO:728) derived from the coding sequence of SEQ ID NO:727 shown in Figure 727.
Figure 729 shows a nucleotide sequence (SEQ ID NO:729) of a native sequence PRO59189 cDNA, wherein SEQ ID NO:729 is a clone designated herein as "DNA270851".
Figure 730 shows the amino acid sequence (SEQ ID NO:730) derived from the coding sequence of SEQ ID NO:729 shown in Figure 729.
Figure 731 shows a nucleotide sequence (SEQ ID NO:731) of a native sequence PR038197 cDNA, wherein SEQ ID NO:731 is a clone designated herein as "DNA227734".
Figure 732 shows the amino acid sequence (SEQ ID NO:732) derived from the coding sequence of SEQ ID NO:731 shown in Figure 731.
Figure 733 shows a nucleotide sequence (SEQ ID NO:733) of a native sequence PR069902 cDNA, wherein SEQ ID NO:733 is a clone designated herein as "DNA287658".
Figure 734 shows the amino acid sequence (SEQ ID NO:734) derived from the coding sequence of SEQ ID NO:733 shown in Figure 733.
Figure 735 shows a nucleotide sequence (SEQ ID NO:735) of a native sequence PR069690 cDNA, wherein SEQ ID NO:735 is a clone designated herein as "DNA287433".
Figure 736 shows the amino acid sequence (SEQ ID NO:736) derived from the coding sequence of SEQ ID NO:735 shown in Figure 735.
Figure 737A-B shows a nucleotide sequence (SEQ ID NO:737A-B) of a native sequence PR061569 cDNA, wherein SEQ ID NO:737A-B is a clone designated herein as "DNA273593".
Figure 738 shows the amino acid sequence (SEQ ID NO:738) derived from the coding sequence of SEQ ID NO:737A-B shown in Figure 737A-B.
Figure 739 shows a nucleotide sequence (SEQ ID NO:739) of a native sequence PR069903 cDNA, wherein SEQ ID NO:739 is a clone designated herein as "DNA287659".
Figure 740 shows the amino acid sequence (SEQ ID NO:740) derived from the coding sequence of SEQ ID NO:739 shown in Figure 739.
Figure 741 shows a nucleotide sequence (SEQ ID NO:741) of a native sequence PRO1970 cDNA, wherein SEQ ID NO:741 is a clone designated herein as "DNA287434".
Figure 742 shows the amino acid sequence (SEQ ID NO:742) derived from the coding sequence of SEQ ID NO:741 shown in Figure 741.

The sequences identified as designated Figures with the corresponding SEQ ID NOs are shown in the form of a sequence listing, indicating the SEQ ID NOs, in the description.

### DETAILED DESCRIPTION OF THE PREFERRED EMBODIMENTS

### 1. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (*e.g.*, an extracellular domain sequence), naturally-occurring variant forms (*e.g.*, alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues.

Unless specifically stated otherwise, all % amino acid sequence identity values used herein are obtained as described in the immediately preceding paragraph using the ALIGN-2 computer program. However, % amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % amino acid sequence identity value is determined by dividing (a) the number of matching identical amino acid residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

Percent amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

"PRO variant polynucleotide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides.

Unless specifically stated otherwise, all % nucleic acid sequence identity values used herein are obtained as described in the immediately preceding paragraph using the ALIGN-2 computer program. However, % nucleic acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

Percent nucleic acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In other embodiments, PRO variant polynucleotides are nucleic acid molecules that encode an active PRO polypeptide and which are capable of hybridizing, preferably under stringent hybridization and wash conditions, to nucleotide sequences encoding a full-length PRO polypeptide as disclosed herein. PRO variant polypeptides may be those that are encoded by a PRO variant polynucleotide.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide in situ within recombinant cells, since at least one component of the PRO polypeptide natural environment will not be present. Ordinarily, however, isolated polypeptide will be prepared by at least one purification step.

An "isolated" PRO polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist, antagonist, and neutralizing antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of a PRO polypeptide which retain a biological and/or an immunological activity of native or naturally-occurring PRO, wherein "biological" activity refers to a biological function (either inhibitory or stimulatory) caused by a native or naturally-occurring PRO other than the ability to induce the production of an antibody against an antigenic epitope possessed by a native or naturally-occurring PRO and an "immunological" activity refers to the ability to induce the production of an antibody against an antigenic epitope possessed by a native or naturally-occurring PRO.

The term "antagonist" is used in the broadest sense, and includes any molecule that partially or fully blocks, inhibits, or neutralizes a biological activity of a native PRO polypeptide disclosed herein. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments or amino acid sequence variants of native PRO polypeptides, peptides, antisense oligonucleotides, small organic molecules, etc. Methods for identifying agonists or antagonists of a PRO polypeptide may comprise contacting a PRO polypeptide with a candidate agonist or antagonist molecule and measuring a detectable change in one or more biological activities normally associated with the PRO polypeptide.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')₂ fragment that has two antigen-combining sites and is still capable of cross-linking antigen.

"Fv" is the minimum antibody fragment which contains a complete antigen-recognition and - binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, *etc.*

The term "T cell mediated disease" means a disease in which T cells directly or indirectly mediate or otherwise contribute to a morbidity in a mammal. The T cell mediated disease may be associated with cell mediated effects, lymphokine mediated effects, *etc.*, and even effects associated with B cells if the B cells are stimulated, for example, by the lymphokines secreted by T cells.

Examples of immune-related and inflammatory diseases, some of which are immune or T cell mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease. Infectious diseases including viral diseases such as AIDS (HIV infection), hepatitis A, B, C, D, and E, herpes, *etc.*, bacterial infections, fungal infections, protozoal infections and parasitic infections.

The term "effective amount" is a concentration or amount of a PRO polypeptide and/or agonist/antagonist which results in achieving a particular stated purpose. An "effective amount" of a PRO polypeptide or agonist or antagonist thereof may be determined empirically. Furthermore, a "therapeutically effective amount" is a concentration or amount of a PRO polypeptide and/or agonist/antagonist which is effective for achieving a stated therapeutic effect. This amount may also be determined empirically.

The term "cytotoxic agent" as used herein refers to a substance that inhibits or prevents the function of cells and/or causes destruction of cells. The term is intended to include radioactive isotopes (*e.g.*, I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, *e.g.*, paclitaxel (Taxol, Bristol-Myers Squibb Oncology, Princeton, NJ), and doxetaxel (Taxotere, Rhône-Poulenc Rorer, Antony, France), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in vivo*. Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i.e.*, is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

As used herein, the term "inflammatory cells" designates cells that enhance the inflammatory response such as mononuclear cells, eosinophils, macrophages, and polymorphonuclear neutrophils (PMN).

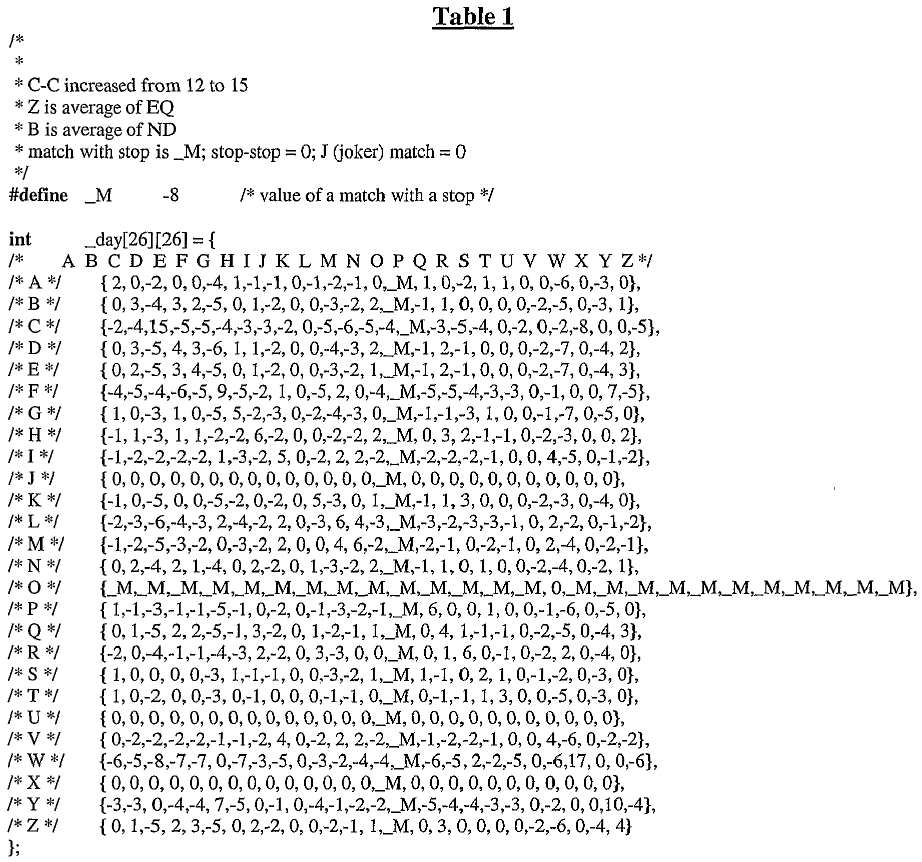

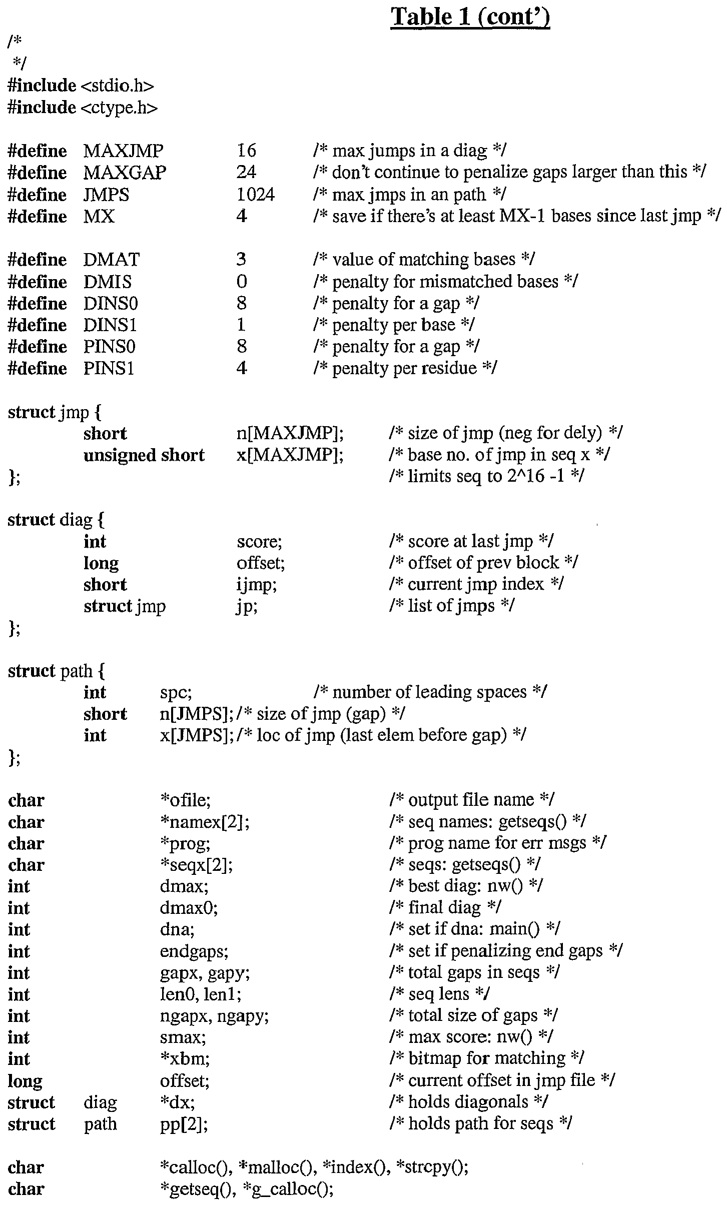

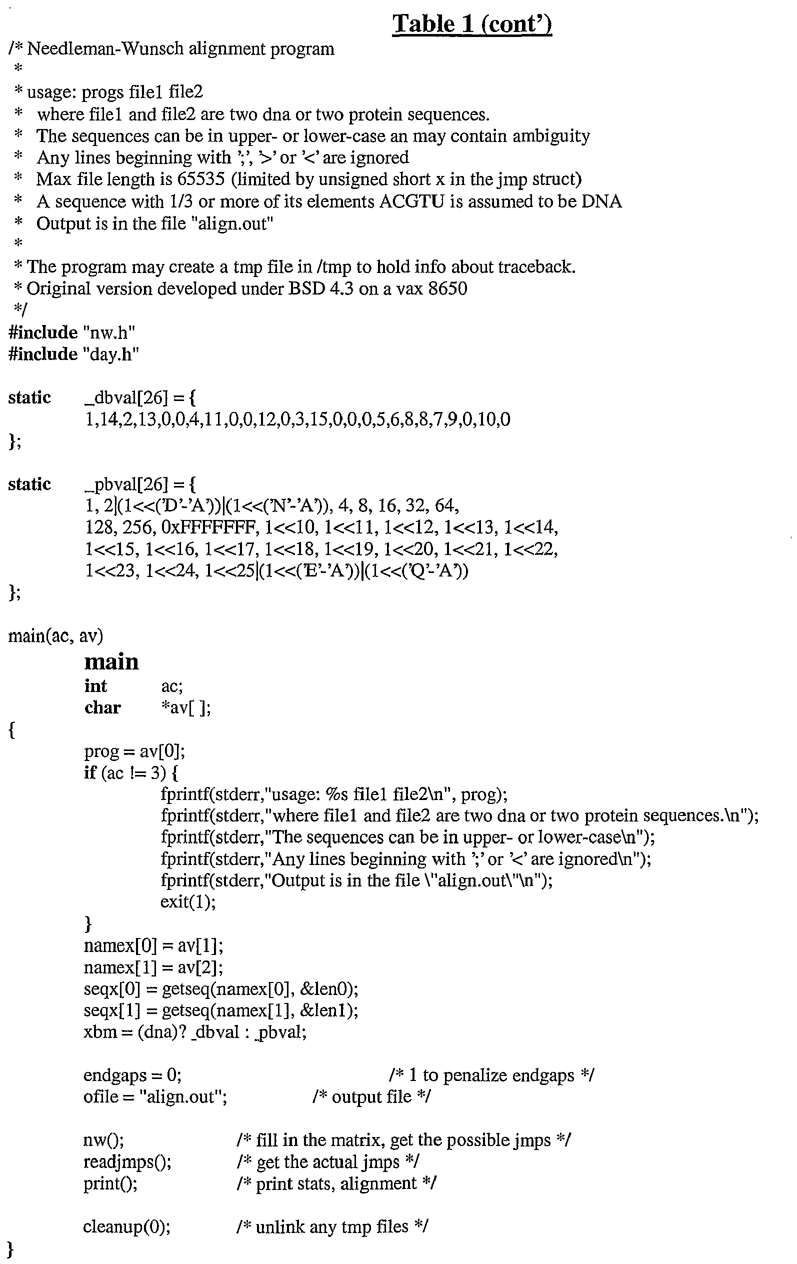

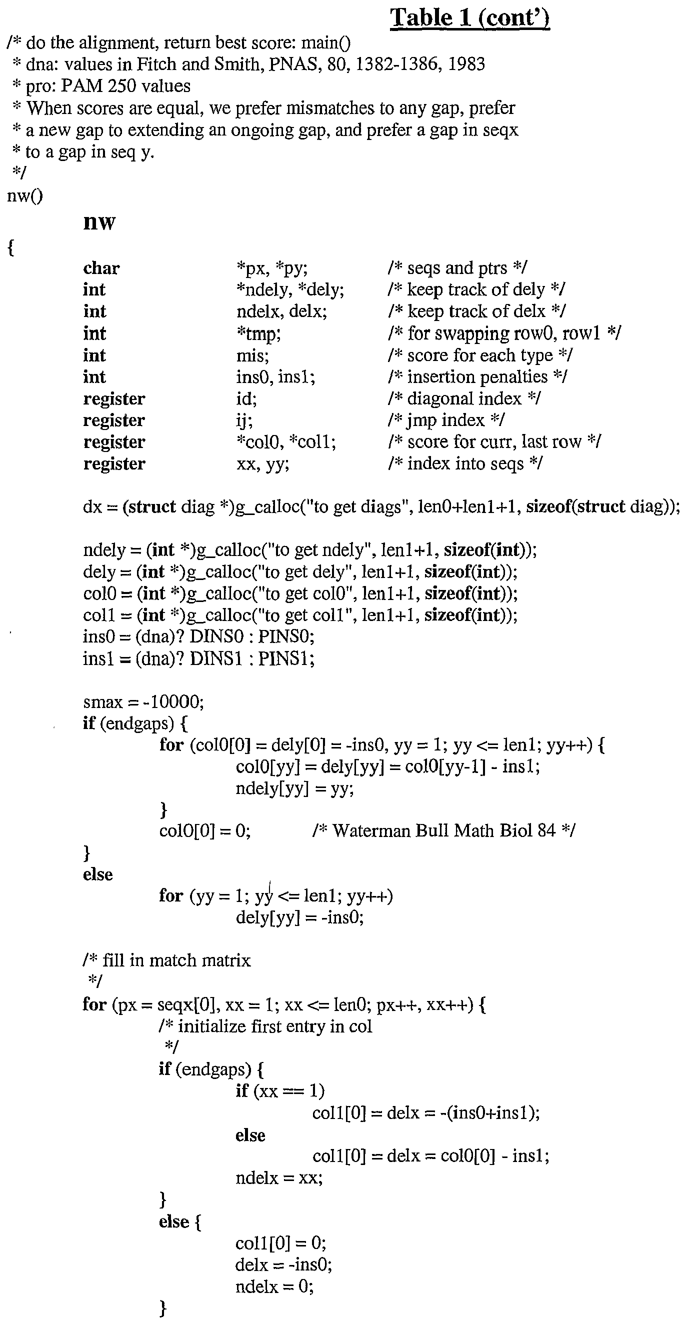

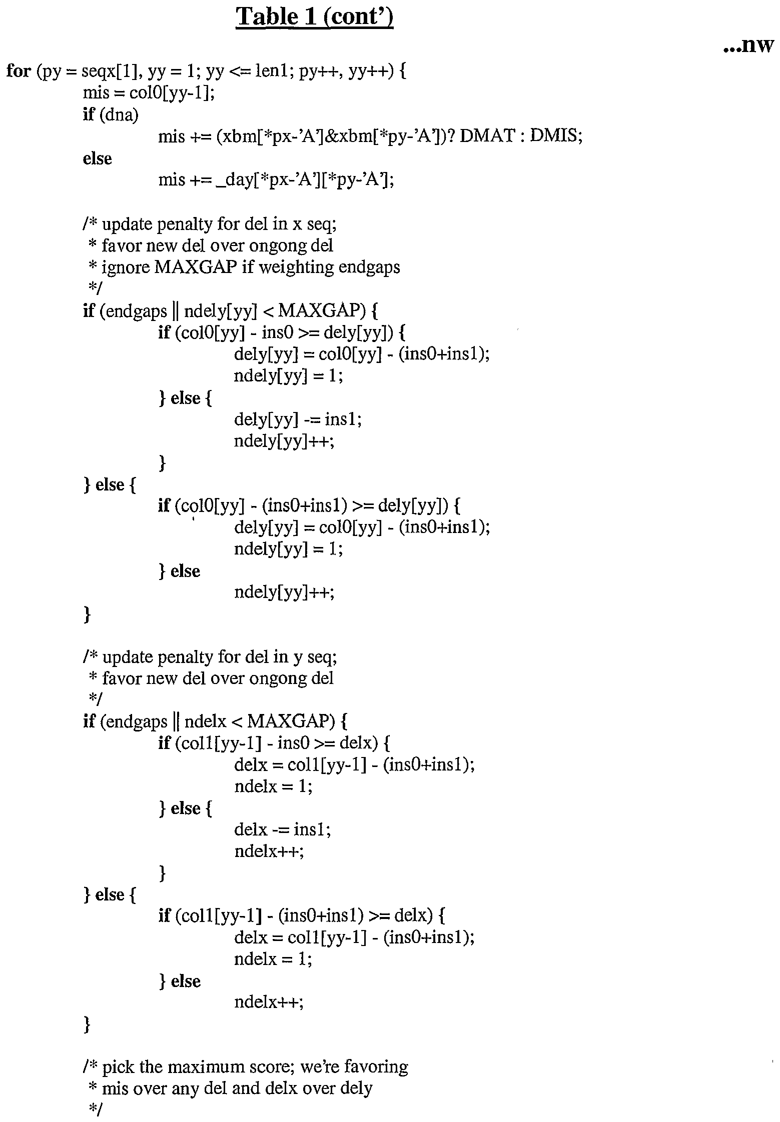

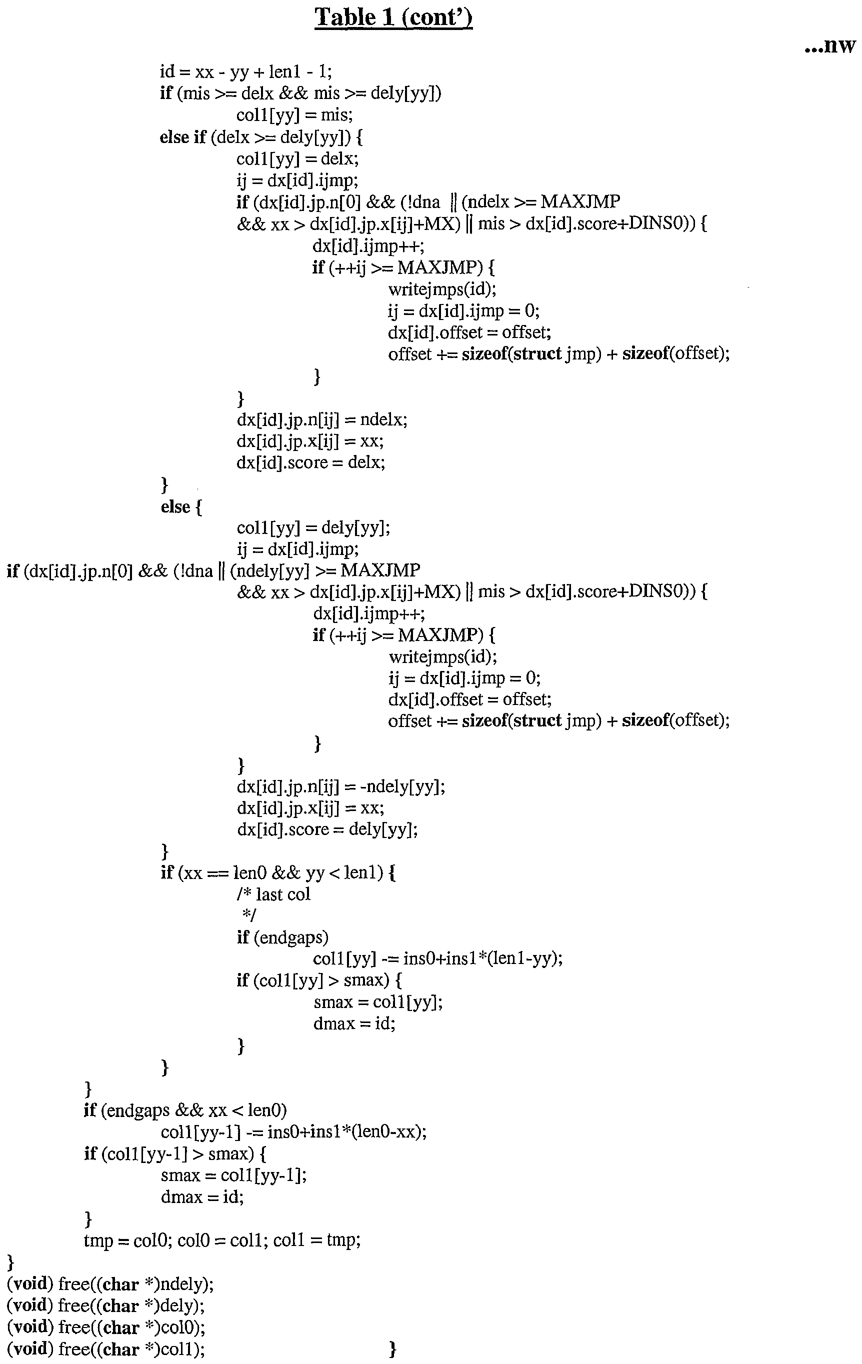

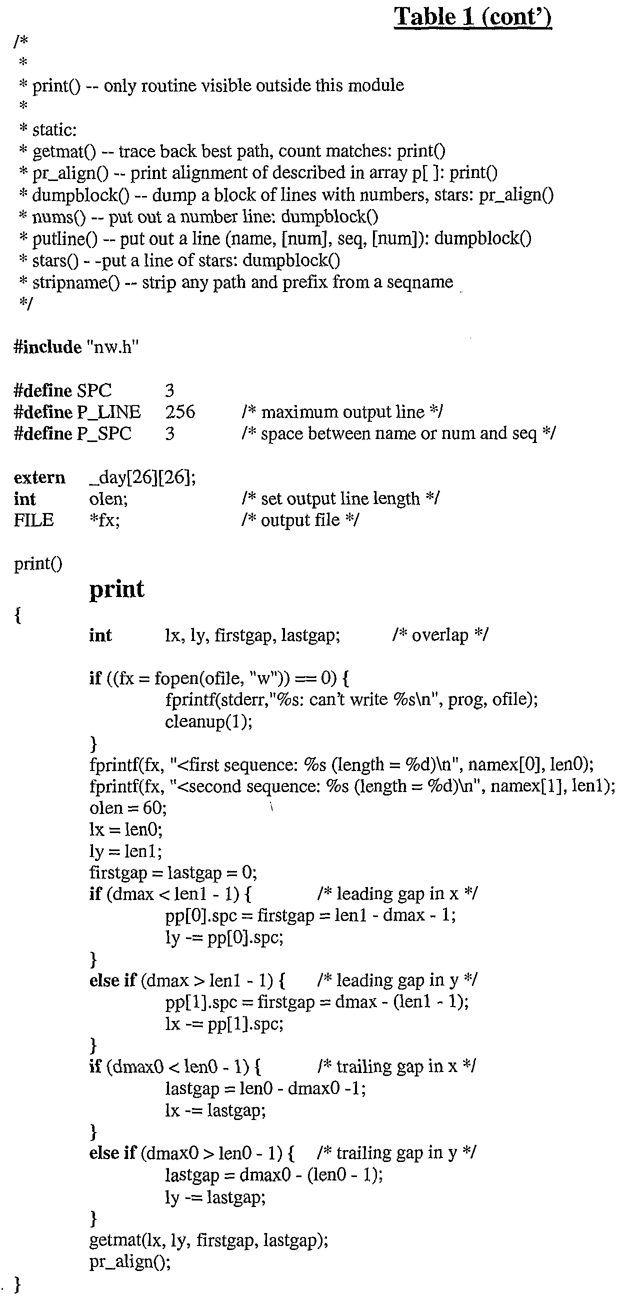

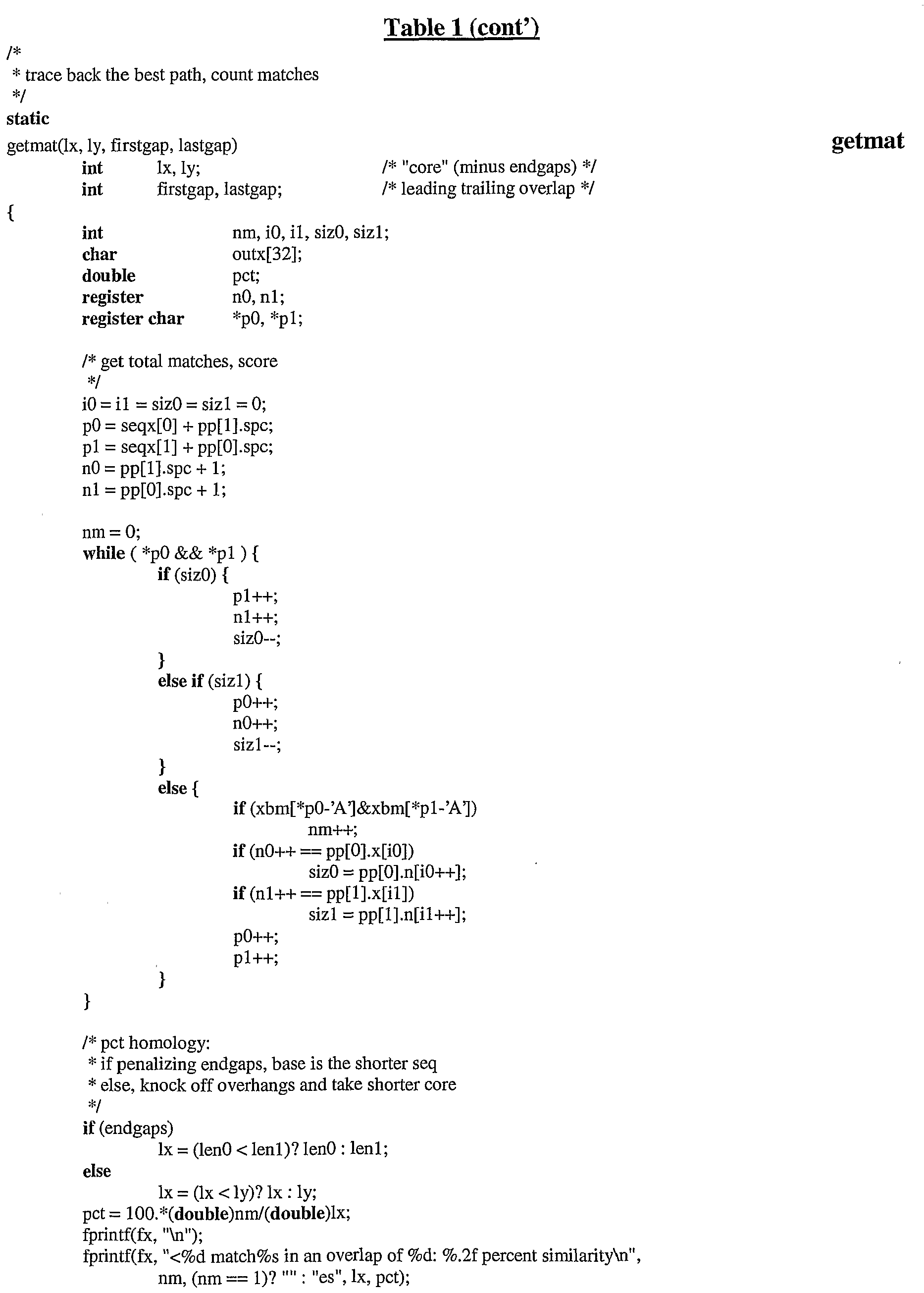

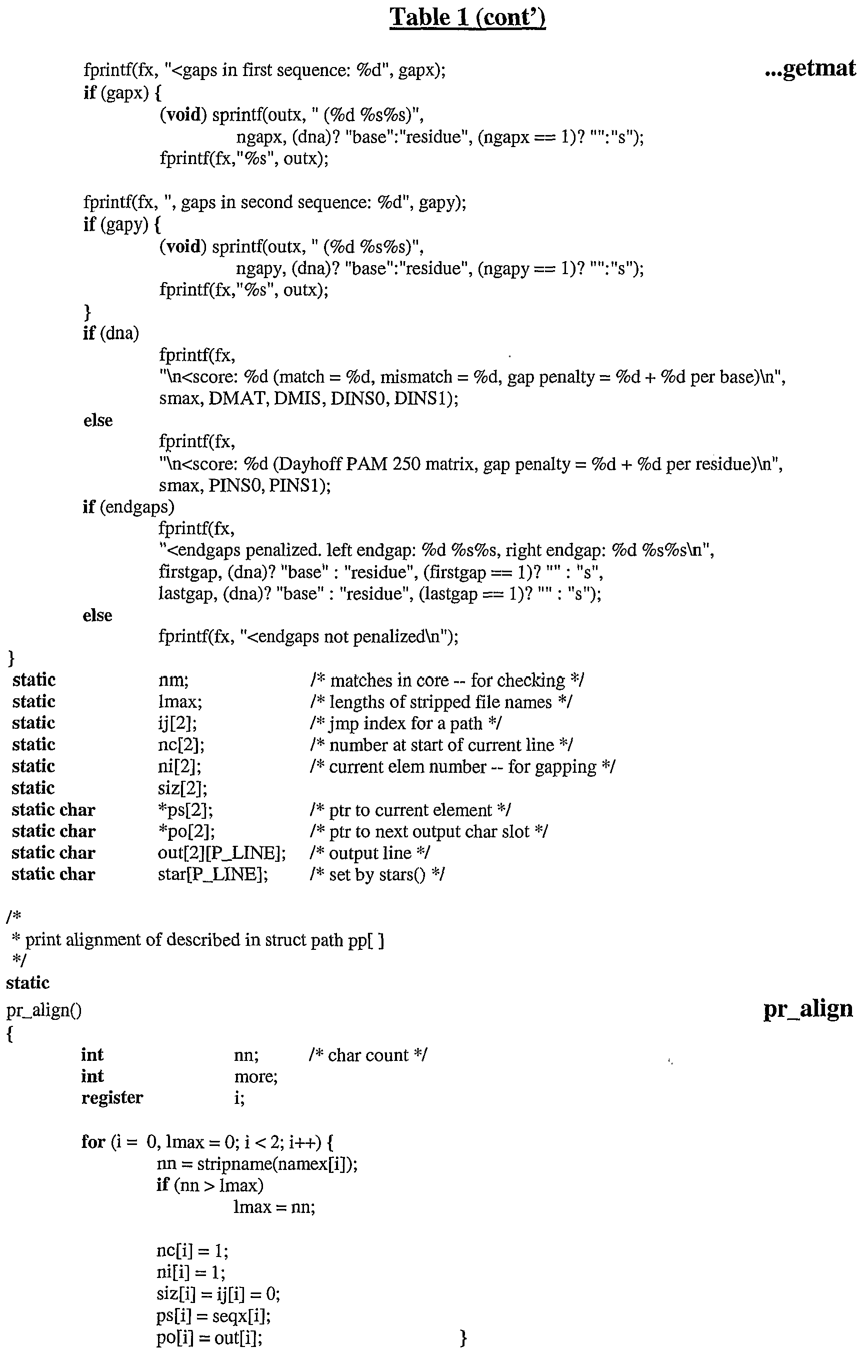

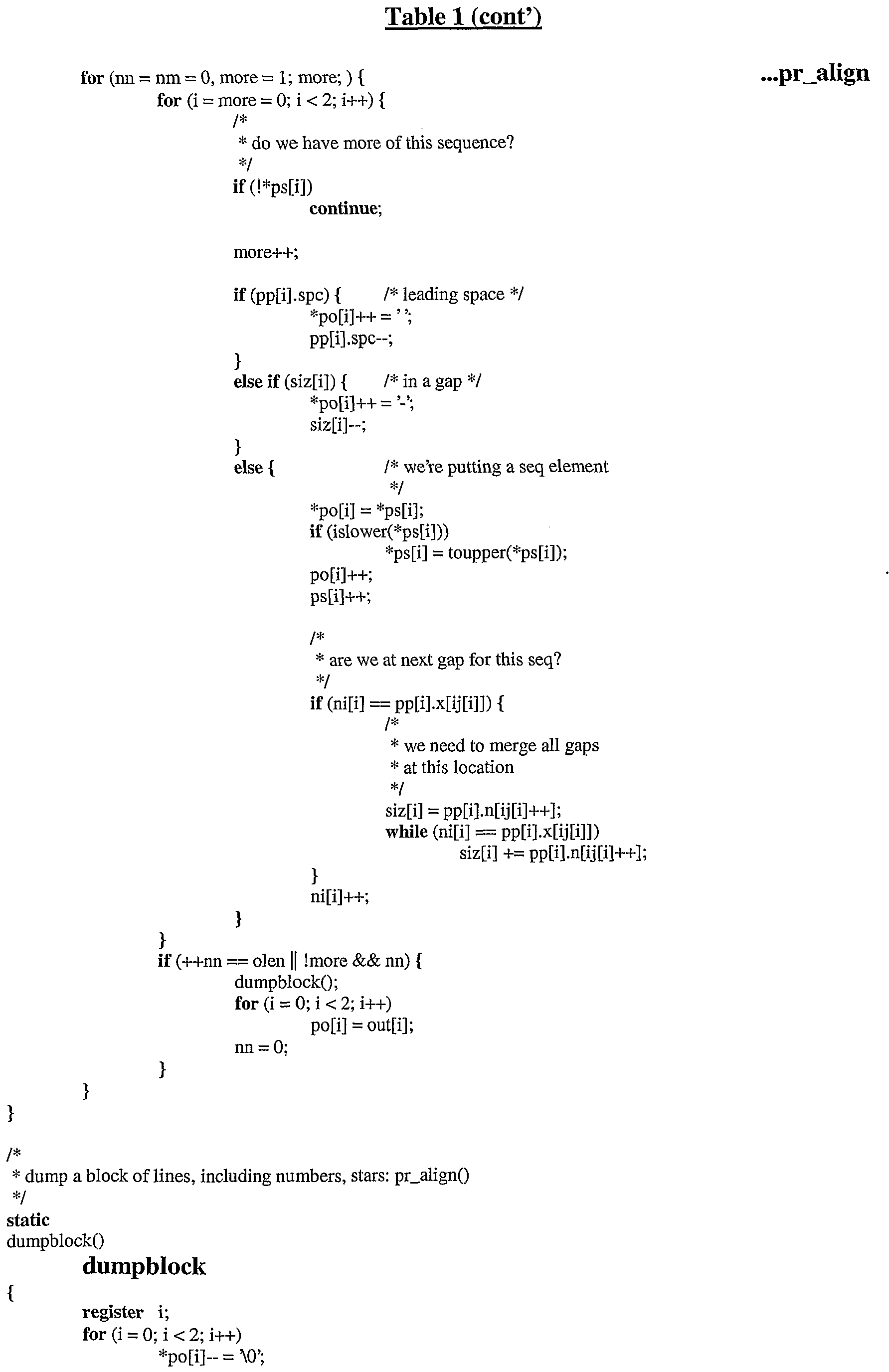

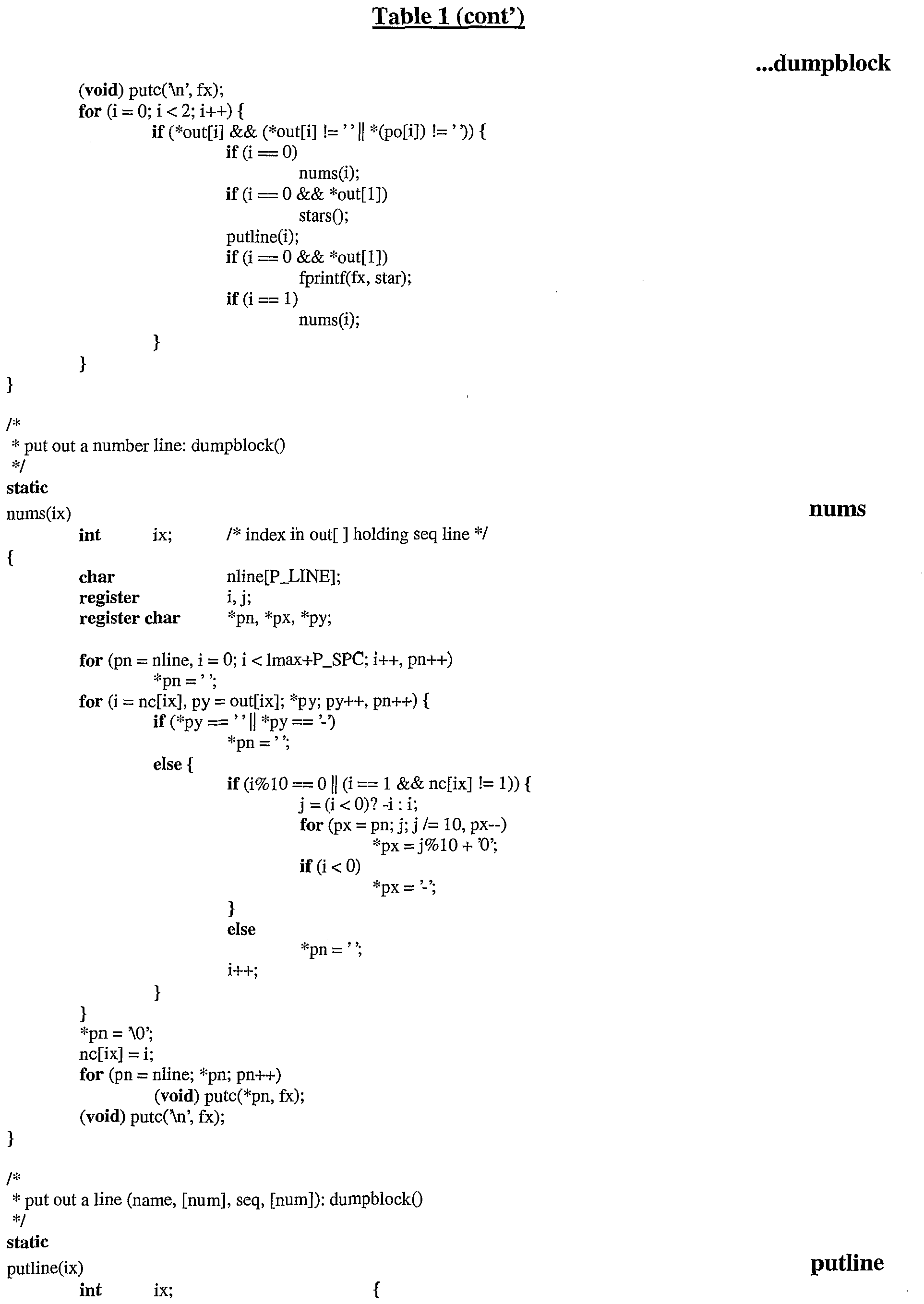

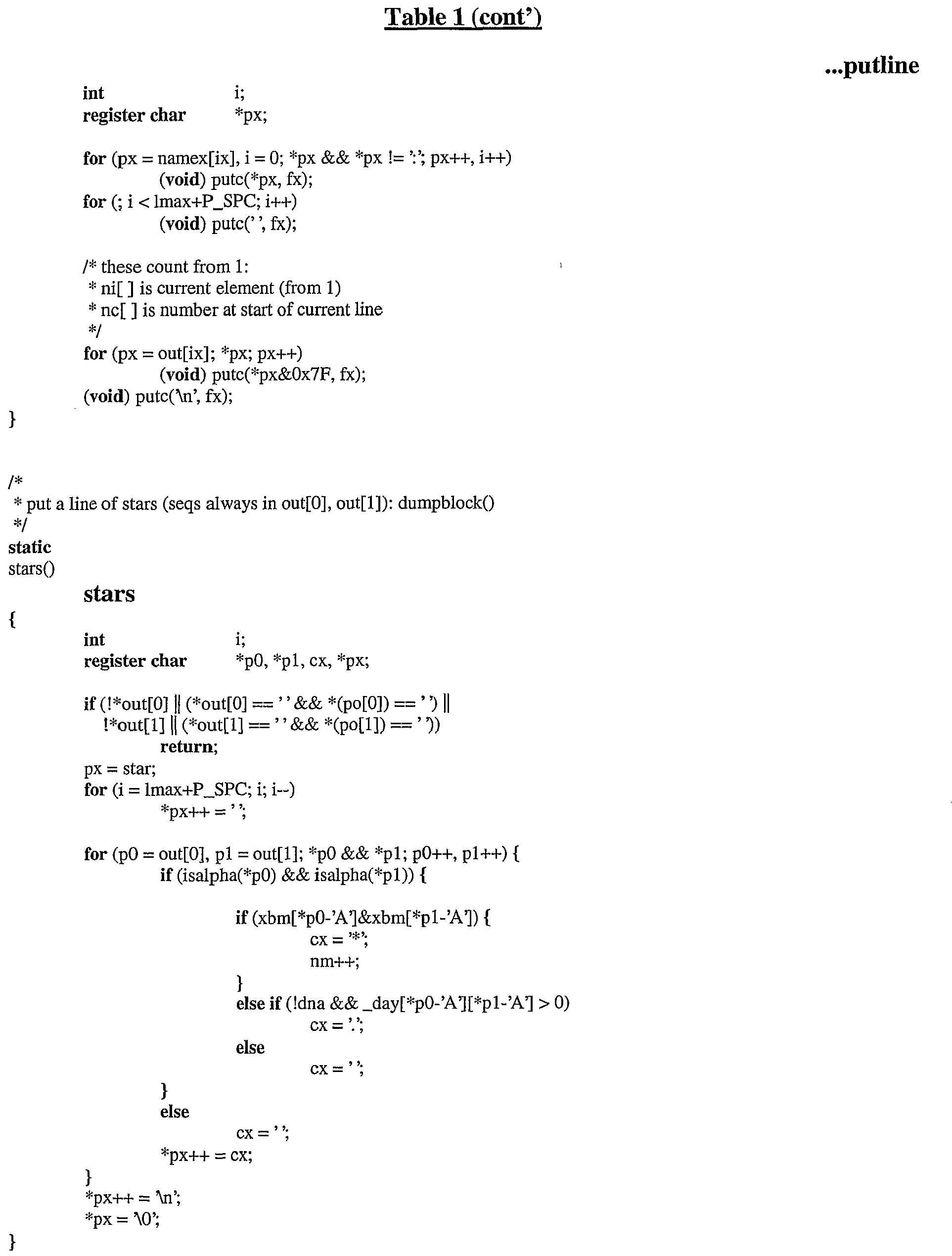

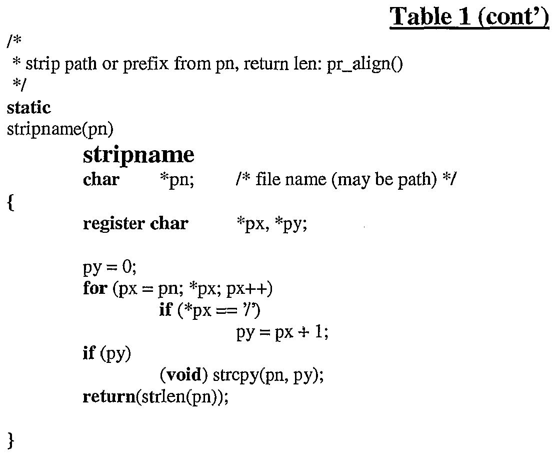

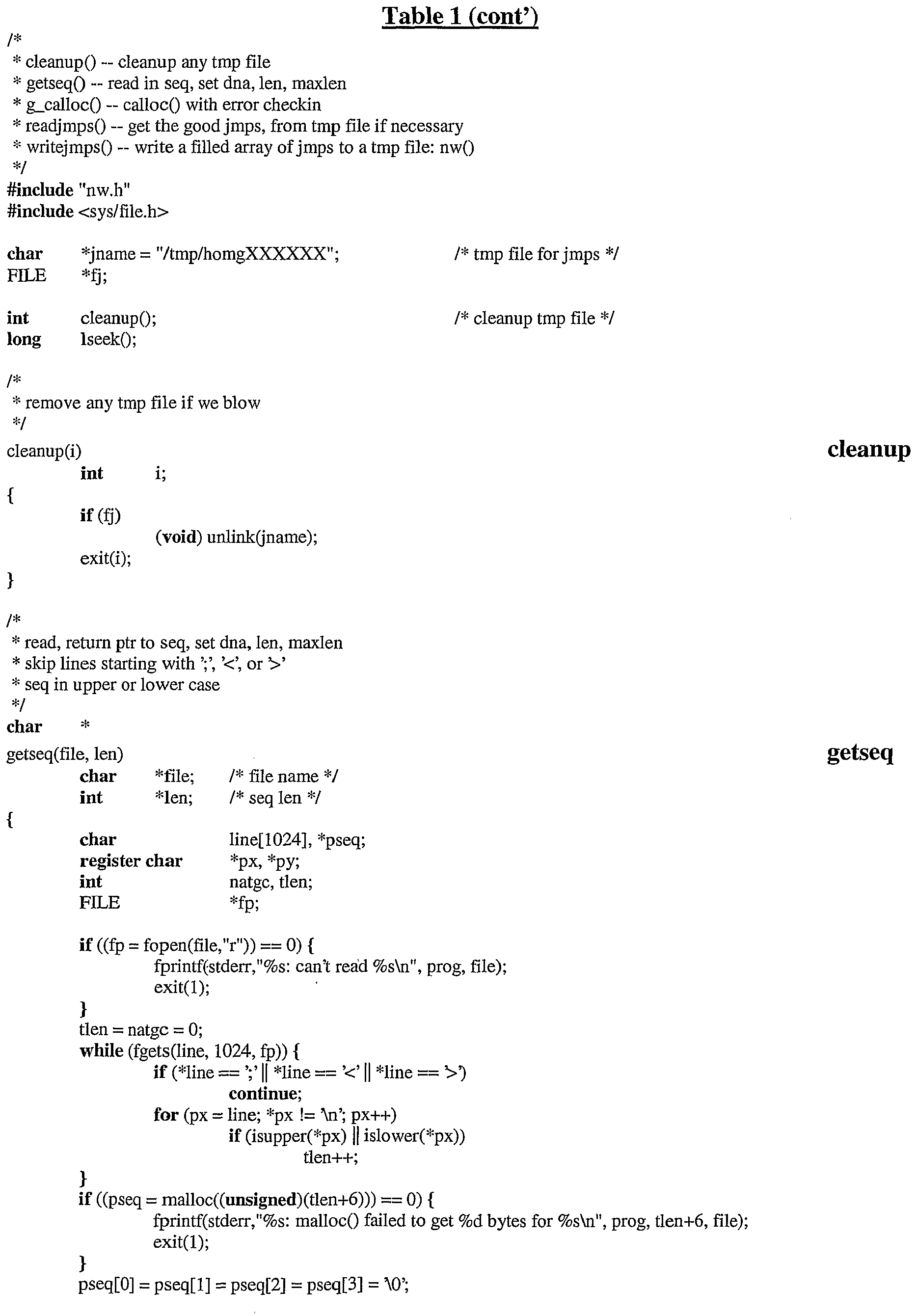

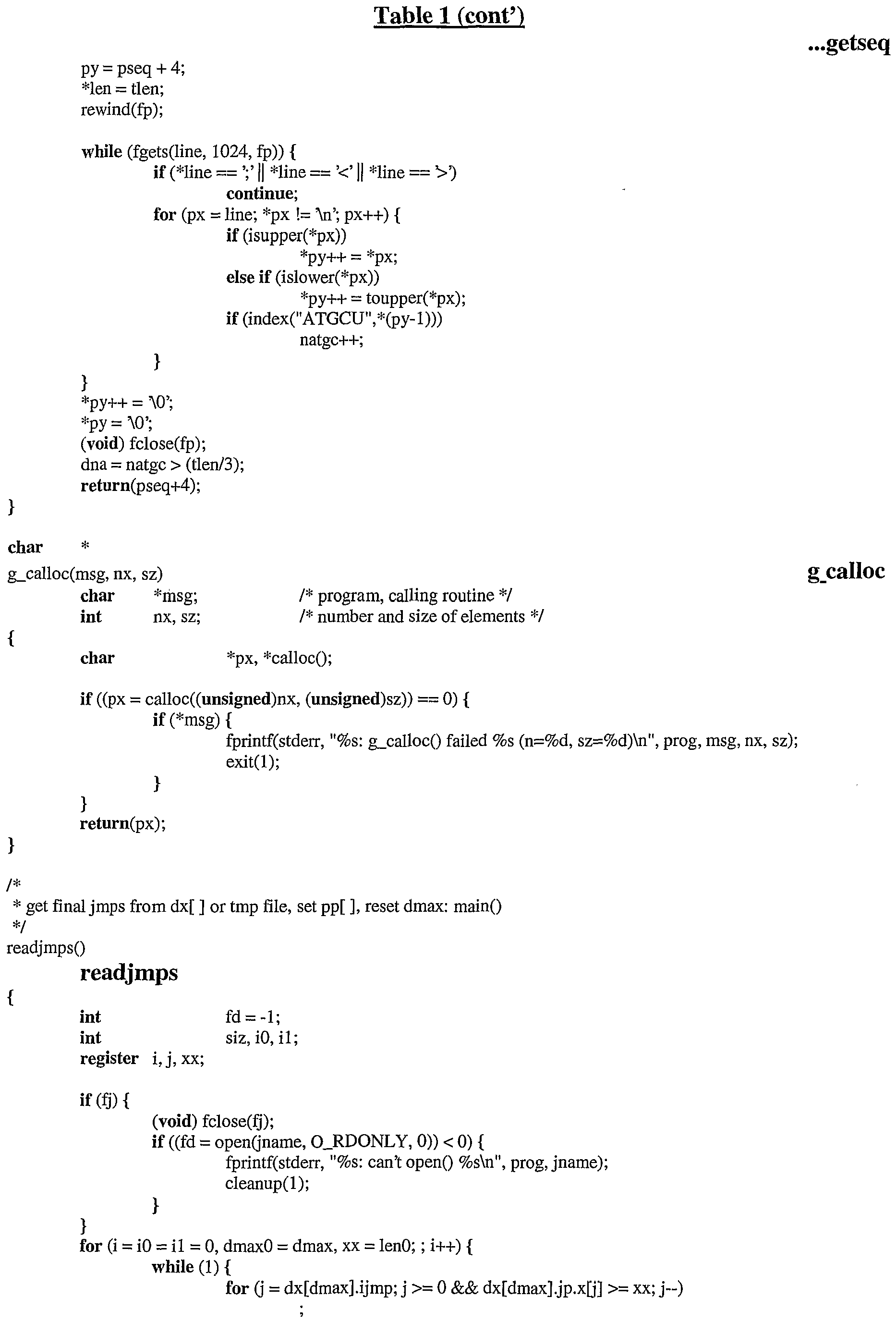

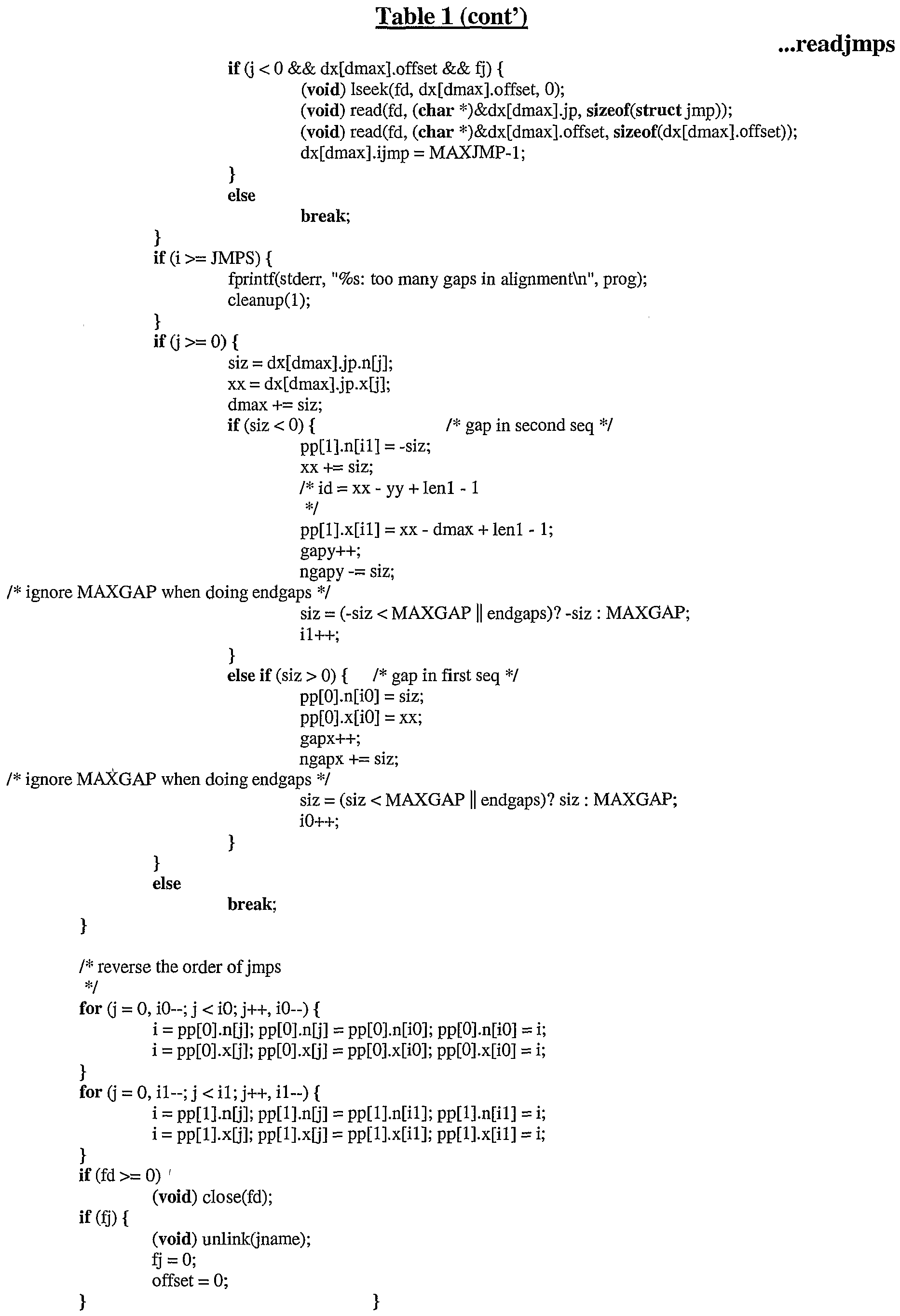

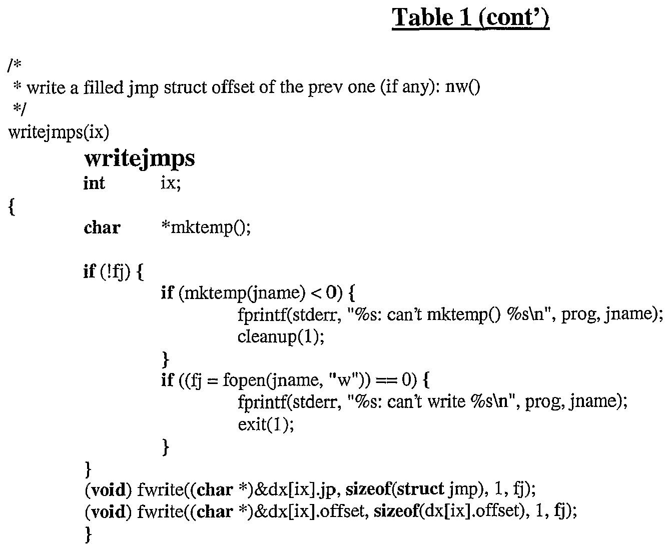

**Table 2**

| | | |
|---|---|---|
| PRO | XXXXXXXXXXXXXXX | (Length = 15 amino acids) |
| Comparison Protein | XXXXXYYYYYYY | (Length = 12 amino acids) |
| % amino acid sequence identity = | | |
| (the number of identically matching amino acid residues between the two polypeptide sequences as determined by ALIGN-2) divided by (the total number of amino acid residues of the PRO polypeptide) = 5 divided by 15 = 33.3% | | |

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |
| % amino acid sequence identity = | | |
| (the number of identically matching amino acid residues between the two polypeptide sequences as determined by ALIGN-2) divided by (the total number of amino acid residues of the PRO polypeptide) = 5 divided by 10 = 50% | | |

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |
| % nucleic acid sequence identity = | | |
| (the number of identically matching nucleotides between the two nucleic acid sequences as determined by ALIGN-2) divided by (the total number of nucleotides of the PRO-DNA nucleic acid sequence) = 6 divided by 14 = 42.9% | | |

**Table 5**

| | | |
|---|---|---|
| PRO-DNA | NNNNNNNNNNNN | (Length = 12 nucleotides) |
| Comparison DNA | NNNNLLLVV | (Length = 9 nucleotides) |
| % nucleic acid sequence identity = | | |
| (the number of identically matching nucleotides between the two nucleic acid sequences as determined by ALIGN-2) divided by (the total number of nucleotides of the PRO-DNA nucleic acid sequence) = 4 divided by 12 = 33.3% | | |

### II. Compositions and Methods of the Invention

### A. Full-Length PRO Polypeptides

The present invention provides newly identified and isolated nucleotide sequences encoding polypeptides referred to in the present application as PRO polypeptides. In particular, cDNAs encoding various PRO polypeptides have been identified and isolated, as disclosed in further detail in the Examples below. It is noted that proteins produced in separate expression rounds may be given different PRO numbers but the UNQ number is unique for any given DNA and the encoded protein, and will not be changed. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been deposited with the ATCC. The actual nucleotide sequences of those clones can readily be determined by the skilled artisan by sequencing of the deposited clone using routine methods in the art. The predicted amino acid sequence can be determined from the nucleotide sequence using routine skill. For the PRO polypeptides and encoding nucleic acids described herein, Applicants have identified what is believed to be the reading frame best identifiable with the sequence information available at the time.

### B. PRO Polypeptide Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO, such as changing the number or position of glycosylation sites or altering the membrane anchoring characteristics.

Variations in the native full-length sequence PRO or in various domains of the PRO described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO that results in a change in the amino acid sequence of the PRO as compared with the native sequence PRO. Optionally, the variation is by substitution of at least one amino acid with any other amino acid in one or more of the domains of the PRO. Guidance in determining which amino acid residue may be inserted, substituted or deleted without adversely affecting the desired activity may be found by comparing the sequence of the PRO with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

**Table 6**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### C. Modifications of PRO

Covalent modifications of PRO are included within the scope of this invention. One type of covalent modification includes reacting targeted amino acid residues of a PRO polypeptide with an organic derivatizing agent that is capable of reacting with selected side chains or the N- or C- terminal residues of the PRO. Derivatization with bifunctional agents is useful, for instance, for crosslinking PRO to a water-insoluble support matrix or surface for use in the method for purifying anti-PRO antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO (either by removing the underlying glycosylation site or by deleting the glycosylation by chemical and/or enzymatic means), and/or adding one or more glycosylation sites that are not present in the native sequence PRO. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO comprises linking the PRO polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO of the present invention may also be modified in a way to form a chimeric molecule comprising PRO fused to another, heterologous polypeptide or amino acid sequence.

In one embodiment, such a chimeric molecule comprises a fusion of the PRO with a tag polypeptide which provides an epitope to which an anti-tag antibody can selectively bind. The epitope tag is generally placed at the amino- or carboxyl- terminus of the PRO. The presence of such epitope-tagged forms of the PRO can be detected using an antibody against the tag polypeptide. Also, provision of the epitope tag enables the PRO to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., Biotechnology, 6:1204-1210 (1988)]; the KT3 epitope peptide [Martin et al., Science, 255:192-194 (1992)]; an alpha-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

### D. Preparation of PRO

The description below relates primarily to production of PRO by culturing cells transformed or transfected with a vector containing PRO nucleic acid. It is, of course, contemplated that alternative methods, which are well known in the art, may be employed to prepare PRO. For instance, the PRO sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO may be chemically synthesized separately and combined using chemical or enzymatic methods to produce the full-length PRO.

### 1. Isolation of DNA Encoring PRO

DNA encoding PRO may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (e.g., automated nucleic acid synthesis).

Libraries can be screened with probes (such as antibodies to the PRO or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for PRO production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as E. *coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA ; E. coli* W3110 strain 9E4, which has the complete genotype *tonA ptr3; E. coli* W3110 strain 27C7 (ATCC 55,244), which has the complete genotype tonA *ptr3 phoA E15 (argF-lac)169 degP ompT kan^{r}; E. coli* W3110 strain 37D6, which has the complete genotype *tonA ptr3 phoA E15 (argF-lac)169 degP ompT rbs7 ilvG kan^{r}; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, in *vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as A. *nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and A. *niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (e.g., cDNA or genomic DNA) encoding PRO may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C*. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO.

Still other methods, vectors, and host cells suitable for adaptation to the synthesis of PRO in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

### 5. Purification of Polypeptide

Forms of PRO may be recovered from culture medium or from host cell lysates. If membrane-bound, it can be released from the membrane using a suitable detergent solution (e.g. Triton-X 100) or by enzymatic cleavage. Cells employed in expression of PRO can be disrupted by various physical or chemical means, such as freeze-thaw cycling, sonication, mechanical disruption, or cell lysing agents.

It may be desired to purify PRO from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO produced.

### E. Tissue Distribution

The location of tissues expressing the PRO can be identified by determining mRNA expression in various human tissues. The location of such genes provides information about which tissues are most likely to be affected by the stimulating and inhibiting activities of the PRO polypeptides. The location of a gene in a specific tissue also provides sample tissue for the activity blocking assays discussed below.

As noted before, gene expression in various tissues may be measured by conventional Southern blotting, Northern blotting to quantitate the transcription of mRNA (Thomas, Proc. Natl. Acad. Sci. USA, 77:5201-5205 [1980]), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence of a PRO polypeptide or against a synthetic peptide based on the DNA sequences encoding the PRO polypeptide or against an exogenous sequence fused to a DNA encoding a PRO polypeptide and encoding a specific antibody epitope. General techniques for generating antibodies, and special protocols for Northern blotting and *in situ* hybridization are provided below.

### F. Antibody Binding Studies

The activity of the PRO polypeptides can be further verified by antibody binding studies, in which the ability of anti-PRO antibodies to inhibit the effect of the PRO polypeptides, respectively, on tissue cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e.g.,* US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tissue sample may be fresh or frozen or may be embedded in paraffin and fixed with a preservative such as formalin, for example.

### G. Cell-Based Assays

Cell-based assays and animal models for immune related diseases can be used to further understand the relationship between the genes and polypeptides identified herein and the development and pathogenesis of immune related disease.

In a different approach, cells of a cell type known to be involved in a particular immune related disease are transfected with the cDNAs described herein, and the ability of these cDNAs to stimulate or inhibit immune function is analyzed. Suitable cells can be transfected with the desired gene, and monitored for immune function activity. Such transfected cell lines can then be used to test the ability of poly- or monoclonal antibodies or antibody compositions to inhibit or stimulate immune function, for example to modulate T-cell proliferation or inflammatory cell infiltration. Cells transfected with the coding sequences of the genes identified herein can further be used to identify drug candidates for the treatment of immune related diseases.

In addition, primary cultures derived from transgenic animals (as described below) can be used in the cell-based assays herein, although stable cell lines are preferred. Techniques to derive continuous cell lines from transgenic animals are well known in the art (see, *e.g.,* Small et al., Mol. Cell. Biol. 5: 642-648 [1985]).

One suitable cell based assay is the mixed lymphocyte reaction (MLR). Current Protocols in Immunology, unit 3.12; edited by J E Coligan, A M Kruisbeek, D H Marglies, E M Shevach, W Strober, National Institutes of Health, Published by John Wiley & Sons, Inc. In this assay, the ability of a test compound to stimulate or inhibit the proliferation of activated T cells is assayed. A suspension of responder T cells is cultured with allogeneic stimulator cells and the proliferation of T cells is measured by uptake of tritiated thymidine. This assay is a general measure of T cell reactivity. Since the majority of T cells respond to and produce IL-2 upon activation, differences in responsiveness in this assay in part reflect differences in IL-2 production by the responding cells. The MLR results can be verified by a standard lymphokine (IL-2) detection assay. *Current Protocols in Immunology,* above, 3.15, 6.3.

A proliferative T cell response in an MLR assay may be due to direct mitogenic properties of an assayed molecule or to external antigen induced activation. Additional verification of the T cell stimulatory activity of the PRO polypeptides can be obtained by a costimulation assay. T cell activation requires an antigen specific signal mediated through the T-cell receptor (TCR) and a costimulatory signal mediated through a second ligand binding interaction, for example, the B7 (CD80, CD86)/CD28 binding interaction. CD28 crosslinking increases lymphokine secretion by activated T cells. T cell activation has both negative and positive controls through the binding of ligands which have a negative or positive effect. CD28 and CTLA-4 are related glycoproteins in the Ig superfamily which bind to B7. CD28 binding to B7 has a positive costimulation effect of T cell activation; conversely, CTLA-4 binding to B7 has a T cell deactivating effect. Chambers, C. A. and Allison, J. P., Curr. Opin. Immunol. (1997) 9:396. Schwartz, R. H., Cell (1992) 71:1065; Linsey, P. S. and Ledbetter, J. A., Annu. Rev. Immunol. (1993) 11:191; June, C. H. et al, Immunol. Today (1994) 15:321; Jenkins, M. K., Immunity (1994) 1:405. In a costimulation assay, the PRO polypeptides are assayed for T cell costimulatory or inhibitory activity.

Direct use of a stimulating compound as in the invention has been validated in experiments with 4-1BB glycoprotein, a member of the tumor necrosis factor receptor family, which binds to a ligand (4-1BBL) expressed on primed T cells and signals T cell activation and growth. Alderson, M. E. et al., J. Immunol. (1994) 24:2219.

The use of an agonist stimulating compound has also been validated experimentally. Activation of 4-1BB by treatment with an agonist anti-4-1BB antibody enhances eradication of tumors. Hellstrom, I. and Hellstrom, K. E., Crit. Rev. Immunol. (1998) 18:1. Immunoadjuvant therapy for treatment of tumors, described in more detail below, is another example of the use of the stimulating compounds of the invention.

Alternatively, an immune stimulating or enhancing effect can also be achieved by administration of a PRO which has vascular permeability enhancing properties. Enhanced vascular permeability would be beneficial to disorders which can be attenuated by local infiltration of immune cells (e.g., monocytes, eosinophils, PMNs) and inflammation.

On the other hand, PRO polypeptides, as well as other compounds of the invention, which are direct inhibitors of T cell proliferation/activation, lymphokine secretion, and/or vascular permeability can be directly used to suppress the immune response. These compounds are useful to reduce the degree of the immune response and to treat immune related diseases characterized by a hyperactive, superoptimal, or autoimmune response. This use of the compounds of the invention has been validated by the experiments described above in which CTLA-4 binding to receptor B7 deactivates T cells. The direct inhibitory compounds of the invention function in an analogous manner. The use of compound which suppress vascular permeability would be expected to reduce inflammation. Such uses would be beneficial in treating conditions associated with excessive inflammation.

Alternatively, compounds, e.g., antibodies, which bind to stimulating PRO polypeptides and block the stimulating effect of these molecules produce a net inhibitory effect and can be used to suppress the T cell mediated immune response by inhibiting T cell proliferation/activation and/or lymphokine secretion. Blocking the stimulating effect of the polypeptides suppresses the immune response of the mammal. This use has been validated in experiments using an anti-IL2 antibody. In these experiments, the antibody binds to IL2 and blocks binding of IL2 to its receptor thereby achieving a T cell inhibitory effect.

### H. Animal Models

The results of the cell based in vitro assays can be further verified using *in vivo* animal models and assays for T-cell function. A variety of well known animal models can be used to further understand the role of the genes identified herein in the development and pathogenesis of immune related disease, and to test the efficacy of candidate therapeutic agents, including antibodies, and other antagonists of the native polypeptides, including small molecule antagonists. *The in vivo* nature of such models makes them predictive of responses in human patients. Animal models of immune related diseases include both non-recombinant and recombinant (transgenic) animals. Non-recombinant animal models include, for example, rodent, e.g., murine models. Such models can be generated by introducing cells into syngeneic mice using standard techniques, e.g., subcutaneous injection, tail vein injection, spleen implantation, intraperitoneal implantation, implantation under the renal capsule, *etc.*

Graft-versus-host disease occurs when immunocompetent cells are transplanted into immunosuppressed or tolerant patients. The donor cells recognize and respond to host antigens. The response can vary from life threatening severe inflammation to mild cases of diarrhea and weight loss. Graft-versus-host disease models provide a means of assessing T cell reactivity against MHC antigens and minor transplant antigens. A suitable procedure is described in detail in Current Protocols in Immunology, above, unit 4.3.

An animal model for skin allograft rejection is a means of testing the ability of T cells to mediate in *vivo* tissue destruction and a measure of their role in transplant rejection. The most common and accepted models use murine tail-skin grafts. Repeated experiments have shown that skin allograft rejection is mediated by T cells, helper T cells and killer-effector T cells, and not antibodies. Auchincloss, H. Jr. and Sachs, D. H., Fundamental Immunology, 2nd ed., W. E. Paul ed., Raven Press, NY, 1989, 889-992. A suitable procedure is described in detail in *Current Protocols in Immunology,* above, unit 4.4. Other transplant rejection models which can be used to test the compounds of the invention are the allogeneic heart transplant models described by Tanabe, M. et al, Transplantation (1994) 58:23 and Tinubu, S. A. et al, J. Immunol. (1994) 4330-4338.

Animal models for delayed type hypersensitivity provides an assay of cell mediated immune function as well. Delayed type hypersensitivity reactions are a T cell mediated *in vivo* immune response characterized by inflammation which does not reach a peak until after a period of time has elapsed after challenge with an antigen. These reactions also occur in tissue specific autoimmune diseases such as multiple sclerosis (MS) and experimental autoimmune encephalomyelitis (EAE, a model for MS). A suitable procedure is described in detail in *Current Protocols in Immunology,* above, unit 4.5.

EAE is a T cell mediated autoimmune disease characterized by T cell and mononuclear cell inflammation and subsequent demyelination of axons in the central nervous system. EAE is generally considered to be a relevant animal model for MS in humans. Bolton, C., Multiple Sclerosis (1995) 1:143. Both acute and relapsing-remitting models have been developed. The compounds of the invention can be tested for T cell stimulatory or inhibitory activity against immune mediated demyelinating disease using the protocol described in *Current Protocols in Immunology,* above, units 15.1 and 15.2. See also the models for myelin disease in which oligodendrocytes or Schwann cells are grafted into the central nervous system as described in Duncan, I. D. et al, Molec. Med. Today (1997) 554-561.

Contact hypersensitivity is a simple delayed type hypersensitivity *in vivo* assay of cell mediated immune function. In this procedure, cutaneous exposure to exogenous haptens which gives rise to a delayed type hypersensitivity reaction which is measured and quantitated. Contact sensitivity involves an initial sensitizing phase followed by an elicitation phase. The elicitation phase occurs when the T lymphocytes encounter an antigen to which they have had previous contact. Swelling and inflammation occur, making this an excellent model of human allergic contact dermatitis. A suitable procedure is described in detail in Current Protocols in Immunology, Eds. J. E. Cologan, A. M. Kruisbeek, D. H. Margulies, E. M. Shevach and W. Strober, John Wiley & Sons, Inc., 1994, unit 4.2. See also Grabbe, S. and Schwarz, T, Immun. Today 19 (1): 37-44 (1998).

An animal model for arthritis is collagen-induced arthritis. This model shares clinical, histological and immunological characteristics of human autoimmune rheumatoid arthritis and is an acceptable model for human autoimmune arthritis. Mouse and rat models are characterized by synovitis, erosion of cartilage and subchondral bone. The compounds of the invention can be tested for activity against autoimmune arthritis using the protocols described in *Current Protocols in Immunology,* above, units 15.5. See also the model using a monoclonal antibody to CD18 and VLA-4 integrins described in Issekutz, A.C. et al., Immunology (1996) 88:569.

A model of asthma has been described in which antigen-induced airway hyper-reactivity, pulmonary eosinophilia and inflammation are induced by sensitizing an animal with ovalbumin and then challenging the animal with the same protein delivered by aerosol. Several animal models (guinea pig, rat, non-human primate) show symptoms similar to atopic asthma in humans upon challenge with aerosol antigens. Murine models have many of the features of human asthma. Suitable procedures to test the compounds of the invention for activity and effectiveness in the treatment of asthma are described by Wolyniec, W. W. et al, Am. J. Respir. Cell Mol. Biol. (1998) 18:777 and the references cited therein.

Additionally, the compounds of the invention can be tested on animal models for psoriasis like diseases. Evidence suggests a T cell pathogenesis for psoriasis. The compounds of the invention can be tested in the scid/scid mouse model described by Schon, M. P. et al, Nat. Med. (1997) 3:183, in which the mice demonstrate histopathologic skin lesions resembling psoriasis. Another suitable model is the human skin/scid mouse chimera prepared as described by Nickoloff, B. J. et al, Am. J. Path. (1995) 146:580.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, e.g., baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (e.g., Van der Putten et al., Proc. Natl. Acad. Sci. USA 82, 6148-615 [1985]); gene targeting in embryonic stem cells (Thompson et al., Cell 56, 313-321 [1989]); electroporation of embryos (Lo, Mol. Cel. Biol. 3, 1803-1814 [1983]); sperm-mediated gene transfer (Lavitrano et al., Cell 57, 717-73 [1989]). For review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, e.g., head-to-head or head-to-tail tandems. Selective introduction of a transgene into a particular cell type is also possible by following, for example, the technique of Lasko et al., Proc. Natl. Acad. Sci. USA 89, 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry.

The animals may be further examined for signs of immune disease pathology, for example by histological examination to determine infiltration of immune cells into specific tissues. Blocking experiments can also be performed in which the transgenic animals are treated with the compounds of the invention to determine the extent of the T cell proliferation stimulation or inhibition of the compounds. In these experiments, blocking antibodies which bind to the PRO polypeptide, prepared as described above, are administered to the animal and the effect on immune function is determined.

Alternatively, "knock out" animals can be constructed which have a defective or altered gene encoding a polypeptide identified herein, as a result of homologous recombination between the endogenous gene encoding the polypeptide and altered genomic DNA encoding the same polypeptide introduced into an embryonic cell of the animal. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see *e.g.,* Thomas and Capecchi, Cell, 51:503 (1987) for a description of homologous recombination vectors]. The vector is introduced into an embryonic stem cell line (*e.g*., by electroporation) and cells in which the introduced DNA has homologously recombined with the endogenous DNA are selected [see *e.g.,* Li et al., Cell, 69:915 (1992)]. The selected cells are then injected into a blastocyst of an animal (*e.g.,* a mouse or rat) to form aggregation chimeras [see *e.g.,* Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

### I. ImmunoAdjuvant Therapy

In one embodiment, the immunostimulating compounds of the invention can be used in immunoadjuvant therapy for the treatment of tumors (cancer). It is now well established that T cells recognize human tumor specific antigens. One group of tumor antigens, encoded by the MAGE, BAGE and GAGE families of genes, are silent in all adult normal tissues , but are expressed in significant amounts in tumors, such as melanomas, lung tumors, head and neck tumors, and bladder carcinomas. DeSmet, C. et al., (1996) Proc. Natl. Acad. Sci. USA, 93:7149. It has been shown that costimulation of T cells induces tumor regression and an antitumor response both *in vitro* and *in vivo.* Melero, I. et al., Nature Medicine (1997) 3:682; Kwon, E. D. et al., Proc. Natl. Acad. Sci. USA (1997) 94: 8099; Lynch, D. H. et al, Nature Medicine (1997) 3:625; Finn, O. J. and Lotze, M. T., J. Immunol. (1998) 21:114. The stimulatory compounds of the invention can be administered as adjuvants, alone or together with a growth regulating agent, cytotoxic agent or chemotherapeutic agent, to stimulate T cell proliferation/activation and an antitumor response to tumor antigens. The growth regulating, cytotoxic, or chemotherapeutic agent may be administered in conventional amounts using known administration regimes. Immunostimulating activity by the compounds of the invention allows reduced amounts of the growth regulating, cytotoxic, or chemotherapeutic agents thereby potentially lowering the toxicity to the patient.

### J. Screening Assays for Drug Candidates

Screening assays for drug candidates are designed to identify compounds that bind to or complex with the polypeptides encoded by the genes identified herein or a biologically active fragment thereof, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art. All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, *e.g*., on a microtiter plate, by covalent or non-covalent attachments. Non-covalent attachment generally is accomplished by coating the solid surface with a solution of the polypeptide and drying. Alternatively, an immobilized antibody, *e.g*., a monoclonal antibody, specific for the polypeptide to be immobilized can be used to anchor it to a solid surface. The assay is performed by adding the non-immobilized component, which may be labeled by a detectable label, to the immobilized component, *e.g*., the coated surface containing the anchored component. When the reaction is complete, the non-reacted components are removed, *e.g*., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labelled antibody specifically binding the immobilized complex.

If the candidate compound interacts with but does not bind to a particular protein encoded by a gene identified herein, its interaction with that protein can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989); Chien et al., Proc. Natl. Acad. Sci. USA 88, 9578-9582 (1991)] as disclosed by Chevray and Nathans, Proc. Natl. Acad. Sci. USA 89, 5789-5793 (1991). Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1*-LacZ* reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

In order to find compounds that interfere with the interaction of a gene identified herein and other intra- or extracellular components can be tested, a reaction mixture is usually prepared containing the product of the gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described above.

The formation of a complex in the control reaction(s) but not in the reaction mixture containing the test compound indicates that the test compound interferes with the interaction of the test compound and its reaction partner.

### K. Compositions and Methods for the Treatment of Immune Related Diseases

The compositions useful in the treatment of immune related diseases include, without limitation, proteins, antibodies, small organic molecules, peptides, phosphopeptides, antisense and ribozyme molecules, triple helix molecules, *etc.* that inhibit or stimulate immune function, for example, T cell proliferation/activation, lymphokine release, or immune cell infiltration.

For example, antisense RNA and RNA molecules act to directly block the translation of mRNA by hybridizing to targeted mRNA and preventing protein translation. When antisense DNA is used, oligodeoxyribonucleotides derived from the translation initiation site, *e.g.,* between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e.g.,* Rossi, *Current Biology* 4, 469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details see, *e.g.*, PCT publication No. WO 97/33551, *supra.*

These molecules can be identified by any or any combination of the screening assays discussed above and/or by any other screening techniques well known for those skilled in the art.

### L. Anti-PRO Antibodies

The present invention further provides anti-PRO antibodies. Exemplary antibodies include polyclonal, monoclonal, humanized, bispecific, and heteroconjugate antibodies.

### 1. Polyclonal Antibodies

The anti-PRO antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The anti-PRO antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### 3. Human and Humanized Antibodies

The anti-PRO antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).
The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

### 4. Bispecific Antibodies

Bispecific antibodies are monoclonal, preferably human or humanized, antibodies that have binding specificities for at least two different antigens. In the present case, one of the binding specificities is for the PRO, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared can be prepared using chemical linkage. Brennan et al., Science 229:81 (1985) describe a procedure wherein intact antibodies are proteolytically cleaved to generate F(ab')2 fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994). Antibodies with more than two valencies are contemplated. For example, trispecific antibodies can be prepared. Tutt et al., J. Immunol. 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 6. Effector Function Engineering

It may be desirable to modify the antibody of the invention with respect to effector function, so as to enhance, *e.g*., the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., Anti-Cancer Drug Design, 3: 219-230 (1989).

### 7. Immunoconjugates

The invention also pertains to immunoconjugates comprising an antibody conjugated to a cytotoxic agent such as a chemotherapeutic agent, toxin (*e.g.,* an enzymatically active toxin of bacterial, fungal, plant, or animal origin, or fragments thereof), or a radioactive isotope (*i.e.,* a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See W094/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.,* avidin) that is conjugated to a cytotoxic agent (*e.g*., a radionucleotide).

### 8. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81 (19): 1484 (1989).

### M. Pharmaceutical Compositions

The active PRO molecules of the invention (*e.g.,* PRO polypeptides, anti-PRO antibodies, and/or variants of each) as well as other molecules identified by the screening assays disclosed above, can be administered for the treatment of immune related diseases, in the form of pharmaceutical compositions.

Therapeutic formulations of the active PRO molecule, preferably a polypeptide or antibody of the invention, are prepared for storage by mixing the active molecule having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.,* Zn-protein complexes); and/or non-ionic surfactants such as TWEEN^{™}, PLURONICS^{™} or polyethylene glycol (PEG).

Compounds identified by the screening assays disclosed herein can be formulated in an analogous manner, using standard techniques well known in the art.

Lipofections or liposomes can also be used to deliver the PRO molecule into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (see, *e.g.,* Marasco et al., Proc. Natl. Acad. Sci. USA 90, 7889-7893 [1993]).

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active PRO molecules may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations or the PRO molecules may be prepared. Suitable examples of sustained-release preparations include semipermeable matrices of solid hydrophobic polymers containing the antibody, which matrices are in the form of shaped articles, *e.g.,* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ-ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### N. Methods of Treatment

It is contemplated that the polypeptides, antibodies and other active compounds of the present invention may be used to treat various immune related diseases and conditions, such as T cell mediated diseases, including those characterized by infiltration of inflammatory cells into a tissue, stimulation of T-cell proliferation, inhibition of T-cell proliferation, increased or decreased vascular permeability or the inhibition thereof.

Exemplary conditions or disorders to be treated with the polypeptides, antibodies and other compounds of the invention, include, but are not limited to systemic lupus erythematosis, rheumatoid arthritis, juvenile chronic arthritis, osteoarthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

In systemic lupus erythematosus, the central mediator of disease is the production of auto-reactive antibodies to self proteins/tissues and the subsequent generation of immune-mediated inflammation. Antibodies either directly or indirectly mediate tissue injury. Though T lymphocytes have not been shown to be directly involved in tissue damage, T lymphocytes are required for the development of auto-reactive antibodies. The genesis of the disease is thus T lymphocyte dependent. Multiple organs and systems are affected clinically including kidney, lung, musculoskeletal system, mucocutaneous, eye, central nervous system, cardiovascular system, gastrointestinal tract, bone marrow and blood.

Rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, intestitial pneumonitis with pulmonary fibrosis, keratoconjunctivitis sicca, and rhematoid nodules.

Juvenile chronic arthritis is a chronic idiopathic inflammatory disease which begins often at less than 16 years of age. Its phenotype has some similarities to RA; some patients which are rhematoid factor positive are classified as juvenile rheumatoid arthritis. The disease is sub-classified into three major categories: pauciarticular, polyarticular, and systemic. The arthritis can be severe and is typically destructive and leads to joint ankylosis and retarded growth. Other manifestations can include chronic anterior uveitis and systemic amyloidosis.

Spondyloarthropathies are a group of disorders with some common clinical features and the common association with the expression of HLA-B27 gene product. The disorders include: ankylosing sponylitis, Reiter's syndrome (reactive arthritis), arthritis associated with inflammatory bowel disease, spondylitis associated with psoriasis, juvenile onset spondyloarthropathy and undifferentiated spondyloarthropathy. Distinguishing features include sacroileitis with or without spondylitis; inflammatory asymmetric arthritis; association with HLA-B27 (a serologically defined allele of the HLA-B locus of class I MHC); ocular inflammation, and absence of autoantibodies associated with other rheumatoid disease. The cell most implicated as key to induction of the disease is the CD8+ T lymphocyte, a cell which targets antigen presented by class I MHC molecules. CD8+ T cells may react against the class I MHC allele HLA-B27 as if it were a foreign peptide expressed by MHC class I molecules. It has been hypothesized that an epitope of HLA-B27 may mimic a bacterial or other microbial antigenic epitope and thus induce a CD8+ T cells response.

Systemic sclerosis (scleroderma) has an unknown etiology. A hallmark of the disease is induration of the skin; likely this is induced by an active inflammatory process. Scleroderma can be localized or systemic; vascular lesions are common and endothelial cell injury in the microvasculature is an early and important event in the development of systemic sclerosis; the vascular injury may be immune mediated. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. Other organs involved include: the gastrointestinal tract: smooth muscle atrophy and fibrosis resulting in abnormal peristalsis/motility; kidney: concentric subendothelial intimal proliferation affecting small arcuate and interlobular arteries with resultant reduced renal cortical blood flow, results in proteinuria, azotemia and hypertension; skeletal muscle: atrophy, interstitial fibrosis; inflammation; lung: interstitial pneumonitis and interstitial fibrosis; and heart: contraction band necrosis, scarring/fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components, proteins and RNA's, involved in protein synthesis.

Sjögren's syndrome is due to immune-mediated inflammation and subsequent functional destruction of the tear glands and salivary glands. The disease can be associated with or accompanied by inflammatory connective tissue diseases. The disease is associated with autoantibody production against Ro and La antigens, both of which are small RNA-protein complexes. Lesions result in keratoconjunctivitis sicca, xerostomia, with other manifestations or associations including bilary cirrhosis, peripheral or sensory neuropathy, and palpable purpura.

Systemic vasculitis are diseases in which the primary lesion is inflammation and subsequent damage to blood vessels which results in ischemia/necrosis/degeneration to tissues supplied by the affected vessels and eventual end-organ dysfunction in some cases. Vasculitides can also occur as a secondary lesion or sequelae to other immune-inflammatory mediated diseases such as rheumatoid arthritis, systemic sclerosis, etc., particularly in diseases also associated with the formation of immune complexes. Diseases in the primary systemic vasculitis group include: systemic necrotizing vasculitis: polyarteritis nodosa, allergic angiitis and granulomatosis, polyangiitis; Wegener's granulomatosis; lymphomatoid granulomatosis; and giant cell arteritis. Miscellaneous vasculitides include: mucocutaneous lymph node syndrome (MLNS or Kawasaki's disease), isolated CNS vasculitis, Behet's disease, thromboangiitis obliterans (Buerger's disease) and cutaneous necrotizing venulitis. The pathogenic mechanism of most of the types of vasculitis listed is believed to be primarily due to the deposition of immunoglobulin complexes in the vessel wall and subsequent induction of an inflammatory response either via ADCC, complement activation, or both.

Sarcoidosis is a condition of unknown etiology which is characterized by the presence of epithelioid granulomas in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

In autoimmune thrombocytopenia including thrombocytopenic purpura, and immune-mediated thrombocytopenia in other clinical settings, platelet destruction/removal occurs as a result of either antibody or complement attaching to platelets and subsequent removal by complement lysis, ADCC or FC-receptor mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase).

Type I diabetes mellitus or insulin-dependent diabetes is the autoimmune destruction of pancreatic islet β cells; this destruction is mediated by auto-antibodies and auto-reactive T cells. Antibodies to insulin or the insulin receptor can also produce the phenotype of insulin-non-responsiveness.

Immune mediated renal diseases, including glomerulonephritis and tubulointerstitial nephritis, are the result of antibody or T lymphocyte mediated injury to renal tissue either directly as a result of the production of autoreactive antibodies or T cells against renal antigens or indirectly as a result of the deposition of antibodies and/or immune complexes in the kidney that are reactive against other, non-renal antigens. Thus other immune-mediated diseases that result in the formation of immune-complexes can also induce immune mediated renal disease as an indirect sequelae. Both direct and indirect immune mechanisms result in inflammatory response that produces/induces lesion development in renal tissues with resultant organ function impairment and in some cases progression to renal failure. Both humoral and cellular immune mechanisms can be involved in the pathogenesis of lesions.

Demyelinating diseases of the central and peripheral nervous systems, including Multiple Sclerosis; idiopathic demyelinating polyneuropathy or Guillain-Barré syndrome; and Chronic Inflammatory Demyelinating Polyneuropathy, are believed to have an autoimmune basis and result in nerve demyelination as a result of damage caused to oligodendrocytes or to myelin directly. In MS there is evidence to suggest that disease induction and progression is dependent on T lymphocytes. Multiple Sclerosis is a demyelinating disease that is T lymphocyte-dependent and has either a relapsing-remitting course or a chronic progressive course. The etiology is unknown; however, viral infections, genetic predisposition, environment, and autoimmunity all contribute. Lesions contain infiltrates of predominantly T lymphocyte mediated, microglial cells and infiltrating macrophages; CD4+ T lymphocytes are the predominant cell type at lesions.

The mechanism of oligodendrocyte cell death and subsequent demyelination is not known but is likely T lymphocyte driven.

Inflammatory and Fibrotic Lung Disease, including Eosinophilic Pneumonias; Idiopathic Pulmonary Fibrosis, and Hypersensitivity Pneumonitis may involve a disregulated immune-inflammatory response. Inhibition of that response would be of therapeutic benefit.

Autoimmune or Immune-mediated Skin Disease including Bullous Skin Diseases, Erythema Multiforme, and Contact Dermatitis are mediated by auto-antibodies, the genesis of which is T lymphocyte-dependent.

Psoriasis is a T lymphocyte-mediated inflammatory disease. Lesions contain infiltrates of T lymphocytes, macrophages and antigen processing cells, and some neutrophils.

Allergic diseases, including asthma; allergic rhinitis; atopic dermatitis; food hypersensitivity; and urticaria are T lymphocyte dependent. These diseases are predominantly mediated by T lymphocyte induced inflammation, IgE mediated-inflammation or a combination of both.

Transplantation associated diseases, including Graft rejection and Graft-Versus-Host-Disease (GVHD) are T lymphocyte-dependent; inhibition of T lymphocyte function is ameliorative.

Other diseases in which intervention of the immune and/or inflammatory response have benefit are infectious disease including but not limited to viral infection (including but not limited to AIDS, hepatitis A, B, C, D, E and herpes) bacterial infection, fungal infections, and protozoal and parasitic infections (molecules (or derivatives/agonists) which stimulate the MLR can be utilized therapeutically to enhance the immune response to infectious agents), diseases of immunodeficiency (molecules/derivatives/agonists) which stimulate the MLR can be utilized therapeutically to enhance the immune response for conditions of inherited, acquired, infectious induced (as in HIV infection), or iatrogenic (i.e., as from chemotherapy) immunodeficiency, and neoplasia.

It has been demonstrated that some human cancer patients develop an antibody and/or T lymphocyte response to antigens on neoplastic cells. It has also been shown in animal models of neoplasia that enhancement of the immune response can result in rejection or regression of that particular neoplasm. Molecules that enhance the T lymphocyte response in the MLR have utility *in vivo* in enhancing the immune response against neoplasia. Molecules which enhance the T lymphocyte proliferative response in the MLR (or small molecule agonists or antibodies that affected the same receptor in an agonistic fashion) can be used therapeutically to treat cancer. Molecules that inhibit the lymphocyte response in the MLR also function *in vivo* during neoplasia to suppress the immune response to a neoplasm; such molecules can either be expressed by the neoplastic cells themselves or their expression can be induced by the neoplasm in other cells. Antagonism of such inhibitory molecules (either with antibody, small molecule antagonists or other means) enhances immune-mediated tumor rejection.

Additionally, inhibition of molecules with proinflammatory properties may have therapeutic benefit in reperfusion injury; stroke; myocardial infarction; atherosclerosis; acute lung injury; hemorrhagic shock; burn; sepsis/septic shock; acute tubular necrosis; endometriosis; degenerative joint disease and pancreatis.

The compounds of the present invention, e.g., polypeptides or antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides and antibodies is preferred.

In immunoadjuvant therapy, other therapeutic regimens, such administration of an anti-cancer agent, may be combined with the administration of the proteins, antibodies or compounds of the instant invention. For example, the patient to be treated with a the immunoadjuvant of the invention may also receive an anti-cancer agent (chemotherapeutic agent) or radiation therapy. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the immunoadjuvant or may be given simultaneously therewith. Additionally, an anti-estrogen compound such as tamoxifen or an anti-progesterone such as onapristone (see, EP 616812) may be given in dosages known for such molecules.

It may be desirable to also administer antibodies against other immune disease associated or tumor associated antigens, such as antibodies which bind to CD20, CD11a, CD18, ErbB2, EGFR, ErbB3, ErbB4, or vascular endothelial factor (VEGF). Alternatively, or in addition, two or more antibodies binding the same or two or more different antigens disclosed herein may be coadministered to the patient. Sometimes, it may be beneficial to also administer one or more cytokines to the patient. In one embodiment, the PRO polypeptides are coadministered with a growth inhibitory agent. For example, the growth inhibitory agent may be administered first, followed by a PRO polypeptide. However, simultaneous administration or administration first is also contemplated. Suitable dosages for the growth inhibitory agent are those presently used and may be lowered due to the combined action (synergy) of the growth inhibitory agent and the PRO polypeptide.

For the treatment or reduction in the severity of immune related disease, the appropriate dosage of an a compound of the invention will depend on the type of disease to be treated, as defined above, the severity and course of the disease, whether the agent is administered for preventive or therapeutic purposes, previous therapy, the patient's clinical history and response to the compound, and the discretion of the attending physician. The compound is suitably administered to the patient at one time or over a series of treatments.

For example, depending on the type and severity of the disease, about 1 µg/kg to 15 mg/kg (*e.g.,* 0.1-20 mg/kg) of polypeptide or antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

### O. Articles of Manufacture

In another embodiment of the invention, an article of manufacture containing materials (*e.g.,* comprising a PRO molecule) useful for the diagnosis or treatment of the disorders described above is provided. The article of manufacture comprises a container and an instruction. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually a polypeptide or an antibody of the invention. An instruction or label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

### P. Diagnosis and Prognosis of Immune Related Disease

Cell surface proteins, such as proteins which are overexpressed in certain immune related diseases, are excellent targets for drug candidates or disease treatment. The same proteins along with secreted proteins encoded by the genes amplified in immune related disease states find additional use in the diagnosis and prognosis of these diseases. For example, antibodies directed against the protein products of genes amplified in multiple sclerosis, rheumatoid arthritis, or another immune related disease, can be used as diagnostics or prognostics.

For example, antibodies, including antibody fragments, can be used to qualitatively or quantitatively detect the expression of proteins encoded by amplified or overexpressed genes ("marker gene products"). The antibody preferably is equipped with a detectable, e.g., fluorescent label, and binding can be monitored by light microscopy, flow cytometry, fluorimetry, or other techniques known in the art. These techniques are particularly suitable, if the overexpressed gene encodes a cell surface protein Such binding assays are performed essentially as described above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* detection.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Microarray analysis of stimulated T-cells

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (in this instance, activated CD4+ T cells) sample is greater than hybridization signal of a probe from a control (in this instance, non-stimulated CD4 + T cells) sample, the gene or genes overexpressed in the test tissue are identified. The implication of this result is that an overexpressed protein in a test tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference.

In this experiment, CD4+ T cells were purified from a single donor using the RossetteSep™ protocol from (Stem Cell Technologies, Vancouver BC) which contains anti-CD8, anti-CD16, anti-CD19, anti-CD36 and anti-CD56 antibodies used to produce a population of isolated CD4 + T cells. Isolated CD4+ T cells were activated with an anti-CD3 antibody (used at a concentration that does not stimulate proliferation) together with either ICAM-1, anti-CD28 antibody or a combination of both ICAM-1, anti-CD28. At 24 or 72 hours cells were harvested, RNA extracted and analysis run on Affimax (Affymetrix Inc. Santa Clara, CA) U95A chips. Non-stimulated (resting) cells were harvested immediately after purification, and subjected to the same analysis. Genes were compared whose expression was upregulated at either of the two timepoints in activated vs. resting cells. These genes were also compared to a panel of normal tissues. A normal "universal" tissue control sample was prepared by pooling non-cancerous, human tissues including liver, kidney, and lung. Microarray hybridization experiments using the universal control samples generated a linear plot in a 2-color analysis. The slope of the line generated in a 2-color analysis was then used to normalize the ratios of (test:control detection) within each experiment. The normalized ratios from various experiments were then compared and used to identify clustering of gene expression. Thus, the universal control sample not only allowed effective relative gene expression determinations in a simple 2-sample comparison, it also allowed multi-sample comparisons across several experiments.

Below are the results of these experiments, demonstrating that various PRO polypeptides of the present invention are significantly overexpressed in isolated CD4 + T cells activated by ICAM-1, anti-CD 28, or a combination of ICAM-1/anti-CD28 as compared to isolated resting CD4+ T cells. As described above, these data demonstrate that the PRO polypeptides of the present invention are useful not only as diagnostic markers for the presence of one or more immune disorders, but also serve as therapeutic targets for the treatment of those immune disorders.
Figures 1-280 are the PRO polypeptides increased by ICAM-1/anti-CD28.
Figures 281-496 are the PRO polypeptides increased by ICAM-1.
Figures 497-742 are the PRO polypeptides increased by anti-CD28.

### EXAMPLE 2: Use of PRO as a hybridization probe

The following method describes use of a nucleotide sequence encoding PRO as a hybridization probe.

DNA comprising the coding sequence of full-length or mature PRO as disclosed herein is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high stringency conditions. Hybridization of radiolabeled PRO-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PRO can then be identified using standard techniques known in the art.

### EXAMPLE 3: Expression of PRO in E. coli

This example illustrates preparation of an unglycosylated form of PRO by recombinant expression in E. *coli.*

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E*. *coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E*. *coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) lon galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g (NH₄)₂SO₄, 0.71 g sodium citrate•2H2O, 1.07 g KCl, 5.36 g Difco yeast extract, 5.36 g Sheffield hycase SF in 500 mL water, as well as 110 mM MPOS, pH 7.3, 0.55% (w/v) glucose and 7 mM MgSO₄) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1% TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as described above.

### EXAMPLE 4: Expression of PRO in mammalian cells

This example illustrates preparation of a potentially glycosylated form of PRO by recombinant expression in mammalian cells.

The vector, pRK5 (see EP 307,247, published March 15, 1989), is employed as the expression vector. Optionally, the PRO DNA is ligated into pRK5 with selected restriction enzymes to allow insertion of the PRO DNA using ligation methods such as described in Sambrook et al., supra. The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PRO DNA is mixed with about 1 µg DNA encoding the VA RNA gene [Thimmappaya et al., Cell, 31:543 (1982)] and dissolved in 500 µl of 1 mM Tris-HCl, 0.1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 1 ttg/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of PRO polypeptide, the culture medium may be replaced with serum free medium. Preferably, the cultures are incubated for about 6 days, and then the conditioned medium is harvested. The medium containing the expressed PRO can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-his tag into a Baculovirus expression vector. The poly-his tagged PRO insert can then be subcloned into a SV40 promoter/enhancer containing vector containing a selection marker such as DHFR for selection of stable clones. Finally, the CHO cells can be transfected (as described above) with the SV40 promoter/enhancer containing vector. Labeling may be performed, as described above, to verify expression. The culture medium containing the expressed poly-His tagged PRO can then be concentrated and purified by any selected method, such as by Ni²⁺-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect^{®} (Quiagen), Dosper^{®} or Fugene^{®} (Boehringer Mannheim). The cells are grown as described in Lucas et al., supra. Approximately 3 x 10⁻⁷ cells are frozen in an ampule for further growth and production as described below.

The ampules containing the plasmid DNA are thawed by placement into water bath and mixed by vortexing. The contents are pipetted into a centrifuge tube containing 10 mL of media and centrifuged at 1000 rpm for 5 minutes. The supernatant is aspirated and the cells are resuspended in 10 mL of selective media (0.2 µm filtered PS20 with 5% 0.2 µm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x 10⁵ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/mL. On day 0, pH is determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Coming 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 µm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 µl of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as described above.

### EXAMPLE 5: Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

### EXAMPLE 6: Expression of PRO in Baculovirus-Infected Insect Cells

The following method describes recombinant expression of PRO in Baculovirus-infected insect cells.

The sequence coding for PRO is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{™} virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or Western blot with Ni²⁺-NTA-conjugated to alkaline phosphatase (Qiagen). Fractions containing the eluted His₁₀-tagged PRO are pooled and dialyzed against loading buffer.

Alternatively, purification of the IgG tagged (or Fc tagged) PRO can be performed using known chromatography techniques, including for instance, Protein A or protein G column chromatography.

Many of the PRO polypeptides disclosed herein were successfully expressed as described above.

### EXAMPLE 7: Preparation of Antibodies that Bind PRO

This example illustrates preparation of monoclonal antibodies which can specifically bind PRO.

Techniques for producing the monoclonal antibodies are known in the art and are described, for instance, in Goding, supra. Immunogens that may be employed include purified PRO, fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 8: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE^{™} (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide (e.g., high ionic strength buffers in the presence of detergent). Then, the column is eluted under conditions that disrupt antibody/PRO polypeptide binding (e.g., a low pH buffer such as approximately pH 2-3, or a high concentration of a chaotrope such as urea or thiocyanate ion), and PRO polypeptide is collected.

### EXAMPLE 9: Drug Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (I) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

### EXAMPLE 10: Rational Drug Design

The goal of rational drug design is to produce structural analogs of biologically active polypeptide of interest (*i.e.,* a PRO polypeptide) or of small molecules with which they interact, *e.g.,* agonists, antagonists, or inhibitors. Any of these examples can be used to fashion drugs which are more active or stable forms of the PRO polypeptide or which enhance or interfere with the function of the PRO polypeptide *in vivo,(c.f.,* Hodgson, Bio/Technology, 9: 19-21 (1991)).

In one approach, the three-dimensional structure of the PRO polypeptide, or of a PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry. 31:7796-7801 (1992) or which act as inhibitors, agonists, or antagonists of native peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

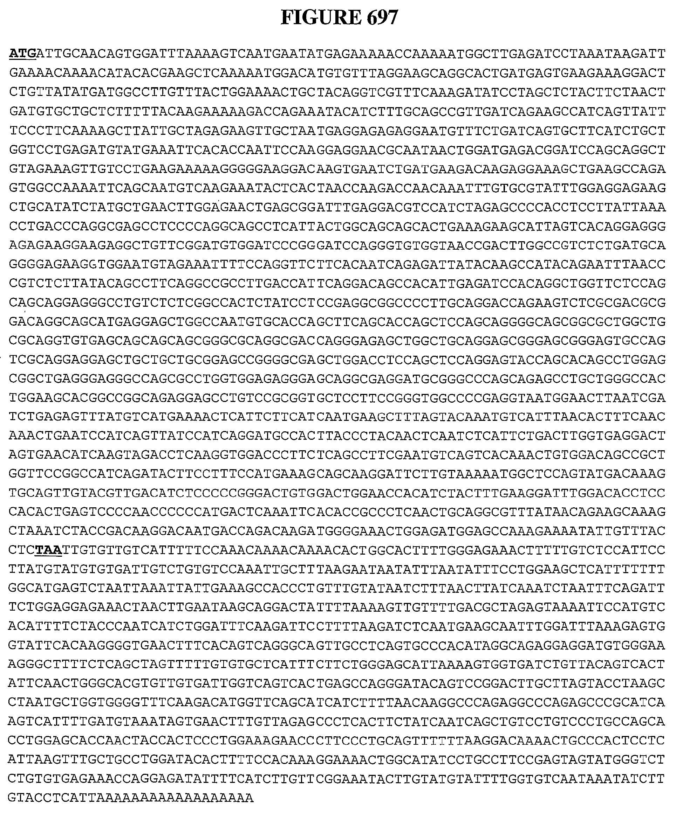

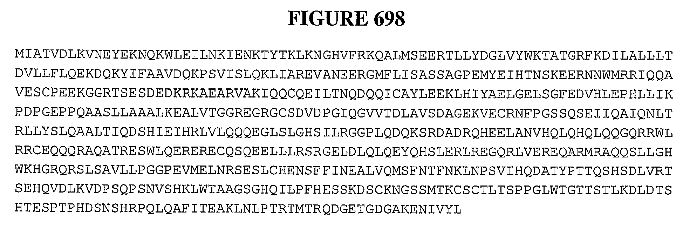

The following numbered paragraphs (paras.) contain further statements of various aspects of the present invention:-
1. Isolated nucleic acid having at least 80% nucleic acid sequence identity to:
   (a) a nucleotide sequence encoding the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 8 (SEQ ID NO:14), Figure 8 (SEQ ID NO:14), Figure 16 (SEQ ID NO:16), Figure 18 (SEQ ID NO:18), Figure 20 (SEQ ID NO:20), Figure 22 (SEQ ID N0:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID N0:40), Figure 42 (SEQ ID NO:42), Figure 44 (SEQ ID NO :44), Figure 46 (SEQ ID NO:46), Figure 48 (SEQ ID NO :48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110 (SEQ ID NO:110), Figure 112 (SEQ ID NO:112), Figure 114 (SEQ ID NO:144), Figure 116 (SEQ ID NO:116), Figure 118 (SEQ ID NO:118), Figure 120 (SEQ ID NO:120), Figure 122 (SEQ ID NO:122), Figure 124 (SEQ ID NO:124), Figure 126 (SEQ ID NO:126), Figure 128 (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144 (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156 (SEQ ID NO:156), Figure 158 (SEQ ID NO:158), Figure 160 (SEQ ID NO:160), Figure 162 (SEQ ID NO:162), Figure 164 (SEQ ID N0:164), Figure 166 (SEQ ID NO:166), Figure 168 (SEQ ID NO:168), Figure 170 (SEQ ID NO:170), Figure 172 (SEQ ID NO:172), Figure 174 (SEQ ID NO:174), Figure 176 (SEQ ID NO:176), Figure 178 (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190 (SEQ ID NO:190), Figure 192 (SEQ ID NO:192), Figure 194 (SEQ ID NO:194), Figure 196 (SEQ ID NO:196), Figure 198 (SEQ ID NO:198), Figure 200 (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216 (SEQ ID NO:216), Figure 218 (SEQ ID NO:218), Figure 220 (SEQ ID NO:220), Figure 222 (SEQ ID NO:222), Figure 224 (SEQ ID NO:224), Figure 226 (SEQ ID NO:226), Figure 228 (SEQ ID NO:228), Figure 230 (SEQ ID NO:230), Figure 232 (SEQ ID NO:232), Figure 234 (SEQ ID NO:234), Figure 236 (SEQ ID NO:236), Figure 238 (SEQ ID NO:238), Figure 240 (SEQ ID NO:240), Figure 242 (SEQ ID NO:242), Figure 244 (SEQ ID NO:244), Figure 246 (SEQ ID NO:246), Figure 248 (SEQ ID NO:248), Figure 250 (SEQ ID NO:250), Figure 252 (SEQ ID NO:252), Figure 254 (SEQ ID NO:254), Figure 256 (SEQ ID NO:256), Figure 258 (SEQ ID NO:258), Figure 260 (SEQ ID NO:260), Figure 262 (SEQ ID NO:262), Figure 264 (SEQ ID NO:264), Figure 266 (SEQ ID NO:266), Figure 268 (SEQ ID NO:268), Figure 270 (SEQ ID NO:270), Figure 272 (SEQ ID NO:272), Figure 274 (SEQ ID NO:274), Figure 276 (SEQ ID NO:276), Figure 278 (SEQ ID NO:278), Figure 280 (SEQ ID NO:280), Figure 282 (SEQ ID NO:282), Figure 284 (SEQ ID N0:284), Figure 286 (SEQ ID N0:286), Figure 288 (SEQ ID NO:288), Figure 290 (SEQ ID NO:290), Figure 292 (SEQ ID NO:292), Figure 294 (SEQ ID NO:294), Figure 296 (SEQ ID NO:296), Figure 298 (SEQ ID NO:298), Figure 300 (SEQ ID NO:300), Figure 302 (SEQ ID NO:302), Figure 304 (SEQ ID NO:304), Figure 306 (SEQ ID NO:306), Figure 308 (SEQ ID NO:308), Figure 310 (SEQ ID NO:310), Figure 312 (SEQ ID NO:312), Figure 314 (SEQ ID NO:314), Figure 316 (SEQ ID NO:316), Figure 318 (SEQ ID NO:318), Figure 320 (SEQ ID NO:320), Figure 322 (SEQ ID NO:322), Figure 324 (SEQ ID NO:324), Figure 326 (SEQ ID NO:326), Figure 328 (SEQ ID NO:328), Figure 330 (SEQ ID NO:330), Figure 332 (SEQ ID NO:332), Figure 334 (SEQ ID NO:334), Figure 336 (SEQ ID NO:336), Figure 338 (SEQ ID NO:338), Figure 340 (SEQ ID NO:340), Figure 342 (SEQ ID NO:342), Figure 344 (SEQ ID NO:344), Figure 346 (SEQ ID NO:346), Figure 348 (SEQ ID NO:348), Figure 350 (SEQ ID NO:350), Figure 352 (SEQ ID NO:352), Figure 354 (SEQ ID NO:354), Figure 356 (SEQ ID NO:356), Figure 358 (SEQ ID NO:358), Figure 360 (SEQ ID NO:360), Figure 362 (SEQ ID NO:362), Figure 364 (SEQ ID NO:364), Figure 366 (SEQ ID NO:366), Figure 368 (SEQ ID NO:368), Figure 370 (SEQ ID NO:370), Figure 372 (SEQ ID NO:372), Figure 374 (SEQ ID NO:374), Figure 376 (SEQ ID NO:376), Figure 378 (SEQ ID NO:378), Figure 380 (SEQ ID NO:380), Figure 382 (SEQ ID NO:382), Figure 384 (SEQ ID NO:384), Figure 386 (SEQ ID NO:386), Figure 390 (SEQ ID NO:390), Figure 392 (SEQ ID NO:392), Figure 394 (SEQ ID NO:394), Figure 396 (SEQ ID NO:396), Figure 398 (SEQ ID NO:398), Figure 400 (SEQ ID NO:400), Figure 402 (SEQ ID NO:402), Figure 404 (SEQ ID NO:404), Figure 406 (SEQ ID NO:406), Figure 408 (SEQ ID NO:408), Figure 410 (SEQ ID NO:410), Figure 412 (SEQ ID NO:412), Figure 414 (SEQ ID NO:414), Figure 416 (SEQ ID NO:416), Figure 418 (SEQ ID NO:418), Figure 420 (SEQ ID NO:420), Figure 422 (SEQ ID NO:422), Figure 424 (SEQ ID NO:424), Figure 426 (SEQ ID NO:426), Figure 428 (SEQ ID NO:428), Figure 430 (SEQ ID NO:430), Figure 432 (SEQ ID NO:432), Figure 434 (SEQ ID NO:434), Figure 436 (SEQ ID NO:436), Figure 438 (SEQ ID NO:438), Figure 440 (SEQ ID NO:440), Figure 442 (SEQ ID NO:442), Figure 444 (SEQ ID NO:444), Figure 446 (SEQ ID NO:446), Figure 448 (SEQ ID NO:448), Figure 450 (SEQ ID NO:450), Figure 452 (SEQ ID NO:452), Figure 454 (SEQ ID NO:454), Figure 456 (SEQ ID NO:456), Figure 458 (SEQ ID NO:458), Figure 460 (SEQ ID NO:460), Figure 462 (SEQ ID NO:462), Figure 464 (SEQ ID NO:464), Figure 466 (SEQ ID NO:466), Figure 468 (SEQ ID NO:468), Figure 470 (SEQ ID NO:470), Figure 472 (SEQ ID NO:472), Figure 474 (SEQ ID NO:474), Figure 476 (SEQ ID NO:476), Figure 478 (SEQ ID NO:478), Figure 480 (SEQ ID NO:480), Figure 482 (SEQ ID NO:482), Figure 484 (SEQ ID NO:484), Figure 486 (SEQ ID NO:486), Figure 488 (SEQ ID NO:488), Figure 490 (SEQ ID NO:490), Figure 492 (SEQ ID NO:492), Figure 494 (SEQ ID NO:494), Figure 496 (SEQ ID NO:496), Figure 498 (SEQ ID NO:498), Figure 500 (SEQ ID NO:500), Figure 502 (SEQ ID NO:502), Figure 504 (SEQ ID NO:504), Figure 506 (SEQ ID NO:506), Figure 508 (SEQ ID NO:508), Figure 510 (SEQ ID NO:510), Figure 512 (SEQ ID NO:512), Figure 514 (SEQ ID NO:514), Figure 516 (SEQ ID NO:516), Figure 518 (SEQ ID NO:518), Figure 520 (SEQ ID NO:520), Figure 522 (SEQ ID NO:522), Figure 524 (SEQ ID NO:524), Figure 526 (SEQ ID NO:526), Figure 528 (SEQ ID NO:528), Figure 530 (SEQ ID NO:530), Figure 532 (SEQ ID NO:532), Figure 534 (SEQ ID NO:534), Figure 536 (SEQ ID NO:536), Figure 540 (SEQ ID NO:540), Figure 542 (SEQ ID NO:542), Figure 544 (SEQ ID NO:544), Figure 546 (SEQ ID NO:546), Figure 548 (SEQ ID NO:548), Figure 550 (SEQ ID NO:550), Figure 552 (SEQ ID NO:552), Figure 554 (SEQ ID NO:554), Figure 556 (SEQ ID NO:556), Figure 558 (SEQ ID NO:558), Figure 560 (SEQ ID NO:560), Figure 562 (SEQ ID NO:562), Figure 564 (SEQ ID NO:564), Figure 566 (SEQ ID NO:566), Figure 568 (SEQ ID NO:568), Figure 570 (SEQ ID NO:570), Figure 572 (SEQ ID NO:572), Figure 574 (SEQ ID NO:574), Figure 576 (SEQ ID NO:576), Figure 578 (SEQ ID NO:578), Figure 580(SEQ ID NO:580), Figure 582 (SEQ ID NO:582), Figure 584 (SEQ ID NO:584), Figure 586 (SEQ ID NO:586), Figure 588 (SEQ ID NO:588), Figure 590 (SEQ ID NO:590), Figure 592 (SEQ ID NO:592), Figure 594 (SEQ ID NO:594), Figure 596 (SEQ ID NO:596), Figure 598 (SEQ ID NO:598), Figure 600 (SEQ ID NO:600), Figure 602 (SEQ ID NO:602), Figure 604 (SEQ ID NO:604), Figure 606 (SEQ ID NO:606), Figure 608 (SEQ ID NO:608), Figure 610 (SEQ ID N0:610), Figure 612 (SEQ ID NO:612), Figure 614 (SEQ ID NO:614), Figure 616 (SEQ ID NO:616), Figure 618 (SEQ ID NO:618), Figure 620 (SEQ ID NO:620), Figure 622 (SEQ ID NO:622), Figure 624 (SEQ ID NO:624), Figure 626 (SEQ ID NO:626), Figure 628 (SEQ ID NO:628), Figure 630 (SEQ ID NO:630), Figure 632 (SEQ ID NO:632), Figure 634 (SEQ ID NO:634), Figure 636 (SEQ ID NO:636), Figure 638 (SEQ ID NO:638), Figure 640 (SEQ ID NO:640), Figure 642 (SEQ ID NO:642), Figure 644 (SEQ ID NO:644), Figure 646 (SEQ ID NO:646), Figure 648 (SEQ ID NO:648), Figure 650 (SEQ ID NO:650), Figure 652 (SEQ ID NO:652), Figure 654 (SEQ ID NO:654), Figure 656 (SEQ ID NO:656), Figure 658 (SEQ ID NO:658), Figure 660 (SEQ ID NO:660), Figure 662 (SEQ ID NO:662), Figure 664 (SEQ ID NO:664), Figure 666 (SEQ ID NO:666), Figure 668 (SEQ ID NO:668), Figure 670 (SEQ ID NO:670), Figure 672 (SEQ ID NO:672), Figure 674 (SEQ ID NO:674), Figure 676 (SEQ ID NO:676), Figure 678 (SEQ ID NO:678), Figure 680 (SEQ ID NO:680), Figure 682 (SEQ ID NO:682), Figure 684 (SEQ ID NO:684), Figure 686 (SEQ ID NO:686), Figure 688 (SEQ ID NO:688), Figure 690 (SEQ ID NO:690), Figure 692 (SEQ ID NO:692), Figure 694 (SEQ ID NO:694), Figure 696 (SEQ ID NO:696), Figure 698 (SEQ ID NO:698), Figure 700 (SEQ ID NO:700), Figure 702 (SEQ ID NO:702), Figure 704 (SEQ ID NO:704), Figure 706 (SEQ ID NO:706), Figure 708 (SEQ ID NO:708), Figure 710 (SEQ ID NO:710), Figure 712 (SEQ ID NO:712), Figure 714 (SEQ ID N0:714), Figure 716 (SEQ ID NO:716), Figure 718 (SEQ ID NO:718), Figure 720 (SEQ ID NO:720), Figure 722 (SEQ ID NO:722), Figure 724 (SEQ ID NO:724), Figure 726 (SEQ ID NO:726), Figure 728 (SEQ ID NO:728), Figure 730 (SEQ ID NO:730), Figure 732 (SEQ ID NO:732), Figure 734 (SEQ ID NO:734), Figure 736 (SEQ ID NO:736), Figure 740 (SEQ ID NO:740), and Figure 742 (SEQ ID NO:742).
2. Isolated nucleic acid having at least 80% nucleic acid sequence identity to a nucleotide sequence selected from the group consisting of the nucleotide sequence shown in Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 11 (SEQ ID NO:11), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID N0:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), Figure 55 (SEQ ID NO:55), Figure 57 (SEQ ID NO:57), Figure 59 (SEQ ID NO:59), Figure 61 (SEQ ID NO:61), Figure 63 (SEQ ID NO:63), Figure 65 (SEQ ID NO:65), Figure 67 (SEQ ID NO:67), Figure 69 (SEQ ID NO:69), Figure 71 (SEQ ID NO:71), Figure 73 (SEQ ID NO:73), Figure 75 (SEQ ID NO:75), Figure 77 (SEQ ID NO:77), Figure 79 (SEQ ID NO:79), Figure 81 (SEQ ID NO:81), Figure 83 (SEQ ID NO:83), Figure 85 (SEQ ID NO:85), Figure 87 (SEQ ID NO:87), Figure 89 (SEQ ID NO:89), Figure 91 (SEQ ID NO:91), Figure 93 (SEQ ID NO:93), Figure 95 (SEQ ID NO:95), Figure 97 (SEQ ID NO:97), Figure 99 (SEQ ID NO:99), Figure 101 (SEQ ID NO:101), Figure 103 (SEQ ID NO:103), Figure 105 (SEQ ID NO:105), Figure 107 (SEQ ID NO:107), Figure 109 (SEQ ID NO:109), Figure 111 (SEQ ID NO:111), Figure 113 (SEQ ID NO:113), Figure 115 (SEQ m NO:115), Figure 117 (SEQ ID NO:117), Figure 119 (SEQ ID NO:119), Figure 121 (SEQ ID NO:121), Figure 123 (SEQ ID NO:123), Figure 125 (SEQ ID NO:125), Figure 127 (SEQ ID NO:127), Figure 131 (SEQ ID NO:131), Figure 133 (SEQ ID NO:133), Figure 135 (SEQ ID NO:135), Figure 137 (SEQ ID NO:137), Figure 139 (SEQ ID NO:139), Figure 141 (SEQ ID NO:141), Figure 143 (SEQ ID NO:143), Figure 145 (SEQ ID NO:145), Figure 147 (SEQ ID NO:147), Figure 149 (SEQ ID NO:149), Figure 151 (SEQ ID NO:151), Figure 153 (SEQ ID NO:153), Figure 155 (SEQ ID NO:155), Figure 157 (SEQ ID NO:157), Figure 159 (SEQ ID NO:159), Figure 161 (SEQ ID NO:161), Figure 163 (SEQ ID NO:163), Figure 165 (SEQ ID NO:165), Figure 167 (SEQ ID NO:167), Figure 169 (SEQ ID NO:169), Figure 171 (SEQ ID NO:171), Figure 173 (SEQ ID NO:173), Figure 175 (SEQ ID NO:175), Figure 177 (SEQ ID NO:177), Figure 179 (SEQ ID NO:179), Figure 181 (SEQ ID NO:181), Figure 183 (SEQ ID NO:183), Figure 185 (SEQ ID NO:185), Figure 187 (SEQ ID NO:187), Figure 189 (SEQ ID NO:189), Figure 191 (SEQ ID NO:191), Figure 193 (SEQ ID NO:193), Figure 195 (SEQ ID NO:195), Figure 197 (SEQ ID NO:197), Figure 199 (SEQ ID NO:199), Figure 201 (SEQ ID NO:201), Figure 203 (SEQ ID NO:203), Figure 205 (SEQ ID NO:205), Figure 207 (SEQ ID NO:207), Figure 209 (SEQ ID NO:209), Figure 211 (SEQ ID NO:211), Figure 213 (SEQ ID NO:213), Figure 215 (SEQ ID N0:215), Figure 217 (SEQ ID NO:217), Figure 219 (SEQ ID NO:219), Figure 221 (SEQ ID NO:221), Figure 223 (SEQ ID NO:223), Figure 225 (SEQ ID NO:225), Figure 227 (SEQ ID NO:227), Figure 229 (SEQ ID NO:229), Figure 231 (SEQ ID NO:231), Figure 231 (SEQ ID NO:231), Figure 233 (SEQ ID NO:233), Figure 235 (SEQ ID NO:235), Figure 237 (SEQ ID NO:237), Figure 239 (SEQ ID NO:239), Figure 241 (SEQ ID NO:241), Figure 243 (SEQ ID NO:243), Figure 245 (SEQ ID NO:245), Figure 247 (SEQ ID NO:247), Figure 249 (SEQ ID NO:249), Figure 251 (SEQ ID NO:251), Figure 253 (SEQ ID NO:253), Figure 255 (SEQ ID NO:255), Figure 257 (SEQ ID NO:257), Figure 259 (SEQ ID NO:259), Figure 261 (SEQ ID NO:261), Figure 263 (SEQ ID NO:263), Figure 265 (SEQ ID NO:265), Figure 267 (SEQ ID NO:267), Figure 269 (SEQ ID NO:269), Figure 271 (SEQ ID NO:271), Figure 273 (SEQ ID NO:273), Figure 275 (SEQ ID NO:275), Figure 277 (SEQ ID NO:277), Figure 279 (SEQ ID NO:279), Figure 281 (SEQ ID NO:281), Figure 283 (SEQ ID NO:283), Figure 285 (SEQ ID NO:285), Figure 287 (SEQ ID NO:287), Figure 289 (SEQ ID NO:289), Figure 291 (SEQ ID NO:291), Figure 293 (SEQ ID NO:293), Figure 295 (SEQ ID NO:295), Figure 297 (SEQ ID NO:297), Figure 299 (SEQ ID NO:299), Figure 301 (SEQ ID NO:301), Figure 303(SEQ ID NO:303), Figure 305 (SEQ ID NO:305), Figure 307 (SEQ ID NO:307), Figure 309 (SEQ ID NO:309), Figure 311 (SEQ ID NO:311), Figure 313 (SEQ ID NO:313), Figure 315 (SEQ ID NO:315), Figure 317 (SEQ ID NO:317), Figure 319 (SEQ ID NO:319), Figure 321 (SEQ ID NO:321), Figure 323 (SEQ ID NO:323), Figure 325 (SEQ ID NO:325), Figure 327 (SEQ ID NO:327), Figure 329 (SEQ ID NO:329), Figure 331 (SEQ ID NO:331), Figure 333 (SEQ ID NO:333), Figure 335 (SEQ ID NO:335), Figure 337 (SEQ ID NO:337), Figure 339 (SEQ ID NO:339), Figure 341 (SEQ ID NO:341), Figure 343 (SEQ ID NO:343), Figure 345 (SEQ ID NO:345), Figure 347 (SEQ ID NO:347), Figure 349 (SEQ ID NO:349), Figure 349 (SEQ ID NO:349), Figure 351 (SEQ ID NO:351), Figure 353 (SEQ ID NO:353), Figure 355 (SEQ ID NO:355), Figure 357 (SEQ ID NO:357), Figure 359 (SEQ ID NO:359), Figure 361 (SEQ ID NO:361), Figure 363 (SEQ ID NO:363), Figure 365 (SEQ ID NO:365), Figure 367 (SEQ ID NO:367), Figure 369 (SEQ ID NO:369), Figure 371 (SEQ ID NO:371), Figure 373 (SEQ ID NO:373), Figure 375 (SEQ ID NO:375), Figure 377 (SEQ ID NO:377), Figure 379 (SEQ ID NO:379), Figure 381 (SEQ ID NO:381), Figure 383 (SEQ ID NO:383), Figure 385 (SEQ ID NO:385), Figure 387 (SEQ ID NO:387), Figure 389 (SEQ ID NO:389), Figure 391 (SEQ ID NO:391), Figure 393 (SEQ ID NO:393), Figure 395 (SEQ ID NO:395), Figure 397 (SEQ ID NO:397), Figure 399 (SEQ ID NO:399), Figure 401 (SEQ ID NO:401), Figure 403 (SEQ ID NO:403), Figure 405 (SEQ ID NO:405), Figure 407 (SEQ ID NO:407), Figure 409 (SEQ ID NO:409), Figure 411 (SEQ ID NO:411), Figure 413 (SEQ ID NO:413), Figure 415 (SEQ ID NO:415), Figure 417 (SEQ ID NO:417), Figure 419 (SEQ ID NO:419), Figure 421 (SEQ ID NO:421), Figure 423 (SEQ ID NO:423), Figure 425 (SEQ ID NO:425), Figure 427 (SEQ ID NO:427), Figure 429 (SEQ ID NO:429), Figure 431 (SEQ ID NO:431), Figure 433 (SEQ ID NO:433), Figure 435 (SEQ ID NO:435), Figure 437 (SEQ ID NO:437), Figure 439 (SEQ ID NO:439), Figure 441 (SEQ ID NO:441), Figure 443 (SEQ ID NO:443), Figure 445 (SEQ ID NO:445), Figure 447 (SEQ ID NO:447), Figure 449 (SEQ ID NO:449), Figure 451 (SEQ ID NO:451), Figure 453 (SEQ ID NO:453), Figure 455 (SEQ ID NO:455), Figure 457 (SEQ ID NO:457), Figure 459 (SEQ ID NO:459), Figure 461 (SEQ ID NO:461), Figure 463 (SEQ ID NO:463), Figure 465 (SEQ ID NO:465), Figure 467 (SEQ ID NO:467), Figure 469 (SEQ ID NO:469), Figure 471 (SEQ ID NO:471), Figure 473 (SEQ ID NO:473), Figure 475 (SEQ ID NO:475), Figure 477 (SEQ ID NO:477), Figure 479 (SEQ ID NO:479), Figure 481 (SEQ ID NO:481), Figure 483 (SEQ ID NO:483), Figure 485 (SEQ ID NO:485), Figure 487 (SEQ ID NO:487), Figure 489 (SEQ ID NO:489), Figure 491 (SEQ ID NO:491), Figure 493 (SEQ ID NO:493), Figure 495 (SEQ ID NO:495), Figure 497 (SEQ ID NO:497), Figure 499 (SEQ ID NO:499), Figure 501 (SEQ ID NO:501), Figure 503 (SEQ ID NO:503), Figure 505 (SEQ ID NO:505), Figure 507 (SEQ ID NO:507), Figure 509 (SEQ ID NO:509), Figure 511 (SEQ ID NO:511), Figure 513 (SEQ ID NO:513), Figure 515 (SEQ ID NO:515), Figure 517 (SEQ ID NO:517), Figure 519 (SEQ ID NO:519), Figure 521 (SEQ ID NO:521), Figure 523 (SEQ ID NO:523), Figure 525 (SEQ ID NO:525), Figure 527 (SEQ ID NO:527), Figure 529 (SEQ ID NO:529), Figure 531 (SEQ ID NO:531), Figure 533 (SEQ ID NO:533), Figure 535 (SEQ ID NO:535), Figure 537 (SEQ ID NO:537), Figure 539 (SEQ ID NO:539), Figure 541 (SEQ ID NO:541), Figure 543 (SEQ ID NO:543), Figure 545 (SEQ ID NO:545), Figure 547 (SEQ ID NO:547), Figure 549 (SEQ ID NO:549), Figure 551 (SEQ ID NO:551), Figure 553 (SEQ ID NO:553), Figure 555 (SEQ ID NO:555), Figure 557 (SEQ ID NO:557), Figure 559 (SEQ ID NO:559), Figure 561 (SEQ ID NO:561), Figure 563 (SEQ ID NO:563), Figure 565 (SEQ ID NO:565), Figure 567 (SEQ ID NO:567), Figure 569 (SEQ ID NO:569), Figure 571 (SEQ ID NO:571), Figure 573 (SEQ ID NO:573), Figure 575 (SEQ ID NO:575), Figure 577 (SEQ ID NO:577), Figure 579 (SEQ ID NO:579), Figure 581 (SEQ ID NO:581), Figure 583 (SEQ ID NO:583), Figure 585 (SEQ ID NO:585), Figure 587 (SEQ ID NO:587), Figure 589 (SEQ ID NO:589), Figure 591 (SEQ ID NO:591), Figure 593 (SEQ ID NO:593), Figure 595 (SEQ ID NO:595), Figure 597 (SEQ ID NO:597), Figure 599 (SEQ ID NO:599), Figure 601 (SEQ ID NO:601), Figure 603 (SEQ ID NO:603), Figure 605 (SEQ ID NO:605), Figure 607 (SEQ ID NO:607), Figure 609 (SEQ ID NO:609), Figure 611 (SEQ ID NO:611), Figure 613 (SEQ ID NO:613), Figure 615 (SEQ ID NO:615), Figure 617 (SEQ ID NO:617), Figure 619 (SEQ ID NO:619), Figure 621 (SEQ ID NO:621), Figure 623 (SEQ ID NO:623), Figure 625 (SEQ ID NO:625), Figure 627 (SEQ ID NO:627), Figure 629 (SEQ ID NO:629), Figure 631 (SEQ ID NO:631), Figure 633 (SEQ ID NO:633), Figure 635 (SEQ ID NO:635), Figure 637 (SEQ ID NO:637), Figure 639 (SEQ ID NO:639), Figure 641 (SEQ ID NO:641), Figure 643 (SEQ ID NO:643), Figure 645 (SEQ ID NO:645), Figure 647 (SEQ ID NO:647), Figure 649 (SEQ ID NO:649), Figure 651 (SEQ ID NO:651), Figure 653 (SEQ ID NO:653), Figure 655 (SEQ ID NO:655), Figure 657 (SEQ ID NO:657), Figure 659 (SEQ ID NO:659), Figure 661 (SEQ ID NO:661), Figure 663 (SEQ ID NO:663), Figure 665 (SEQ ID NO:665), Figure 667 (SEQ ID NO:667), Figure 669 (SEQ ID NO:669), Figure 671 (SEQ ID NO:671), Figure 673 (SEQ ID NO:673), Figure 675 (SEQ ID NO:675), Figure 677 (SEQ ID NO:677), Figure 679 (SEQ ID NO:679), Figure 681 (SEQ ID NO:681), Figure 683 (SEQ ID NO:683), Figure 685 (SEQ ID NO:685), Figure 687 (SEQ ID NO:687), Figure 689 (SEQ ID NO:689), Figure 691 (SEQ ID NO:691), Figure 693 (SEQ ID NO:693), Figure 695 (SEQ ID NO:695), Figure 697 (SEQ ID NO:697), Figure 699 (SEQ ID NO:699), Figure 701 (SEQ ID NO:701), Figure 703 (SEQ ID NO:703), Figure 705 (SEQ ID NO:705), Figure 707 (SEQ ID NO:707), Figure 709 (SEQ ID NO:709), Figure 711 (SEQ ID NO:711), Figure 713 (SEQ ID NO:713), Figure 715 (SEQ ID NO:715), Figure 717 (SEQ ID NO:717), Figure 719 (SEQ ID NO:719), Figure 721 (SEQ ID NO:721), Figure 723 (SEQ ID NO:723), Figure 725 (SEQ ID NO:725), Figure 727 (SEQ ID NO:727), Figure 729 (SEQ ID NO:729), Figure 731 (SEQ ID NO:731), Figure 733 (SEQ ID NO:733), Figure 735 (SEQ ID NO:735), Figure 737 (SEQ ID NO:737), Figure 739 (SEQ ID NO:739), and Figure 741 (SEQ ID NO:741).
3. Isolated nucleic acid having at least 80% nucleic acid sequence identity to a nucleotide sequence selected from the group consisting of the full-length coding sequence of the nucleotide sequence shown in Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 11 (SEQ ID NO:11), Figure 11 (SEQ ID NO:11), Figure 13 (SEQ ID NO:13), Figure 15 (SEQ ID NO:15), Figure 17 (SEQ ID NO: 17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), Figure 55 (SEQ ID NO:55), Figure 57 (SEQ ID NO:57), Figure 59 (SEQ ID NO:59), Figure 61 (SEQ ID NO:61), Figure 63 (SEQ ID NO:63), Figure 65 (SEQ ID NO:65), Figure 67 (SEQ ID NO:67), Figure 69 (SEQ ID NO:69), Figure 71 (SEQ ID NO:71), Figure 73 (SEQ ID NO:73), Figure 75 (SEQ ID NO:75), Figure 77 (SEQ ID NO:77), Figure 79 (SEQ ID NO:79), Figure 81 (SEQ ID NO:81), Figure 83 (SEQ ID NO:83), Figure 85 (SEQ ID NO:85), Figure 87 (SEQ ID NO:87), Figure 89 (SEQ ID NO:89), Figure 91 (SEQ ID NO:91), Figure 93 (SEQ ID NO:93), Figure 95 (SEQ ID NO:95), Figure 97 (SEQ ID NO:97), Figure 99 (SEQ ID NO:99), Figure 101 (SEQ ID NO:101), Figure 103 (SEQ ID NO:103), Figure 105 (SEQ ID NO:105), Figure 107 (SEQ ID NO:107), Figure 109 (SEQ ID NO:109), Figure 111 (SEQ ID NO:111), Figure 113 (SEQ ID NO:113), Figure 115 (SEQ ID NO:115), Figure 117 (SEQ ID NO:117), Figure 119 (SEQ ID NO:119), Figure 121 (SEQ ID NO:121), Figure 123 (SEQ ID NO:123), Figure 125 (SEQ ID NO:125), Figure 127 (SEQ ID NO:127), Figure 131 (SEQ ID NO:131), Figure 133 (SEQ ID NO:133), Figure 135 (SEQ ID NO:135), Figure 137 (SEQ ID NO:137), Figure 139 (SEQ ID NO:139), Figure 141 (SEQ ID NO:141), Figure 143 (SEQ ID NO:143), Figure 145 (SEQ ID NO:145), Figure 147 (SEQ ID NO:147), Figure 149 (SEQ ID NO:149), Figure 151 (SEQ ID NO:151), Figure 153 (SEQ ID NO:153), Figure 155 (SEQ ID NO:155), Figure 157 (SEQ ID NO:157), Figure 159 (SEQ ID NO:159), Figure 161 (SEQ ID NO:161), Figure 163 (SEQ ID NO:163), Figure 165 (SEQ ID NO:165), Figure 167 (SEQ ID NO:167), Figure 169 (SEQ ID NO:169), Figure 171 (SEQ ID NO:171), Figure 173 (SEQ ID NO:173), Figure 175 (SEQ ID NO:175), Figure 177 (SEQ ID NO:177), Figure 179 (SEQ ID NO:179), Figure 181 (SEQ ID NO:181), Figure 183 (SEQ ID NO:183), Figure 185 (SEQ ID NO:185), Figure 187 (SEQ ID NO:187), Figure 189 (SEQ ID NO:189), Figure 191 (SEQ ID NO:191), Figure 193 (SEQ ID NO:193), Figure 195 (SEQ ID NO:195), Figure 197 (SEQ ID NO:197), Figure 199 (SEQ ID NO:199), Figure 201 (SEQ ID NO:201), Figure 203 (SEQ ID NO:203), Figure 205 (SEQ ID NO:205), Figure 207 (SEQ ID NO:207), Figure 209 (SEQ ID NO:209), Figure 211 (SEQ ID NO:211), Figure 213 (SEQ ID NO:213), Figure 215 (SEQ ID NO:215), Figure 217 (SEQ ID NO:217), Figure 219 (SEQ ID NO:219), Figure 221 (SEQ ID NO:221), Figure 223 (SEQ ID NO:223), Figure 225 (SEQ ID NO:225), Figure 227 (SEQ ID NO:227), Figure 229 (SEQ ID NO:229), Figure 231 (SEQ ID NO:231), Figure 231 (SEQ ID NO:231), Figure 233 (SEQ ID NO:233), Figure 235 (SEQ ID NO:235), Figure 237 (SEQ ID NO:237), Figure 239 (SEQ ID NO:239), Figure 241 (SEQ ID NO:241), Figure 243 (SEQ ID NO:243), Figure 245 (SEQ ID NO:245), Figure 247 (SEQ ID NO:247), Figure 249 (SEQ ID NO:249), Figure 251 (SEQ ID NO:251), Figure 253 (SEQ ID NO:253), Figure 255 (SEQ ID NO:255), Figure 257 (SEQ ID NO:257), Figure 259 (SEQ ID NO:259), Figure 261 (SEQ ID NO:261), Figure 263 (SEQ ID NO:263), Figure 265 (SEQ ID NO:265), Figure 267 (SEQ ID NO:267), Figure 269 (SEQ ID NO:269), Figure 271 (SEQ ID NO:271), Figure 273 (SEQ ID NO:273), Figure 275 (SEQ ID NO:275), Figure 277 (SEQ ID NO:277), Figure 279 (SEQ ID NO:279), Figure 281 (SEQ ID NO:281), Figure 283 (SEQ ID NO:283), Figure 285 (SEQ ID NO:285), Figure 287 (SEQ ID NO:287), Figure 289 (SEQ ID NO:289), Figure 291 (SEQ ID NO:291), Figure 293 (SEQ ID NO:293), Figure 295 (SEQ ID NO:295), Figure 297 (SEQ ID NO:297), Figure 299 (SEQ ID NO:299), Figure 301 (SEQ ID NO:301), Figure 303(SEQ ID NO:303), Figure 305 (SEQ ID NO:305), Figure 307 (SEQ ID NO:307), Figure 309 (SEQ ID NO:309), Figure 311 (SEQ ID NO:311), Figure 313 (SEQ ID NO:313), Figure 315 (SEQ ID NO:315), Figure 317 (SEQ ID NO:317), Figure 319 (SEQ ID NO:319), Figure 321 (SEQ ID NO:321), Figure 323 (SEQ ID NO:323), Figure 325 (SEQ ID NO:325), Figure 327 (SEQ ID NO:327), Figure 329 (SEQ ID NO:329), Figure 331 (SEQ ID NO:331), Figure 333 (SEQ ID NO:333), Figure 335 (SEQ ID NO:335), Figure 337 (SEQ ID NO:337), Figure 339 (SEQ ID NO:339), Figure 341 (SEQ ID NO:341), Figure 343 (SEQ ID NO:343), Figure 345 (SEQ ID NO:345), Figure 347 (SEQ ID NO:347), Figure 349 (SEQ ID NO:349), Figure 349 (SEQ ID NO:349), Figure 351 (SEQ ID NO:351), Figure 353 (SEQ ID NO:353), Figure 355 (SEQ ID NO:355), Figure 357 (SEQ ID NO:357), Figure 359 (SEQ ID NO:359), Figure 361 (SEQ ID NO:361), Figure 363 (SEQ ID NO:363), Figure 365 (SEQ ID NO:365), Figure 367 (SEQ ID NO:367), Figure 369 (SEQ ID NO:369), Figure 371 (SEQ ID NO:371), Figure 373 (SEQ ID NO:373), Figure 375 (SEQ ID NO:375), Figure 377 (SEQ ID NO:377), Figure 379 (SEQ ID NO:379), Figure 381 (SEQ ID NO:381), Figure 383 (SEQ ID NO:383), Figure 385 (SEQ ID NO:385), Figure 387 (SEQ ID NO:387), Figure 389 (SEQ ID NO:389), Figure 391 (SEQ ID NO:391), Figure 393 (SEQ ID NO:393), Figure 395 (SEQ ID NO:395), Figure 397 (SEQ ID NO:397), Figure 399 (SEQ ID NO:399), Figure 401 (SEQ ID NO:401), Figure 403 (SEQ ID NO:403), Figure 405 (SEQ ID NO:405), Figure 407 (SEQ ID NO:407), Figure 409 (SEQ ID NO:409), Figure 411 (SEQ ID NO:411), Figure 413 (SEQ ID NO:413), Figure 415 (SEQ ID NO:415), Figure 417 (SEQ ID NO:417), Figure 419 (SEQ ID NO:419), Figure 421 (SEQ ID NO:421), Figure 423 (SEQ ID NO:423), Figure 425 (SEQ ID NO:425), Figure 427 (SEQ ID NO:427), Figure 429 (SEQ ID NO:429), Figure 431 (SEQ ID NO:431), Figure 433 (SEQ ID NO:433), Figure 435 (SEQ ID NO:435), Figure 437 (SEQ ID NO:437), Figure 439 (SEQ ID NO:439), Figure 441 (SEQ ID NO:441), Figure 443 (SEQ ID NO:443), Figure 445 (SEQ ID NO:445), Figure 447 (SEQ ID NO:447), Figure 449 (SEQ ID NO:449), Figure 451 (SEQ ID NO:451), Figure 453 (SEQ ID NO:453), Figure 455 (SEQ ID NO:455), Figure 457 (SEQ ID NO:457), Figure 459 (SEQ ID NO:459), Figure 461 (SEQ ID NO:461), Figure 463 (SEQ ID NO:463), Figure 465 (SEQ ID NO:465), Figure 467 (SEQ ID NO:467), Figure 469 (SEQ ID NO:469), Figure 471 (SEQ ID NO:471), Figure 473 (SEQ ID NO:473), Figure 475 (SEQ ID NO:475), Figure 477 (SEQ ID NO:477), Figure 479 (SEQ ID NO:479), Figure 481 (SEQ ID NO:481), Figure 483 (SEQ ID NO:483), Figure 485 (SEQ ID NO:485), Figure 487 (SEQ ID NO:487), Figure 489 (SEQ ID NO:489), Figure 491 (SEQ ID NO:491), Figure 493 (SEQ ID NO:493), Figure 495 (SEQ ID NO:495), Figure 497 (SEQ ID NO:497), Figure 499 (SEQ ID NO:499), Figure 501 (SEQ ID NO:501), Figure 503 (SEQ ID NO:503), Figure 505 (SEQ ID NO:505), Figure 507 (SEQ ID NO:507), Figure 509 (SEQ ID NO:509), Figure 511 (SEQ ID NO:511), Figure 513 (SEQ ID NO:513), Figure 515 (SEQ ID NO:515), Figure 517 (SEQ ID NO:517), Figure 519 (SEQ ID NO:519), Figure 521 (SEQ ID NO:521), Figure 523 (SEQ ID NO:523), Figure 525 (SEQ ID NO:525), Figure 527 (SEQ ID NO:527), Figure 529 (SEQ ID NO:529), Figure 531 (SEQ ID NO:531), Figure 533 (SEQ ID NO:533), Figure 535 (SEQ ID NO:535), Figure 537 (SEQ ID NO:537), Figure 539 (SEQ ID NO:539), Figure 541 (SEQ ID NO:541), Figure 543 (SEQ ID NO:543), Figure 545 (SEQ ID NO:545), Figure 547 (SEQ ID NO:547), Figure 549 (SEQ ID NO:549), Figure 551 (SEQ ID NO:551), Figure 553 (SEQ ID NO:553), Figure 555 (SEQ ID NO:555), Figure 557 (SEQ ID NO:557), Figure 559 (SEQ ID NO:559), Figure 561 (SEQ ID NO:561), Figure 563 (SEQ ID NO:563), Figure 565 (SEQ ID NO:565), Figure 567 (SEQ ID NO:567), Figure 569 (SEQ ID NO:569), Figure 571 (SEQ ID NO:571), Figure 573 (SEQ ID NO:573), Figure 575 (SEQ ID NO:575), Figure 577 (SEQ ID NO:577), Figure 579 (SEQ ID NO:579), Figure 581 (SEQ ID NO:581), Figure 583 (SEQ ID NO:583), Figure 585 (SEQ ID NO:585), Figure 587 (SEQ ID NO:587), Figure 589 (SEQ ID NO:589), Figure 591 (SEQ ID NO:591), Figure 593 (SEQ ID NO:593), Figure 595 (SEQ ID NO:595), Figure 597 (SEQ ID NO:597), Figure 599 (SEQ ID NO:599), Figure 601 (SEQ ID NO:601), Figure 603 (SEQ ID NO:603), Figure 605 (SEQ ID NO:605), Figure 607 (SEQ ID NO:607), Figure 609 (SEQ ID NO:609), Figure 611 (SEQ ID NO:611), Figure 613 (SEQ ID NO:613), Figure 615 (SEQ ID NO:615), Figure 617 (SEQ ID NO:617), Figure 619 (SEQ ID NO:619), Figure 621 (SEQ ID NO:621), Figure 623 (SEQ ID NO:623), Figure 625 (SEQ ID NO:625), Figure 627 (SEQ ID NO:627), Figure 629 (SEQ ID NO:629), Figure 631 (SEQ ID NO:631), Figure 633 (SEQ ID NO:633), Figure 635 (SEQ ID NO:635), Figure 637 (SEQ ID NO:637), Figure 639 (SEQ ID NO:639), Figure 641 (SEQ ID NO:641), Figure 643 (SEQ ID NO:643), Figure 645 (SEQ ID NO:645), Figure 647 (SEQ ID NO:647), Figure 649 (SEQ ID NO:649), Figure 651 (SEQ ID NO:651), Figure 653 (SEQ ID NO:653), Figure 655 (SEQ ID NO:655), Figure 657 (SEQ ID NO:657), Figure 659 (SEQ ID NO:659), Figure 661 (SEQ ID NO:661), Figure 663 (SEQ ID NO:663), Figure 665 (SEQ ID NO:665), Figure 667 (SEQ ID NO:667), Figure 669 (SEQ ID NO:669), Figure 671 (SEQ ID NO:671), Figure 673 (SEQ ID NO:673), Figure 675 (SEQ ID NO:675), Figure 677 (SEQ ID NO:677), Figure 679 (SEQ ID NO:679), Figure 681 (SEQ ID NO:681), Figure 683 (SEQ ID NO:683), Figure 685 (SEQ ID NO:685), Figure 687 (SEQ ID NO:687), Figure 689 (SEQ ID NO:689), Figure 691 (SEQ ID NO:691), Figure 693 (SEQ ID NO:693), Figure 695 (SEQ ID NO:695), Figure 697 (SEQ ID NO:697), Figure 699 (SEQ ID NO:699), Figure 701 (SEQ ID NO:701), Figure 703 (SEQ ID NO:703), Figure 705 (SEQ ID NO:705), Figure 707 (SEQ ID NO:707), Figure 709 (SEQ ID NO:709), Figure 711 (SEQ ID NO:711), Figure 713 (SEQ ID NO:713), Figure 715 (SEQ ID NO:715), Figure 717 (SEQ ID NO:717), Figure 719 (SEQ ID NO:719), Figure 721 (SEQ ID NO:721), Figure 723 (SEQ ID NO:723), Figure 725 (SEQ ID NO:725), Figure 727 (SEQ ID NO:727), Figure 729 (SEQ ID NO:729), Figure 731 (SEQ ID NO:731), Figure 733 (SEQ ID NO:733), Figure 735 (SEQ ID NO:735), Figure 737 (SEQ ID NO:737), Figure 739 (SEQ ID NO:739), and Figure 741 (SEQ ID NO:741).
4. A vector comprising the nucleic acid of Para. 1.
5. The vector of Para. 4 operably linked to control sequences recognized by a host cell transformed with the vector.
6. A host cell comprising the vector of Para. 4.
7. The host cell of Para. 6, wherein said cell is a CHO cell, an *E*. *coli* cell or a yeast cell.
8. A process for producing a PRO polypeptide comprising culturing the host cell of Para. 7 under conditions suitable for expression of said PRO polypeptide and recovering said PRO polypeptide from the cell culture.
9. An isolated polypeptide having at least 80% amino acid sequence identity to:
   (a) an amino acid sequence of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 8 (SEQ ID NO:14), Figure 8 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), Figure 56 (SEQ ID NO:56), Figure 58 (SEQ ID NO:58), Figure 60 (SEQ ID NO:60), Figure 62 (SEQ ID NO:62), Figure 64 (SEQ ID NO:64), Figure 66 (SEQ ID NO:66), Figure 68 (SEQ ID NO:68), Figure 70 (SEQ ID NO:70), Figure 72 (SEQ ID NO:72), Figure 74 (SEQ ID NO:74), Figure 76 (SEQ ID NO:76), Figure 78 (SEQ ID NO:78), Figure 80 (SEQ ID NO:80), Figure 82 (SEQ ID NO:82), Figure 84 (SEQ ID NO:84), Figure 86 (SEQ ID NO:86), Figure 88 (SEQ ID NO:88), Figure 90 (SEQ ID NO:90), Figure 92 (SEQ ID NO:92), Figure 94 (SEQ ID NO:94), Figure 96 (SEQ ID NO:96), Figure 98 (SEQ ID NO:98), Figure 100 (SEQ ID NO:100), Figure 102 (SEQ ID NO:102), Figure 104 (SEQ ID NO:104), Figure 106 (SEQ ID NO:106), Figure 108 (SEQ ID NO:108), Figure 110 (SEQ ID NO:110), Figure 112 (SEQ ID NO:112), Figure 114 (SEQ ID NO:144), Figure 116 (SEQ ID NO:116), Figure 118 (SEQ ID NO:118), Figure 120 (SEQ ID NO:120), Figure 122 (SEQ ID NO:122), Figure 124 (SEQ ID NO:124), Figure 126 (SEQ ID NO:126), Figure 128 (SEQ ID NO:128), Figure 130 (SEQ ID NO:130), Figure 132 (SEQ ID NO:132), Figure 134 (SEQ ID NO:134), Figure 136 (SEQ ID NO:136), Figure 138 (SEQ ID NO:138), Figure 140 (SEQ ID NO:140), Figure 142 (SEQ ID NO:142), Figure 144 (SEQ ID NO:144), Figure 146 (SEQ ID NO:146), Figure 148 (SEQ ID NO:148), Figure 150 (SEQ ID NO:150), Figure 152 (SEQ ID NO:152), Figure 154 (SEQ ID NO:154), Figure 156 (SEQ ID NO:156), Figure 158 (SEQ ID NO:158), Figure 160 (SEQ ID NO:160), Figure 162 (SEQ ID NO:162), Figure 164 (SEQ ID NO:164), Figure 166 (SEQ ID NO:166), Figure 168 (SEQ ID NO:168), Figure 170 (SEQ ID NO:170), Figure 172 (SEQ ID NO:172), Figure 174 (SEQ ID NO:174), Figure 176 (SEQ ID NO:176), Figure 178 (SEQ ID NO:178), Figure 180 (SEQ ID NO:180), Figure 182 (SEQ ID NO:182), Figure 184 (SEQ ID NO:184), Figure 186 (SEQ ID NO:186), Figure 188 (SEQ ID NO:188), Figure 190 (SEQ ID NO:190), Figure 192 (SEQ ID NO:192), Figure 194 (SEQ ID NO:194), Figure 196 (SEQ ID NO:196), Figure 198 (SEQ ID NO:198), Figure 200 (SEQ ID NO:200), Figure 202 (SEQ ID NO:202), Figure 204 (SEQ ID NO:204), Figure 206 (SEQ ID NO:206), Figure 208 (SEQ ID NO:208), Figure 210 (SEQ ID NO:210), Figure 212 (SEQ ID NO:212), Figure 214 (SEQ ID NO:214), Figure 216 (SEQ ID NO:216), Figure 218 (SEQ ID NO:218), Figure 220 (SEQ ID NO:220), Figure 222 (SEQ ID NO:222), Figure 224 (SEQ ID NO:224), Figure 226 (SEQ ID NO:226), Figure 228 (SEQ ID NO:228), Figure 230 (SEQ ID NO:230), Figure 232 (SEQ ID NO:232), Figure 234 (SEQ ID NO:234), Figure 236 (SEQ ID NO:236), Figure 238 (SEQ ID NO:238), Figure 240 (SEQ ID NO:240), Figure 242 (SEQ ID NO:242), Figure 244 (SEQ ID NO:244), Figure 246 (SEQ ID NO:246), Figure 248 (SEQ ID NO:248), Figure 250 (SEQ ID NO:250), Figure 252 (SEQ ID NO:252), Figure 254 (SEQ ID NO:254), Figure 256 (SEQ ID NO:256), Figure 258 (SEQ ID NO:258), Figure 260 (SEQ ID NO:260), Figure 262 (SEQ ID NO:262), Figure 264 (SEQ ID NO:264), Figure 266 (SEQ ID NO:266), Figure 268 (SEQ ID NO:268), Figure 270 (SEQ ID NO:270), Figure 272 (SEQ ID NO:272), Figure 274 (SEQ ID NO:274), Figure 276 (SEQ ID NO:276), Figure 278 (SEQ ID NO:278), Figure 280 (SEQ ID NO:280), Figure 282 (SEQ ID NO:282), Figure 284 (SEQ ID NO:284), Figure 286 (SEQ ID NO:286), Figure 288 (SEQ ID NO:288), Figure 290 (SEQ ID NO:290), Figure 292 (SEQ ID NO:292), Figure 294 (SEQ ID NO:294), Figure 296 (SEQ ID NO:296), Figure 298 (SEQ ID NO:298), Figure 300 (SEQ ID NO:300), Figure 302 (SEQ ID NO:302), Figure 304 (SEQ ID NO:304), Figure 306 (SEQ ID NO:306), Figure 308 (SEQ ID NO:308), Figure 310 (SEQ ID NO:310), Figure 312 (SEQ ID NO:312), Figure 314 (SEQ ID NO:314), Figure 316 (SEQ ID NO:316), Figure 318 (SEQ ID NO:318), Figure 320 (SEQ ID NO:320), Figure 322 (SEQ ID NO:322), Figure 324 (SEQ ID NO:324), Figure 326 (SEQ ID NO:326), Figure 328 (SEQ ID NO:328), Figure 330 (SEQ ID NO:330), Figure 332 (SEQ ID NO:332), Figure 334 (SEQ ID NO:334), Figure 336 (SEQ ID NO:336), Figure 338 (SEQ ID NO:338), Figure 340 (SEQ ID NO:340), Figure 342 (SEQ ID NO:342), Figure 344 (SEQ ID NO:344), Figure 346 (SEQ ID NO:346), Figure 348 (SEQ ID NO:348), Figure 350 (SEQ ID NO:350), Figure 352 (SEQ ID NO:352), Figure 354 (SEQ ID NO:354), Figure 356 (SEQ ID NO:356), Figure 358 (SEQ ID NO:358), Figure 360 (SEQ ID NO:360), Figure 362 (SEQ ID NO:362), Figure 364 (SEQ ID NO:364), Figure 366 (SEQ ID NO:366), Figure 368 (SEQ ID NO:368), Figure 370 (SEQ ID NO:370), Figure 372 (SEQ ID NO:372), Figure 374 (SEQ ID NO:374), Figure 376 (SEQ ID NO:376), Figure 378 (SEQ ID NO:378), Figure 380 (SEQ ID NO:380), Figure 382 (SEQ ID NO:382), Figure 384 (SEQ ID NO:384), Figure 386 (SEQ ID NO:386), Figure 390 (SEQ ID NO:390), Figure 392 (SEQ ID NO:392), Figure 394 (SEQ ID NO:394), Figure 396 (SEQ ID NO:396), Figure 398 (SEQ ID NO:398), Figure 400 (SEQ ID NO:400), Figure 402 (SEQ ID NO:402), Figure 404 (SEQ ID NO:404), Figure 406 (SEQ ID NO:406), Figure 408 (SEQ ID NO:408), Figure 410 (SEQ ID NO:410), Figure 412 (SEQ ID NO:412), Figure 414 (SEQ ID NO:414), Figure 416 (SEQ ID NO:416), Figure 418 (SEQ ID NO:418), Figure 420 (SEQ ID NO:420), Figure 422 (SEQ ID NO:422), Figure 424 (SEQ ID NO:424), Figure 426 (SEQ ID NO:426), Figure 428 (SEQ ID NO:428), Figure 430 (SEQ ID NO:430), Figure 432 (SEQ ID NO:432), Figure 434 (SEQ ID NO:434), Figure 436 (SEQ ID NO:436), Figure 438 (SEQ ID NO:438), Figure 440 (SEQ ID NO:440), Figure 442 (SEQ ID NO:442), Figure 444 (SEQ ID NO:444), Figure 446 (SEQ ID NO:446), Figure 448 (SEQ ID NO:448), Figure 450 (SEQ ID NO:450), Figure 52 (SEQ ID NO:452), Figure 454 (SEQ ID NO:454), Figure 456 (SEQ ID NO:456), Figure 458 (SEQ ID NO:458), Figure 460 (SEQ ID NO:460), Figure 462 (SEQ ID NO:462), Figure 464 (SEQ ID NO:464), Figure 466 (SEQ ID NO:466), Figure 468 (SEQ ID NO:468), Figure 470 (SEQ ID NO:470), Figure 472 (SEQ ID NO:472), Figure 474 (SEQ ID NO:474), Figure 476 (SEQ ID NO:476), Figure 478 (SEQ ID NO:478), Figure 480 (SEQ ID NO:480), Figure 482 (SEQ ID NO:482), Figure 484 (SEQ ID NO:484), Figure 486 (SEQ ID NO:486), Figure 488 (SEQ ID NO:488), Figure 490 (SEQ ID NO:490), Figure 492 (SEQ ID NO:492), Figure 494 (SEQ ID NO:494), Figure 496 (SEQ ID NO:496), Figure 498 (SEQ ID NO:498), Figure 500 (SEQ ID NO:500), Figure 502 (SEQ ID NO:502), Figure 504 (SEQ ID NO:504), Figure 506 (SEQ ID NO:506), Figure 508 (SEQ ID NO:508), Figure 510 (SEQ ID NO:510), Figure 512 (SEQ ID NO:512), Figure 514 (SEQ ID NO:514), Figure 516 (SEQ ID NO:516), Figure 518 (SEQ ID NO:518), Figure 520 (SEQ ID NO:520), Figure 522 (SEQ ID NO:522), Figure 524 (SEQ ID NO:524), Figure 526 (SEQ ID NO:526), Figure 528 (SEQ ID NO:528), Figure 530 (SEQ ID NO:530), Figure 532 (SEQ ID NO:532), Figure 534 (SEQ ID NO:534), Figure 536 (SEQ ID NO:536), Figure 540 (SEQ ID NO:540), Figure 542 (SEQ ID NO:542), Figure 544 (SEQ ID NO:544), Figure 546 (SEQ ID NO:546), Figure 548 (SEQ ID NO:548), Figure 550 (SEQ ID NO:550), Figure 552 (SEQ ID NO:552), Figure 554 (SEQ ID NO:554), Figure 556 (SEQ ID NO:556), Figure 558 (SEQ ID NO:558), Figure 560 (SEQ ID NO:560), Figure 562 (SEQ ID NO:562), Figure 564 (SEQ ID NO:564), Figure 566 (SEQ ID NO:566), Figure 568 (SEQ ID NO:568), Figure 570 (SEQ ID NO:570), Figure 572 (SEQ ID NO:572), Figure 574 (SEQ ID NO:574), Figure 576 (SEQ ID NO:576), Figure 578 (SEQ ID NO:578), Figure 580(SEQ ID NO:580), Figure 582 (SEQ ID NO:582), Figure 584 (SEQ ID NO:584), Figure 586 (SEQ ID NO:586), Figure 588 (SEQ ID NO:588), Figure 590 (SEQ ID NO:590), Figure 592 (SEQ ID NO:592), Figure 594 (SEQ ID NO:594), Figure 596 (SEQ ID NO:596), Figure 598 (SEQ ID NO:598), Figure 600 (SEQ ID NO:600), Figure 602 (SEQ ID NO:602), Figure 604 (SEQ ID NO:604), Figure 606 (SEQ ID NO:606), Figure 608 (SEQ ID NO:608), Figure 610 (SEQ ID NO:610), Figure 612 (SEQ ID NO:612), Figure 614 (SEQ ID NO:614), Figure 616 (SEQ ID NO:616), Figure 618 (SEQ ID NO:618), Figure 620 (SEQ ID NO:620), Figure 622 (SEQ ID NO:622), Figure 624 (SEQ ID NO:624), Figure 626 (SEQ ID NO:626), Figure 628 (SEQ ID NO:628), Figure 630 (SEQ ID NO:630), Figure 632 (SEQ ID NO:632), Figure 634 (SEQ ID NO:634), Figure 636 (SEQ ID NO:636), Figure 638 (SEQ ID NO:638), Figure 640 (SEQ ID NO:640), Figure 642 (SEQ ID NO:642), Figure 644 (SEQ ID NO:644), Figure 646 (SEQ ID NO:646), Figure 648 (SEQ ID NO:648), Figure 650 (SEQ ID NO:650), Figure 652 (SEQ ID NO:652), Figure 654 (SEQ ID NO:654), Figure 656 (SEQ ID NO:656), Figure 658 (SEQ ID NO:658), Figure 660 (SEQ ID NO:660), Figure 662 (SEQ ID NO:662), Figure 664 (SEQ ID NO:664), Figure 666 (SEQ ID NO:666), Figure 668 (SEQ ID NO:668), Figure 670 (SEQ ID NO:670), Figure 672 (SEQ ID NO:672), Figure 674 (SEQ ID NO:674), Figure 676 (SEQ ID NO:676), Figure 678 (SEQ ID NO:678), Figure 680 (SEQ ID NO:680), Figure 682 (SEQ ID NO:682), Figure 684 (SEQ ID NO:684), Figure 686 (SEQ ID NO:686), Figure 688 (SEQ ID NO:688), Figure 690 (SEQ ID NO:690), Figure 692 (SEQ ID NO:692), Figure 694 (SEQ ID NO:694), Figure 696 (SEQ ID NO:696), Figure 698 (SEQ ID NO:698), Figure 700 (SEQ ID NO:700), Figure 702 (SEQ ID NO:702), Figure 704 (SEQ ID NO:704), Figure 706 (SEQ ID NO:706), Figure 708 (SEQ ID NO:708), Figure 710 (SEQ ID NO:710), Figure 712 (SEQ ID NO:712), Figure 714 (SEQ ID NO:714), Figure 716 (SEQ ID NO:716), Figure 718 (SEQ ID NO:718), Figure 720 (SEQ ID NO:720), Figure 722 (SEQ ID NO:722), Figure 724 (SEQ ID NO:724), Figure 726 (SEQ ID NO:726), Figure 728 (SEQ ID NO:728), Figure 730 (SEQ ID NO:730), Figure 732 (SEQ ID NO:732), Figure 734 (SEQ ID NO:734), Figure 736 (SEQ ID NO:736), Figure 740 (SEQ ID NO:740), or Figure 742 (SEQ ID NO:742).
10. A chimeric molecule comprising a polypeptide according to Para. 9 fused to a heterologous amino acid sequence.
11. The chimeric molecule of Para. 10, wherein said heterologous amino acid sequence is an epitope tag sequence or an Fc region of an immunoglobulin.
12. An antibody which specifically binds to a polypeptide according to Para. 9.
13. The antibody of Para. 12, wherein said antibody is a monoclonal antibody, a humanized antibody or a single-chain antibody.
14. A composition of matter comprising (a) a polypeptide of Para. 9, (b) an agonist of said polypeptide, (c) an antagonist of said polypeptide, or (d) an antibody that binds to said polypeptide, in combination with a carrier.
15. The composition of matter of Para. 14, wherein said carrier is a pharmaceutically acceptable carrier.
16. The composition of matter of Para. 14 comprising a therapeutically effective amount of (a), (b), (c) or (d).
17. An article of manufacture, comprising:
   a container;
   a label on said container; and
   a composition of matter comprising (a) a polypeptide of Para. 9, (b) an agonist of said polypeptide, (c) an antagonist of said polypeptide, or (d) an antibody that binds to said polypeptide, contained within said container, wherein label on said container indicates that said composition of matter can be used for treating an immune related disease.
18. A method of treating an immune related disorder in a mammal in need thereof comprising administering to said mammal a therapeutically effective amount of (a) a polypeptide of Para. 9, (b) an agonist of said polypeptide, (c) an antagonist of said polypeptide, or (d) an antibody that binds to said polypeptide.
19. The method of Para. 18, wherein the immune related disorder is systemic lupus erythematosis, rheumatoid arthritis, osteoarthritis, juvenile chronic arthritis, a spondyloarthropathy, systemic sclerosis, an idiopathic inflammatory myopathy, Sjögren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barré syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.
20. A method for determining the presence of a PRO69457, PRO69458, PRO52268, PRO69459, PRO62927, PRO59136, PRO37121, PRO69460, PRO60475, PRO34451, PRO38070, PRO23756, PRO10404, PRO69461, PRO70006, PRO69462, PRO2081, PRO70007, PRO69463, PRO62908, PRO69464, PRO52804, PRO60438, PRO69465, PRO37421, PRO37596, PRO36124, PRO69466, PRO60499, PRO69467, PRO61824, PRO69468, PRO21341, PRO38213, PRO69469, PRO37172, PRO35991, PRO36905, PRO69470, PRO36451, PRO69471, PRO37492, PRO70008, PRO69472, PRO69473, PRO36996, PRO22613, PRO69475, PRO61755, PRO70009, PRO69476, PRO4881, PRO12876, PRO70010, PRO37534, PRO21928, PRO69478, PRO69479, PRO69480, PRO69481, PRO69482, PRO69483, PRO38642, PRO69484, PRO66269, PRO1723, PRO22297, PRO61349, PRO69485, PRO69486, PRO69487, PRO36963, PRO23814, PRO57980, PRO20128, PRO4551, PRO69488, PRO39268, PRO69489, PRO69490, PRO69491, PRO69492, PRO37713, PRO58993, PRO69493, PRO69494, PRO69495, PRO70011, PRO62861, PRO36640, PRO36766, PRO69497, PRO69498, PRO69499, PRO69500, PRO69501, PRO70012, PRO69503, PRO69474, PRO69505, PRO69506, PRO69507, PRO51301, PRO69508, PRO69509, PRO69510, PRO69511, PRO51309, PRO50578, PRO69512, PRO69513, PRO69514, PRO10607, PRO61705, PRO49214, PRO39648, PRO69515, PRO38497, PRO29371, PRO70013, PRO69516, PRO69517, PRO69518, PRO70014, PRO69520, PRO69521, PRO69522, PRO69523, PRO60513, PRO2512, PRO69524, PRO12569, PRO69525, PRO69526, PRO69527, PRO69528, PRO69529, PRO12166, PRO2154, PRO69530, PRO51916, PRO52174, PRO69531, PRO69532, PRO69533, PRO69534, PRO54728, PRO70015, PRO69536, PRO69537, PRO37498, PRO22175, PRO69538, PRO37015, PRO12187, PRO69539, PRO69880, PRO69541, PRO69542, PRO69543, PRO70016, PRO69545, PRO50197, PRO69546, PRO69547, PRO69548, PRO69549, PRO69550, PRO69551, PRO69552, PRO37460, PRO42223, PRO69553, PRO69554, PRO69555, PRO61014, PRO59915, PRO37891, PRO69556, PRO12875, PRO70017, PRO70018, PRO4426, PRO69558, PRO69559, PRO37676, PRO69560, PRO69561, PRO69562, PRO63204, PRO70019, PRO69564, PRO62830, PRO69565, PRO69566, PRO69567, PRO49675, PRO69568, PRO2013, PRO69569, PRO69570, PRO69571, PRO36403, PRO4676, PRO37657, PRO62097, PRO38081, PRO69572, PRO69573, PRO69574, PRO69883, PRO69576, PRO37584, PRO11603, PRO70020, PRO51695, PRO69579, PRO69580, PRO69581, PRO69582, PRO69583, PRO69584, PRO69585, PRO69586, PRO69587, PRO69588, PRO69589, PRO70021, PRO69590, PRO70022, PRO69592, PRO37029, PRO69593, PRO69594, PRO69595, PRO1207, PRO69596, PRO69597, PRO51139, PRO62545, PRO3615, PRO38036, PRO69598, PRO4701, PRO69599, PRO69600, PRO69601, PRO69887, PRO69603, PRO69604, PRO70023, PRO69606, PRO69607, PRO69608, PRO69609, PRO69610, PRO9902, PRO69611, PRO69612, PRO69613, PRO69614, PRO69615, PRO70024, PRO69616, PRO49619, PRO69617, PRO69618, PRO38040, PRO69619, PRO69620, PRO69621, PRO69622, PRO4401, PRO70025, PRO69625, PRO12025, PRO70026, PRO69627, PRO69628, PRO22637, PRO69629, PRO70027, PRO70028, PRO69632, PRO69634, PRO36857, PRO69893, PRO69635, PRO6180, PRO69637, PRO69638, PRO69639, PRO69640, PRO69641, PRO62766, PRO53782, PRO61472, PRO38179, PRO69642, PRO69643, PRO69644, PRO69645, PRO11608, PRO69646, PRO59825, PRO69647, PRO69648, PRO70029, PRO1213, PRO70030, PRO50195, PRO69651, PRO37538, PRO69652, PRO59210, PRO23374, PRO24844, PRO70031, PRO69653, PRO69654, PRO69655, PRO69656, PRO70032, PRO69659, PRO69660, PRO58054, PRO69661, PRO69662, PRO69898, PRO69664, PRO69665, PRO69666, PRO69667, PRO69669, PRO69671, PRO69672, PRO58204, PRO49419, PRO69673, PRO69674, PRO49810, PRO70033, PRO69676, PRO58076, PRO69677, PRO69678, PRO69679, PRO1718, PRO51161, PRO69680, PRO59281, PRO36102, PRO61799, PRO69681, PRO69682, PRO69901, PRO69684, PRO69685, PRO69686, PRO69687, PRO38469, PRO69688, PRO70034, PRO59354, PRO59189, PRO38197, PRO69902, PRO69690, PRO61569, PRO69903, or PRO1970 polypeptide in a sample suspected of containing said polypeptide, said method comprising exposing said sample to an anti-PRO69457, anti-PRO69458, anti-PRO52268, anti-PRO69459, anti-PRO62927, anti-PRO59136, anti-PRO37121, anti-PRO69460, anti-PRO60475, anti-PRO34451, anti-PRO38070, anti-PRO23756, anti-PRO10404, anti-PRO69461, anti-PRO70006, anti-PRO69462, anti-PRO2081, anti-PRO70007, anti-PRO69463, anti-PRO62908, anti-PRO69464, anti-PRO52804, anti-PRO60438, anti-PRO69465, anti-PRO37421, anti-PRO37596, anti-PRO36124, anti-PRO69466, anti-PRO60499, anti-PRO69467, anti-PRO61824, anti-PRO69468, anti-PRO21341, anti-PRO38213, anti-PRO69469, anti-PRO37172, anti-PRO35991, anti-PRO36905, anti-PRO69470, anti-PRO36451, anti-PRO69471, anti-PRO37492, anti-PRO70008, anti-PRO69472, anti-PRO69473, anti-PRO36996, anti-PRO22613, anti-PRO69475, anti-PRO61755, anti-PRO70009, anti-PRO69476, anti-PRO4881, anti-PRO12876, anti-PRO70010, anti-PRO37534, anti-PRO21928, anti-PRO69478, anti-PRO69479, anti-PRO69480, anti-PRO69481, anti-PRO69482, anti-PRO69483, anti-PRO38642, anti-PRO69484, anti-PRO66269, anti-PRO1723, anti-PRO22297, anti-PRO61349, anti-PRO69485, anti-PRO69486, anti-PRO69487, anti-PRO36963, anti-PRO23814, anti-PRO57980, anti-PRO20128, anti-PRO4551, anti-PRO69488, anti-PRO39268, anti-PRO69489, anti-PRO69490, anti-PRO69491, anti-PRO69492, anti-PRO37713, anti-PRO58993, anti-PRO69493, anti-PRO69494, anti-PRO69495, anti-PRO70011, anti-PRO62861, anti-PRO36640, anti-PRO36766, anti-PRO69497, anti-PRO69498, anti-PRO69499, anti-PRO69500, anti-PRO69501, anti-PRO70012, anti-PRO69503, anti-PRO69474, anti-PRO69505, anti-PRO69506, anti-PRO69507, anti-PRO51301, anti-PRO69508, anti-PRO69509, anti-PRO69510, anti-PRO69511, anti-PRO51309, anti-PRO50578, anti-PRO69512, anti-PRO69513, anti-PRO69514, anti-PRO10607, anti-PRO61705, anti-PRO49214, anti-PRO39648, anti-PRO69515, anti-PRO38497, anti-PRO29371, anti-PRO70013, anti-PRO69516, anti-PRO69517, anti-PRO69518, anti-PRO70014, anti-PRO69520, anti-PRO69521, anti-PRO69522, anti-PRO69523, anti-PRO60513, anti-PRO2512, anti-PRO69524, anti-PRO12569, anti-PRO69525, anti-PRO69526, anti-PRO69527, anti-PRO69528, anti-PRO69529, anti-PRO12166, anti-PRO2154, anti-PRO69530, anti-PRO51916, anti-PRO52174, anti-PRO69531, anti-PRO69532, anti-PRO69533, anti-PRO69534, anti-PRO54728, anti-PRO70015, anti-PRO69536, anti-PRO69537, anti-PRO37498, anti-PRO22175, anti-PRO69538, anti-PRO37015, anti-PRO12187, anti-PRO69539, anti-PRO69880, anti-PRO69541, anti-PRO69542, anti-PRO69543, anti-PRO70016, anti-PRO69545, anti-PRO50197, anti-PRO69546, anti-PRO69547, anti-PRO69548, anti-PRO69549, anti-PRO69550, anti-PRO69551, anti-PRO69552, anti-PRO37460, anti-PRO42223, anti-PRO69553, anti-PRO69554, anti-PRO69555, anti-PRO61014, anti-PRO59915, anti-PRO37891, anti-PRO69556, anti-PRO12875, anti-PRO70017, anti-PRO70018, anti-PRO4426, anti-PRO69558, anti-PRO69559, anti-PRO37676, anti-PRO69560, anti-PRO69561, anti-PRO69562, anti-PRO63204, anti-PRO70019, anti-PRO69564, anti-PRO62830, anti-PRO69565, anti-PRO69566, anti-PRO69567, anti-PRO49675, anti-PRO69568, anti-PRO2013, anti-PRO69569, anti-PRO69570, anti-PRO69571, anti-PRO36403, anti-PRO4676, anti-PRO37657, anti-PRO62097, anti-PRO38081, anti-PRO69572, anti-PRO69573, anti-PRO69574, anti-PRO69883, anti-PRO69576, anti-PRO37584, anti-PRO11603, anti-PRO70020, anti-PRO51695, anti-PRO69579, anti-PRO69580, anti-PRO69581, anti-PRO69582, anti-PRO69583, anti-PRO69584, anti-PRO69585, anti-PRO69586, anti-PRO69587, anti-PRO69588, anti-PRO69589, anti-PRO70021, anti-PRO69590, anti-PRO70022, anti-PRO69592, anti-PRO37029, anti-PRO69593, anti-PRO69594, anti-PRO69595, anti-PRO1207, anti-PRO69596, anti-PRO69597, anti-PRO51139, anti-PRO62545, anti-PRO3615, anti-PRO38036, anti-PRO69598, anti-PRO4701, anti-PRO69599, anti-PRO69600, anti-PRO69601, anti-PRO69887, anti-PRO69603, anti-PRO69604, anti-PRO70023, anti-PRO69606, anti-PRO69607, anti-PRO69608, anti-PRO69609, anti-PRO69610, anti-PRO9902, anti-PRO69611, anti-PRO69612, anti-PRO69613, anti-PRO69614, anti-PRO69615, anti-PRO70024, anti-PRO69616, anti-PRO49619, anti-PRO69617, anti-PRO69618, anti-PRO38040, anti-PRO69619, anti-PRO69620, anti-PRO69621, anti-PRO69622, anti-PRO4401, anti-PRO70025, anti-PRO69625, anti-PRO12025, anti-PRO70026, anti-PRO69627, anti-PRO69628, anti-PRO22637, anti-PRO69629, anti-PRO70027, anti-PRO70028, anti-PRO69632, anti-PRO69634, anti-PRO36857, anti-PRO69893, anti-PRO69635, anti-PRO6180, anti-PRO69637, anti-PRO69638, anti-PRO69639, anti-PRO69640, anti-PRO69641, anti-PRO62766, anti-PRO53782, anti-PRO61472, anti-PRO38179, anti-PRO69642, anti-PRO69643, anti-PRO69644, anti-PRO69645, anti-PRO11608, anti-PRO69646, anti-PRO59825, anti-PRO69647, anti-PRO69648, anti-PRO70029, anti-PRO1213, anti-PRO70030, anti-PRO50195, anti-PRO69651, anti-PRO37538, anti-PRO69652, anti-PRO59210, anti-PRO23374, anti-PRO24844, anti-PRO70031, anti-PRO69653, anti-PRO69654, anti-PRO69655, anti-PRO69656, anti-PRO70032, anti-PRO69659, anti-PRO69660, anti-PRO58054, anti-PRO69661, anti-PRO69662, anti-PRO69898, anti-PRO69664, anti-PRO69665, anti-PRO69666, anti-PRO69667, anti-PRO69669, anti-PRO69671, anti-PRO69672, anti-PRO58204, anti-PRO49419, anti-PRO69673, anti-PRO69674, anti-PRO49810, anti-PRO70033, anti-PRO69676, anti-PRO58076, anti-PRO69677, anti-PRO69678, anti-PRO69679, anti-PRO1718, anti-PRO51161, anti-PRO69680, anti-PRO59281, anti-PRO36102, anti-PRO61799, anti-PRO69681, anti-PRO69682, anti-PRO69901, anti-PRO69684, anti-PRO69685, anti-PRO69686, anti-PRO69687, anti-PRO38469, anti-PRO69688, anti-PRO70034, anti-PRO59354, anti-PRO59189, anti-PRO38197, anti-PRO69902, anti-PRO69690, anti-PRO61569, anti-PRO69903, or anti-PRO1970 antibody and determining binding of said antibody to a component of said sample.
21. A method of diagnosing an immune related disease in a mammal, said method comprising detecting the level of expression of a gene encoding PRO69457, PRO69458, PRO52268, PRO69459, PRO62927, PRO59136, PRO37121, PRO69460, PRO60475, PRO34451, PRO38070, PRO23756, PRO10404, PRO69461, PRO70006, PRO69462, PRO2081, PRO70007, PRO69463, PRO62908, PRO69464, PRO52804, PRO60438, PRO69465, PRO37421, PRO37596, PRO36124, PRO69466, PRO60499, PRO69467, PRO61824, PRO69468, PRO21341, PRO38213, PRO69469, PRO37172, PRO35991, PRO36905, PRO69470, PRO36451, PRO69471, PRO37492, PRO70008, PRO69472, PRO69473, PRO36996, PRO22613, PRO69475, PRO61755, PRO70009, PRO69476, PRO4881, PRO12876, PRO70010, PRO37534, PRO21928, PRO69478, PRO69479, PRO69480, PRO69481, PRO69482, PRO69483, PRO38642, PRO69484, PRO66269, PRO1723, PRO22297, PRO61349, PRO69485, PRO69486, PRO69487, PRO36963, PRO23814, PRO57980, PRO20128, PRO4551, PRO69488, PRO39268, PRO69489, PRO69490, PRO69491, PRO69492, PRO37713, PRO58993, PRO69493, PRO69494, PRO69495, PRO70011, PRO62861, PRO36640, PRO36766, PRO69497, PRO69498, PRO69499, PRO69500, PRO69501, PRO70012, PRO69503, PRO69474, PRO69505, PRO69506, PRO69507, PRO51301, PRO69508, PRO69509, PRO69510, PRO69511, PRO51309, PRO50578, PRO69512, PRO69513, PRO69514, PRO10607, PRO61705, PRO49214, PRO39648, PRO69515, PRO38497, PRO29371, PRO70013, PRO69516, PRO69517, PRO69518, PRO70014, PRO69520, PRO69521, PRO69522, PRO69523, PRO60513, PRO2512, PRO69524, PRO12569, PRO69525, PRO69526, PRO69527, PRO69528, PRO69529, PRO12166, PRO2154, PRO69530, PRO51916, PRO52174, PRO69531, PRO69532, PRO69533, PRO69534, PRO54728, PRO70015, PRO69536, PRO69537, PRO37498, PRO22175, PRO69538, PRO37015, PRO12187, PRO69539, PRO69880, PRO69541, PRO69542, PRO69543, PRO70016, PRO69545, PRO50197, PRO69546, PRO69547, PRO69548, PRO69549, PRO69550, PRO69551, PRO69552, PRO37460, PRO42223, PRO69553, PRO69554, PRO69555, PRO61014, PRO59915, PRO37891, PRO69556, PRO12875, PRO70017, PRO70018, PRO4426, PRO69558, PRO69559, PRO37676, PRO69560, PRO69561, PRO69562, PRO63204, PRO70019, PRO69564, PRO62830, PRO69565, PRO69566, PRO69567, PRO49675, PRO69568, PRO2013, PRO69569, PRO69570, PRO69571, PRO36403, PRO4676, PRO37657, PRO62097, PRO38081, PRO69572, PRO69573, PRO69574, PRO69883, PRO69576, PRO37584, PRO11603, PRO70020, PRO51695, PRO69579, PRO69580, PRO69581, PRO69582, PRO69583, PRO69584, PRO69585, PRO69586, PRO69587, PRO69588, PRO69589, PRO70021, PRO69590, PRO70022, PRO69592, PRO37029, PRO69593, PRO69594, PRO69595, PRO1207, PRO69596, PRO69597, PRO51139, PRO62545, PRO3615, PRO38036, PRO69598, PRO4701, PRO69599, PRO69600, PRO69601, PRO69887, PRO69603, PRO69604, PRO70023, PRO69606, PRO69607, PRO69608, PRO69609, PRO69610, PRO9902, PRO69611, PRO69612, PRO69613, PRO69614, PRO69615, PRO70024, PRO69616, PRO49619, PRO69617, PRO69618, PRO38040, PRO69619, PRO69620, PRO69621, PRO69622, PRO4401, PRO70025, PRO69625, PRO12025, PRO70026, PRO69627, PRO69628, PRO22637, PRO69629, PRO70027, PRO70028, PRO69632, PRO69634, PRO36857, PRO69893, PRO69635, PRO6180, PRO69637, PRO69638, PRO69639, PRO69640, PRO69641, PRO62766, PRO53782, PRO61472, PRO38179, PRO69642, PRO69643, PRO69644, PRO69645, PRO11608, PRO69646, PRO59825, PRO69647, PRO69648, PRO70029, PRO1213, PRO70030, PRO50195, PRO69651, PRO37538, PRO69652, PRO59210, PRO23374, PRO24844, PRO70031, PRO69653, PRO9654, PRO69655, PRO69656, PRO70032, PRO69659, PRO69660, PRO58054, PRO69661, PRO69662, PRO69898, PRO69664, PRO69665, PRO69666, PRO69667, PRO69669, PRO69671, PRO69672, PRO58204, PRO49419, PRO69673, PRO69674, PRO49810, PRO70033, PRO69676, PRO58076, PRO69677, PRO69678, PRO69679, PRO1718, PRO51161, PRO69680, PRO59281, PRO36102, PRO61799, PRO69681, PRO69682, PRO69901, PRO69684, PRO69685, PRO69686, PRO69687, PRO38469, PRO69688, PRO70034, PRO59354, PRO59189, PRO38197, PRO69902, PRO69690, PRO61569, PRO69903 or PRO1970 polypeptide (a) in a test sample of tissue cells obtained from the mammal, and (b) in a control sample of known normal tissue cells of the same cell type, wherein a higher or lower level of expression of said gene in the test sample as compared to the control sample is indicative of the presence of an immune related disease in the mammal from which the test tissue cells were obtained.
22. A method of diagnosing an immune related disease in a mammal, said method comprising (a) contacting an an anti-PRO69457, anti-PRO69458, anti-PRO52268, anti-PRO69459, anti-PRO62927, anti-PRO59136, anti-PRO37121, anti-PRO69460, anti-PRO60475, anti-PRO34451, anti-PRO38070, anti-PRO23756, anti-PRO10404, anti-PRO69461, anti-PRO70006, anti-PRO69462, anti-PRO2081, anti-PRO70007, anti-PRO69463, anti-PRO62908, anti-PRO69464, anti-PRO52804, anti-PRO60438, anti-PRO69465, anti-PRO37421, anti-PRO37596, anti-PRO36124, anti-PRO69466, anti-PRO60499, anti-PRO69467, anti-PRO61824, anti-PRO69468, anti-PRO21341, anti-PRO38213, anti-PRO69469, anti-PRO37172, anti-PRO35991, anti-PRO36905, anti-PRO69470, anti-PRO36451, anti-PRO69471, anti-PRO37492, anti-PRO70008, anti-PRO69472, anti-PRO69473, anti-PRO36996, anti-PRO22613, anti-PRO69475, anti-PRO61755, anti-PRO70009, anti-PRO69476, anti-PRO4881, anti-PRO12876, anti-PRO70010, anti-PRO37534, anti-PRO21928, anti-PRO69478, anti-PRO69479, anti-PRO69480, anti-PRO69481, anti-PRO69482, anti-PRO69483, anti-PRO38642, anti-PRO69484, anti-PRO66269, anti-PRO1723, anti-PRO22297, anti-PRO61349, anti-PRO69485, anti-PRO69486, anti-PRO69487, anti-PRO36963, anti-PRO23814, anti-PRO57980, anti-PRO20128, anti-PRO4551, anti-PRO69488, anti-PRO39268, anti-PRO69489, anti-PRO69490, anti-PRO69491, anti-PRO69492, anti-PRO37713, anti-PRO58993, anti-PRO69493, anti-PRO69494, anti-PRO69495, anti-PRO70011, anti-PRO62861, anti-PRO36640, anti-PRO36766, anti-PRO69497, anti-PRO69498, anti-PRO69499, anti-PRO69500, anti-PRO69501, anti-PRO70012, anti-PRO69503, anti-PRO69474, anti-PRO69505, anti-PRO69506, anti-PRO69507, anti-PRO51301, anti-PRO69508, anti-PRO69509, anti-PRO69510, anti-PRO69511, anti-PRO51309, anti-PRO50578, anti-PRO69512, anti-PRO69513, anti-PRO69514, anti-PRO10607, anti-PRO61705, anti-PRO49214, anti-PRO39648, anti-PRO69515, anti-PRO38497, anti-PRO29371, anti-PRO70013, anti-PRO69516, anti-PRO69517, anti-PRO69518, anti-PRO70014, anti-PRO69520, anti-PRO69521, anti-PRO69522, anti-PRO69523, anti-PRO60513, anti-PRO2512, anti-PRO69524, anti-PRO12569, anti-PRO69525, anti-PRO69526, anti-PRO69527, anti-PRO69528, anti-PRO69529, anti-PRO12166, anti-PRO2154, anti-PRO69530, anti-PRO51916, anti-PRO52174, anti-PRO69531, anti-PRO69532, anti-PRO69533, anti-PRO69534, anti-PRO54728, anti-PRO70015, anti-PRO69536, anti-PRO69537, anti-PRO37498, anti-PRO22175, anti-PRO69538, anti-PRO37015, anti-PRO12187, anti-PRO69539, anti-PRO69880, anti-PRO69541, anti-PRO69542, anti-PRO69543, anti-PRO70016, anti-PRO69545, anti-PRO50197, anti-PRO69546, anti-PRO69547, anti-PRO69548, anti-PRO69549, anti-PRO69550, anti-PRO69551, anti-PRO69552, anti-PRO37460, anti-PRO42223, anti-PRO69553, anti-PRO69554, anti-PRO69555, anti-PRO61014, anti-PRO59915, anti-PRO37891, anti-PRO69556, anti-PRO12875, anti-PRO70017, anti-PRO70018, anti-PRO4426, anti-PRO69558, anti-PRO69559, anti-PRO37676, anti-PRO69560, anti-PRO69561, anti-PRO69562, anti-PRO63204, anti-PRO70019, anti-PRO69564, anti-PRO62830, anti-PRO69565, anti-PRO69566, anti-PRO69567, anti-PRO49675, anti-PRO69568, anti-PRO2013, anti-PRO69569, anti-PRO69570, anti-PRO69571, anti-PRO36403, anti-PRO4676, anti-PRO37657, anti-PRO62097, anti-PRO38081, anti-PRO69572, anti-PRO69573, anti-PRO69574, anti-PRO69883, anti-PRO69576, anti-PRO37584, anti-PRO11603, anti-PRO70020, anti-PRO51695, anti-PRO69579, anti-PRO69580, anti-PRO69581, anti-PRO69582, anti-PRO69583, anti-PRO69584, anti-PRO69585, anti-PRO69586, anti-PRO69587, anti-PRO69588, anti-PRO69589, anti-PRO70021, anti-PRO69590, anti-PRO70022, anti-PRO69592, anti-PRO37029, anti-PRO69593, anti-PRO69594, anti-PRO69595, anti-PRO1207, anti-PRO69596, anti-PRO69597, anti-PRO51139, anti-PRO62545, anti-PRO3615, anti-PRO38036, anti-PRO69598, anti-PRO4701, anti-PRO69599, anti-PRO69600, anti-PRO69601, anti-PRO69887, anti-PRO69603, anti-PRO69604, anti-PRO70023, anti-PRO69606, anti-PRO69607, anti-PRO69608, anti-PRO69609, anti-PRO69610, anti-PRO9902, anti-PRO69611, anti-PRO69612, anti-PRO69613, anti-PRO69614, anti-PRO69615, anti-PRO70024, anti-PRO69616, anti-PRO49619, anti-PRO69617, anti-PRO69618, anti-PRO38040, anti-PRO69619, anti-PRO69620, anti-PRO69621, anti-PRO69622, anti-PRO4401, anti-PRO70025, anti-PRO69625, anti-PRO12025, anti-PRO70026, anti-PRO69627, anti-PRO69628, anti-PRO22637, anti-PRO69629, anti-PRO70027, anti-PRO70028, anti-PRO69632, anti-PRO69634, anti-PRO36857, anti-PRO69893, anti-PRO69635, anti-PRO6180, anti-PRO69637, anti-PRO69638, anti-PRO69639, anti-PRO69640, anti-PRO69641, anti-PRO62766, anti-PRO53782, anti-PRO61472, anti-PRO38179, anti-PRO69642, anti-PRO69643, anti-PRO69644, anti-PRO69645, anti-PRO11608, anti-PRO69646, anti-PRO59825, anti-PRO69647, anti-PRO69648, anti-PRO70029, anti-PRO1213, anti-PRO70030, anti-PRO50195, anti-PRO69651, anti-PRO37538, anti-PRO69652, anti-PRO59210, anti-PRO23374, anti-PRO24844, anti-PRO70031, anti-PRO69653, anti-PRO69654, anti-PRO69655, anti-PRO69656, anti-PRO70032, anti-PRO69659, anti-PRO69660, anti-PRO58054, anti-PRO69661, anti-PRO69662, anti-PRO69898, anti-PRO69664, anti-PRO69665, anti-PRO69666, anti-PRO69667, anti-PRO69669, anti-PRO69671, anti-PRO69672, anti-PRO58204, anti-PRO49419, anti-PRO69673, anti-PRO69674, anti-PRO49810, anti-PRO70033, anti-PRO69676, anti-PRO58076, anti-PRO69677, anti-PRO69678, anti-PRO69679, anti-PRO1718, anti-PRO51161, anti-PRO69680, anti-PRO59281, anti-PRO36102, anti-PRO61799, anti-PRO69681, anti-PRO69682, anti-PRO69901, anti-PRO69684, anti-PRO69685, anti-PRO69686, anti-PRO69687, anti-PRO38469, anti-PRO69688, anti-PRO70034, anti-PRO59354, anti-PRO59189, anti-PRO38197, anti-PRO69902, anti-PRO69690, anti-PRO61569, anti-PRO69903 or anti-PRO 1970, antibody with a test sample of tissue cells obtained from said mammal and (b) detecting the formation of a complex between the antibody and the polypeptide in the test sample, wherein formation of said complex is indicative of the presence of an immune related disease in the mammal from which the test tissue cells were obtained.
23. A method of identifying a compound that inhibits the activity of PRO69457, PRO69458, PRO52268, PRO69459, PRO62927, PRO59136, PRO37121, PRO69460, PRO60475, PRO34451, PRO38070, PRO23756, PRO10404, PRO69461, PRO70006, PRO69462, PRO2081, PRO70007, PRO69463, PRO62908, PRO69464, PRO52804, PRO60438, PRO69465, PRO37421, PRO37596, PRO36124, PRO69466, PRO60499, PRO69467, PRO61824, PRO69468, PRO21341, PRO38213, PRO69469, PRO37172, PRO35991, PRO36905, PRO69470, PRO36451, PRO69471, PRO37492, PRO70008, PRO69472, PRO69473, PRO36996, PRO22613, PRO69475, PRO61755, PRO70009, PRO69476, PRO4881, PRO12876, PRO70010, PRO37534, PRO21928, PRO69478, PRO69479, PRO69480, PRO69481, PRO69482, PRO69483, PRO38642, PRO69484, PRO66269, PRO1723, PRO22297, PRO61349, PRO69485, PRO69486, PRO69487, PRO36963, PRO23814, PRO57980, PRO20128, PRO4551, PRO69488, PRO39268, PRO69489, PRO69490, PRO69491, PRO69492, PRO37713, PRO58993, PRO69493, PRO69494, PRO69495, PRO70011, PRO62861, PRO36640, PRO36766, PRO69497, PRO69498, PRO69499, PRO69500, PRO69501, PRO70012, PRO69503, PRO69474, PRO69505, PRO69506, PRO69507, PRO51301, PRO69508, PRO69509, PRO69510, PRO69511, PRO51309, PRO50578, PRO69512, PRO69513, PRO69514, PRO10607, PRO61705, PRO49214, PRO39648, PRO69515, PRO38497, PRO29371, PRO70013, PRO69516, PRO69517, PRO69518, PRO70014, PRO69520, PRO69521, PRO69522, PRO69523, PRO60513, PRO2512, PRO69524, PRO12569, PRO69525, PRO69526, PRO69527, PRO69528, PRO69529, PRO12166, PRO2154, PRO69530, PRO51916, PRO52174, PRO69531, PRO69532, PRO69533, PRO69534, PRO54728, PRO70015, PRO69536, PRO69537, PRO37498, PRO22175, PRO69538, PRO37015, PRO12187, PRO69539, PRO69880, PRO69541, PRO69542, PRO69543, PRO70016, PRO69545, PRO50197, PRO69546, PRO69547, PRO69548, PRO69549, PRO69550, PRO69551, PRO69552, PRO37460, PRO42223, PRO69553, PRO69554, PRO69555, PRO61014, PRO59915, PRO37891, PRO69556, PRO12875, PRO70017, PRO70018, PRO4426, PRO69558, PRO69559, PRO37676, PRO69560, PRO69561, PRO69562, PRO63204, PRO70019, PRO69564, PRO62830, PRO69565, PRO69566, PRO69567, PRO49675, PRO69568, PRO2013, PRO69569, PRO69570, PRO69571, PRO36403, PRO4676, PRO37657, PRO62097, PRO38081, PRO69572, PRO69573, PRO69574, PRO69883, PRO69576, PRO37584, PRO11603, PRO70020, PRO51695, PRO69579, PRO69580, PRO69581, PRO69582, PRO69583, PRO69584, PRO69585, PRO69586, PRO69587, PRO69588, PRO69589, PRO70021, PRO69590, PRO70022, PRO69592, PRO37029, PRO69593, PRO69594, PRO69595, PRO1207, PRO69596, PRO69597, PRO51139, PRO62545, PRO3615, PRO38036, PRO69598, PRO4701, PRO69599, PRO69600, PRO69601, PRO69887, PRO69603, PRO69604, PRO70023, PRO69606, PRO69607, PRO69608, PRO69609, PRO69610, PRO9902, PRO69611, PRO69612, PRO69613, PRO69614, PRO69615, PRO70024, PRO69616, PRO49619, PRO69617, PRO69618, PRO38040, PRO69619, PRO69620, PRO69621, PRO69622, PRO4401, PRO70025, PRO69625, PRO12025, PRO70026, PRO69627, PRO69628, PRO22637, PRO69629, PRO70027, PRO70028, PRO69632, PRO69634, PRO6857, PRO69893, PRO69635, PRO6180, PRO69637, PRO69638, PRO69639, PRO69640, PRO69641, PRO62766, PRO53782, PRO61472, PRO38179, PRO69642, PRO69643, PRO69644, PRO69645, PRO11608, PRO69646, PRO59825, PRO69647, PRO69648, PRO70029, PRO1213, PRO70030, PRO50195, PRO69651, PRO37538, PRO69652, PRO59210, PRO23374, PRO24844, PRO70031, PRO69653, PRO69654, PRO69655, PRO69656, PRO70032, PRO69659, PRO69660, PRO58054, PRO69661, PRO69662, PRO69898, PRO69664, PRO69665, PRO69666, PRO69667, PRO69669, PRO69671, PRO69672, PRO58204, PRO49419, PRO69673, PRO69674, PRO49810, PRO70033, PRO69676, PRO58076, PRO69677, PRO69678, PRO69679, PRO1718, PRO51161, PRO69680, PRO59281, PRO36102, PRO61799, PRO69681, PRO69682, PRO69901, PRO69684, PRO69685, PRO69686, PRO69687, PRO38469, PRO69688, PRO70034, PRO59354, PRO59189, PRO38197, PRO69902, PRO69690, PRO61569, PRO69903 or PRO1970 polypeptide, said method comprising contacting cells which normally respond to said polypeptide with (a) said polypeptide and (b) a candidate compound, and determining the lack responsiveness by said cell to (a).
24. A method of identifying a compound that inhibits the expression of a gene encoding a PRO69457, PRO69458, PRO52268, PRO69459, PRO62927, PRO59136, PRO37121, PRO69460, PRO60475, PRO34451, PRO38070, PRO23756, PRO10404, PRO69461, PRO70006, PRO69462, PRO2081, PRO70007, PRO69463, PRO62908, PRO69464, PRO52804, PRO60438, PRO69465, PRO37421, PRO37596, PRO36124, PRO69466, PRO60499, PRO69467, PRO61824, PRO69468, PRO21341, PRO38213, PRO69469, PRO37172, PRO35991, PRO36905, PRO69470, PRO36451, PRO69471, PRO37492, PRO70008, PRO69472, PRO69473, PRO36996, PRO22613, PRO69475, PRO61755, PRO70009, PRO69476, PRO4881, PRO12876, PRO70010, PRO37534, PRO21928, PRO69478, PRO69479, PRO69480, PRO69481, PRO69482, PRO69483, PRO38642, PRO69484, PRO66269, PRO1723, PRO22297, PRO61349, PRO69485, PRO69486, PRO69487, PRO36963, PRO23814, PRO57980, PRO20128, PRO4551, PRO69488, PRO39268, PRO69489, PRO69490, PRO69491, PRO69492, PRO37713, PRO58993, PRO69493, PRO69494, PRO69495, PRO70011, PRO62861, PRO36640, PRO36766, PRO69497, PRO69498, PRO69499, PRO69500, PRO69501, PRO70012, PRO69503, PRO69474, PRO69505, PRO69506, PRO69507, PRO51301, PRO69508, PRO69509, PRO69510, PRO69511, PRO51309, PRO50578, PRO69512, PRO69513, PRO69514, PRO10607, PRO61705, PRO49214, PRO39648, PRO69515, PRO38497, PRO29371, PRO70013, PRO69516, PRO69517, PRO69518, PRO70014, PRO69520, PRO69521, PRO69522, PRO69523, PRO60513, PRO2512, PRO69524, PRO12569, PRO69525, PRO69526, PRO69527, PRO69528, PRO69529, PRO12166, PRO2154, PRO69530, PRO51916, PRO52174, PRO69531, PRO69532, PRO69533, PRO69534, PRO54728, PRO70015, PRO69536, PRO69537, PRO37498, PRO22175, PRO69538, PRO37015, PRO12187, PRO69539, PRO69880, PRO69541, PRO69542, PRO69543, PRO70016, PRO69545, PRO50197, PRO69546, PRO69547, PRO69548, PRO69549, PRO69550, PRO69551, PRO69552, PRO37460, PRO42223, PRO69553, PRO69554, PRO69555, PRO61014, PRO59915, PRO37891, PRO9556, PRO12875, PRO70017, PRO70018, PRO4426, PRO69558, PRO69559, PRO37676, PRO69560, PRO69561, PRO69562, PRO63204, PRO70019, PRO69564, PRO62830, PRO69565, PRO69566, PRO69567, PRO49675, PRO69568, PRO2013, PRO69569, PRO69570, PRO69571, PRO36403, PRO4676, PRO37657, PRO62097, PRO38081, PRO69572, PRO69573, PRO69574, PRO69883, PRO69576, PRO37584, PRO11603, PRO70020, PRO51695, PRO69579, PRO69580, PRO69581, PRO69582, PRO69583, PRO69584, PRO69585, PRO69586, PRO69587, PRO69588, PRO69589, PRO70021, PRO69590, PRO70022, PRO69592, PRO37029, PRO69593, PRO69594, PRO69595, PRO1207, PRO69596, PRO69597, PRO51139, PRO62545, PRO3615, PRO38036, PRO69598, PRO4701, PRO69599, PRO69600, PRO69601, PRO69887, PRO69603, PRO69604, PRO70023, PRO69606, PRO69607, PRO69608, PRO69609, PRO69610, PRO9902, PRO69611, PRO69612, PRO69613, PRO69614, PRO69615, PRO70024, PRO69616, PRO49619, PRO69617, PRO69618, PRO38040, PRO69619, PRO69620, PRO69621, PRO69622, PRO4401, PRO70025, PRO69625, PRO12025, PRO70026, PRO69627, PRO69628, PRO22637, PRO69629, PRO70027, PRO70028, PRO69632, PRO69634, PRO36857, PRO69893, PRO69635, PRO6180, PRO69637, PRO69638, PRO69639, PRO69640, PRO69641, PRO62766, PRO53782, PRO61472, PRO38179, PRO69642, PRO69643, PRO69644, PRO69645, PRO11608, PRO69646, PRO59825, PRO69647, PRO69648, PRO70029, PRO1213, PRO70030, PRO50195, PRO69651, PRO37538, PRO69652, PRO59210, PRO23374, PRO24844, PRO70031, PRO69653, PRO69654, PRO69655, PRO69656, PRO70032, PRO69659, PRO69660, PRO58054, PRO69661, PRO69662, PRO69898, PRO69664, PRO69665, PRO69666, PRO69667, PRO69669, PRO69671, PRO69672, PRO58204, PRO49419, PRO69673, PRO69674, PRO49810, PRO70033, PRO69676, PRO58076, PRO69677, PRO69678, PRO69679, PRO1718, PRO51161, PRO69680, PRO59281, PRO36102, PRO61799, PRO69681, PRO69682, PRO69901, PRO69684, PRO69685, PRO69686, PRO69687, PRO38469, PRO69688, PRO70034, PRO59354, PRO59189, PRO38197, PRO69902, PRO69690, PRO61569, PRO69903 or PRO1970 polypeptide, said method comprising contacting cells which normally express said polypeptide with a candidate compound, and determining the lack of expression said gene.
25. The method of Para. 24, wherein said candidate compound is an antisense nucleic acid.
26. A method of identifying a compound that mimics the activity of a PRO69457, PRO69458, PRO52268, PRO69459, PRO62927, PRO59136, PRO37121, PRO69460, PRO60475, PRO34451, PRO38070, PRO23756, PRO10404, PRO69461, PRO70006, PRO69462, PRO2081, PRO70007, PRO69463, PRO62908, PRO69464, PRO52804, PRO60438, PRO69465, PRO37421, PRO37596, PRO36124, PRO69466, PRO60499, PRO69467, PRO61824, PRO69468, PRO21341, PRO38213, PRO69469, PRO37172, PRO35991, PRO36905, PRO69470, PRO36451, PRO69471, PRO37492, PRO70008, PRO69472, PRO69473, PRO36996, PRO22613, PRO69475, PRO61755, PRO70009, PRO69476, PRO4881, PRO12876, PRO70010, PRO37534, PRO21928, PRO69478, PRO69479, PRO69480, PRO69481, PRO69482, PRO69483, PRO38642, PRO69484, PRO66269, PRO1723, PRO22297, PRO61349, PRO69485, PRO69486, PRO69487, PRO36963, PRO23814, PRO57980, PRO20128, PRO4551, PRO69488, PRO39268, PRO69489, PRO69490, PRO69491, PRO69492, PRO37713, PRO58993, PRO69493, PRO69494, PRO69495, PRO70011, PRO62861, PRO36640, PRO36766, PRO69497, PRO69498, PRO69499, PRO69500, PRO69501, PRO70012, PRO69503, PRO69474, PRO69505, PRO69506, PRO69507, PRO51301, PRO69508, PRO69509, PRO69510, PRO69511, PRO51309, PRO50578, PRO69512, PRO69513, PRO69514, PRO10607, PRO61705, PRO49214, PRO39648, PRO69515, PRO38497, PRO29371, PRO70013, PRO69516, PRO69517, PRO69518, PRO70014, PRO69520, PRO69521, PRO69522, PRO69523, PRO60513, PRO2512, PRO69524, PRO12569, PRO69525, PRO69526, PRO69527, PRO69528, PRO69529, PRO12166, PRO2154, PRO69530, PRO51916, PRO52174, PRO69531, PRO69532, PRO69533, PRO69534, PRO54728, PRO70015, PRO69536, PRO69537, PRO37498, PRO22175, PRO69538, PRO37015, PRO12187, PRO69539, PRO69880, PRO69541, PRO69542, PRO69543, PRO70016, PRO69545, PRO50197, PRO69546, PRO69547, PRO69548, PRO69549, PRO69550, PRO69551, PRO69552, PRO37460, PRO42223, PRO69553, PRO69554, PRO69555, PRO61014, PRO59915, PRO37891, PRO69556, PRO12875, PRO70017, PRO70018, PRO4426, PRO69558, PRO69559, PRO37676, PRO69560, PRO69561, PRO69562, PRO63204, PRO70019, PRv69564, PRO62830, PRO69565, PRO69566, PRO69567, PRO49675, PRO69568, PRO2013, PRO69569, PRO69570, PRO69571, PRO36403, PRO4676, PRO37657, PRO62097, PRO38081, PRO69572, PRO69573, PRO69574, PRO69883, PRO69576, PRO37584, PRO11603, PRO70020, PRO51695, PRO69579, PRO69580, PRO69581, PRO69582, PRO69583, PRO69584, PRO69585, PRO69586, PRO69587, PRO69588, PRO69589, PRO70021, PRO69590, PRO70022, PRO69592, PRO37029, PRO69593, PRO69594, PRO69595, PRO1207, PRO69596, PRO69597, PRO51139, PRO62545, PRO3615, PRO38036, PRO69598, PRO4701, PRO69599, PRO69600, PRO69601, PRO69887, PRO69603, PRO69604, PRO70023, PRO69606, PRO69607, PRO69608, PRO69609, PRO69610, PRO9902, PRO69611, PRO69612, PRO69613, PRO69614, PRO69615, PRO70024, PRO69616, PRO49619, PRO69617, PRO69618, PRO38040, PRO69619, PRO69620, PRO69621, PRO69622, PRO4401, PRO70025, PRO69625, PRO12025, PRO70026, PRO69627, PRO69628, PRO22637, PRO69629, PRO70027, PRO70028, PRO69632, PRO69634, PRO36857, PRO69893, PRO69635, PRO6180, PRO69637, PRO69638, PRO69639, PRO69640, PRO69641, PRO62766, PRO53782, PRO61472, PRO38179, PRO69642, PRO69643, PRO69644, PRO69645, PRO11608, PRO69646, PRO59825, PRO69647, PRO69648, PRO70029, PRO1213, PRO70030, PRO50195, PRO69651, PRO37538, PRO69652, PRO59210, PRO23374, PRO24844, PRO70031, PRO69653, PRO69654, PRO69655, PRO69656, PRO70032, PRO69659, PRO69660, PRO58054, PRO69661, PRO69662, PRO69898, PRO69664, PRO69665, PRO69666, PRO69667, PRO69669, PRO69671, PRO69672, PRO58204, PRO49419, PRO69673, PRO69674, PRO49810, PRO70033, PRO69676, PRO58076, PRO69677, PRO69678, PRO69679, PRO1718, PRO51161, PRO69680, PRO59281, PRO36102, PRO61799, PRO69681, PRO69682, PRO69901, PRO69684, PRO69685, PRO69686, PRO69687, PRO38469, PRO69688, PRO70034, PRO59354, PRO59189, PRO38197, PRO69902, PRO69690, PRO61569, PRO69903 or PRO1970 polypeptide, said method comprising contacting cells which normally respond to said polypeptide with a candidate compound, and determining the responsiveness by said cell to said candidate compound.
27. A method of stimulating the immune response in a mammal, said method comprising administering to said mammal an effective amount of a PRO69457, PRO69458, PRO52268, PRO69459, PRO62927, PRO59136, PRO37121, PRO69460, PRO60475, PRO34451, PRO38070, PRO23756, PRO10404, PRO69461, PRO70006, PRO69462, PRO2081, PRO70007, PRO69463, PRO62908, PRO69464, PRO52804, PRO60438, PRO69465, PRO37421, PRO37596, PRO36124, PRO69466, PRO60499, PRO69467, PRO61824, PRO69468, PRO21341, PRO38213, PRO69469, PRO37172, PRO35991, PRO36905, PRO69470, PRO36451, PRO69471, PRO37492, PRO70008, PRO69472, PRO69473, PRO36996, PRO22613, PRO69475, PRO61755, PRO70009, PRO69476, PRO4881, PRO12876, PRO70010, PRO37534, PRO1928, PRO69478, PRO69479, PRO69480, PRO9481, PRO69482, PRO69483, PRO38642, PRO69484, PRO66269, PRO1723, PRO22297, PRO61349, PRO69485, PRO69486, PRO69487, PRO36963, PRO3814, PRO57980, PRO0128, PRO551, PRO69488, PRO39268, PRO69489, PRO69490, PRO9491, PRO69492, PRO37713, PRO58993, PRO69493, PRO69494, PRO69495, PRO70011, PRO2861, PRO36640, PRO36766, PRO69497, PRO69498, PRO69499, PRO69500, PRO9501, PRO70012, PRO69503, PRO69474, PRO69505, PRO69506, PRO69507, PRO51301, PRO69508, PRO69509, PRO69510, PRO69511, PRO51309, PRO50578, PRO69512, PRO69513, PRO69514, PRO10607, PRO61705, PRO49214, PRO39648, PRO69515, PRO38497, PRO29371, PRO70013, PRO69516, PRO69517, PRO69518, PRO70014, PRO69520, PRO69521, PRO69522, PRO69523, PRO60513, PRO2512, PRO69524, PRO12569, PRO69525, PRO69526, PRO69527, PRO69528, PRO69529, PRO12166, PRO2154, PRO69530, PRO51916, PRO52174, PRO69531, PRO69532, PRO69533, PRO69534, PRO54728, PRO70015, PRO69536, PRO69537, PRO37498, PRO22175, PRO69538, PRO37015, PRO12187, PRO69539, PRO69880, PRO69541, PRO69542, PRO69543, PRO70016, PRO69545, PRO50197, PRO69546, PRO69547, PRO69548, PRO69549, PRO69550, PRO69551, PRO69552, PRO37460, PRO42223, PRO69553, PRO69554, PRO69555, PRO61014, PRO59915, PRO37891, PRO69556, PRO12875, PRO70017, PRO70018, PRO4426, PRO69558, PRO69559, PRO37676, PRO69560, PRO69561, PRO69562, PRO63204, PRO70019, PRO69564, PRO62830, PRO69565, PRO69566, PRO69567, PRO49675, PRO69568, PRO2013, PRO69569, PRO69570, PRO69571, PRO36403, PRO4676, PRO37657, PRO62097, PRO38081, PRO69572, PRO69573, PRO69574, PRO69883, PRO69576, PRO37584, PRO11603, PRO70020, PRO51695, PRO69579, PRO69580, PRO69581, PRO69582, PRO69583, PRO69584, PRO69585, PRO69586, PRO69587, PRO69588, PRO69589, PRO70021, PRO69590, PRO70022, PRO69592, PRO37029, PRO69593, PRO69594, PRO69595, PRO1207, PRO69596, PRO69597, PRO51139, PRO62545, PRO3615, PRO38036, PRO69598, PRO4701, PRO69599, PRO69600, PRO69601, PRO69887, PRO69603, PRO69604, PRO70023, PRO69606, PRO69607, PRO69608, PRO69609, PRO69610, PRO9902, PRO69611, PRO69612, PRO69613, PRO69614, PRO69615, PRO70024, PRO69616, PRO49619, PRO69617, PRO69618, PRO38040, PRO69619, PRO69620, PRO69621, PRO69622, PRO4401, PRO70025, PRO69625, PRO12025, PRO70026, PRO69627, PRO69628, PRO22637, PRO69629, PRO70027, PRO70028, PRO69632, PRO69634, PRO36857, PRO69893, PRO69635, PRO6180, PRO69637, PRO69638, PRO69639, PRO69640, PRO69641, PRO62766, PRO53782, PRO61472, PRO38179, PRO69642, PRO69643, PRO69644, PRO69645, PRO11608, PRO69646, PRO59825, PRO69647, PRO69648, PR070029, PRO1213, PR070030, PRO50195, PRO69651, PR037538, PR069652, PR059210, PR023374, PR024844, PRO070031, PR069653, PR069654, PR069655, PR069656, PR070032, PR069659, PR069660, PR058054, PRO69661, PR069662, PR069898, PR069664, PR069665, PR069666, PR069667, PR069669, PR069671, PR069672, PR058204, PRO49419, PR069673, PR069674, PR049810, PR070033, PR069676, PR058076, PR069677, PR069678, PR069679, PRO1718, PRO51161, PR069680, PRO59281, PR036102, PR061799, PRO69681, PR069682, PRO69901, PR069684, PR069685, PR069686, PR069687, PR038469, PR069688, PR070034, PR059354, PRO59189, PRO38197, PR069902, PR069690, PRO61569, PR06990 or PRO1970 polypeptide antagonist, wherein said immune response is stimulated.
28. A method of diagnosing an inflammatory immune response in a mammal, said method comprising detecting the level of expression of a gene encoding PR069457, PR069458, PR052268, PR069459, PR062927, PR059136, PR037121, PR069460, PR060475, PRO34451, PR038070, PR023756, PRO10404, PRO69461, PR070006, PR069462, PRO2081, PR070007, PR069463, PR062908, PR069464, PR052804, PR060438, PR069465, PRO37421, PR037596, PRO36124, PR069466, PR060499, PR069467, PRO61824, PR069468, PRO21341, PR038213, PR069469, PR037172, PRO35991, PR036905, PR069470, PRO36451, PR069471, PR037492, PR070008, PR069472, PR069473, PR036996, PR022613, PR069475, PR061755, PR070009, PR069476, PRO4881, PRO12876, PRO70010, PR037534, PRO21928, PR069478, PR069479, PR069480, PRO69481, PR069482, PR069483, PR038642, PR069484, PR066269, PRO1723, PR022297, PR061349, PR069485, PR069486, PR069487, PR036963, PRO23814, PR057980, PRO20128, PRO4551, PR069488, PR039268, PR069489, PR069490, PRO69491, PR069492, PR037713, PR058993, PR069493, PR069494, PR069495, PR070011, PRO62861, PR036640, PR036766, PR069497, PR069498, PR069499, PR069500, PRO69501, PRO70012, PR069503, PRO69474, PR069505, PR069506, PR069507, PRO51301, PR069508, PR069509, PR069510, PR069511, PR051309, PR050578, PRO69512, PR069513, PRO69514, PRO10607, PR061705, PRO49214, PR039648, PRO69515, PR038497, PR029371, PR070013, PRO69516, PR069517, PRO69518, PRO70014, PR069520, PRO69521, PR069522, PR069523, PR060513, PRO2512, PR069524, PRO12569, PR069525, PR069526, PR069527, PR069528, PR069529, PRO12166, PRO2154, PR069530, PRO51916, PR052174, PRO69531, PR069532, PR069533, PR069534, PR054728, PRO70015, PR069536, PR069537, PR037498, PR022175, PR069538, PRO37015, PRO12187, PR069539, PR069880, PRO69541, PR069542, PR069543, PRO70016, PR069545, PRO50197, PR069546, PR069547, PR069548, PR069549, PR069550, PRO69551, PR069552, PR037460, PR042223, PR069553, PR069554, PR069555, PRO61014, PRO59915, PRO37891, PR069556, PRO12875, PR070017, PRO70018, PR04426, PR069558, PR069559, PR037676, PR069560, PRO69561, PR069562, PR063204, PRO70019, PR069564, PR062830, PR069565, PR069566, PR069567, PR049675, PR069568, PRO2013, PR069569, PR069570, PR069571, PR036403, PR04676, PR037657, PR062097, PRO38081, PR069572, PR069573, PR069574, PR069883, PR069576, PR037584, PRO11603, PR070020, PRO51695, PR069579, PR069580, PRO69581, PR069582, PR069583, PR069584, PR069585, PR069586, PR069587, PR069588, PR069589, PRO70021, PR069590, PR070022, PR069592, PR037029, PR069593, PR069594, PR069595, PRO1207, PR069596, PR069597, PR051139, PR062545, PRO3615, PR038036, PR069598, PRO4701, PR069599, PR069600, PRO69601, PR069887, PR069603, PR069604, PR070023, PR069606, PR069607, PR069608, PR069609, PR069610, PR09902, PR069611, PRO69612, PR069613, PRO69614, PRO69615, PR070024, PRO69616, PRO49619, PR069617, PRO69618, PR038040, PRO69619, PR069620, PRO69621, PR069622, PRO4401, PR070025, PR069625, PRO12025, PR070026, PR069627, PR069628, PR022637, PR069629, PR070027, PR070028, PR069632, PR069634, PR036857, PR069893, PR069635, PRO6180, PR069637, PR069638, PR069639, PR069640, PRO69641, PR062766, PR053782, PRO61472, PRO38179, PR069642, PR069643, PR069644, PR069645, PRO11608, PR069646, PR059825, PR069647, PR069648, PR070029, PRO1213, PR070030, PRO50195, PRO69651, PR037538, PR069652, PR059210, PR023374, PR024844, PRO70031, PR069653, PR069654, PR069655, PR069656, PR070032, PR069659, PR069660, PR058054, PRO69661, PR069662, PR069898, PR069664, PR069665, PR069666, PR069667, PR069669, PR069671, PR069672, PR058204, PRO49419, PR069673, PR069674, PR049810, PR070033, PR069676, PR058076, PR069677, PR069678, PR069679, PRO1718, PRO51161, PR069680, PRO59281, PR036102, PR061799, PRO69681, PR069682, PRO69901, PR069684, PR069685, PR069686, PR069687, PR038469, PR069688, PR070034, PR059354, PRO59189, PRO38197, PR069902, PR069690, PRO61569, PR069903 or PRO1970 polypeptide (a) in a test sample of tissue cells obtained from the mammal, and (b) in a control sample of known normal tissue cells of the same cell type, wherein a higher or lower level of expression of said gene in the test sample as compared to the control sample is indicative of the presence of an inflammatory immune response in the mammal from which the test tissue cells were obtained.

## Claims

1. A method of diagnosing an immune related disease or an inflammatory immune response in a mammal, said method comprising:
(1) detecting the level of expression of a gene encoding a PRO51161 polypeptide, wherein the gene (i) has at least 80% nucleic acid sequence identity to the nucleic acid sequence shown as Figure 697 (SEQ ID NO: 697) or (ii) encodes a polypeptide having at least 80% amino acid sequence identity to the amino acid sequence shown as Figure 698 (SEQ ID NO: 698) (a) in a test sample of tissue cells obtained from the mammal, and (b) in a control sample of known normal tissue cells of the same cell type, wherein a higher level of expression of said gene in the test sample as compared to the control sample is indicative of the presence of an immune related disease in the mammal from which the test tissue cells were obtained; or
(2) (a) contacting an anti-PRO51161 antibody to a polypeptide having at least 80% amino acid sequence identity to (i) the polypeptide sequence shown as Figure 698 (SEQ ID NO: 698) or (ii) a polypeptide encoded by the full length coding region of the nucleotide sequence shown as Figure 697 (SEQ ID NO: 697) with a test sample of tissue cells obtained from said mammal and (b) detecting the formation of a complex between the antibody and the polypeptide in the test sample, wherein formation of said complex is indicative of the presence of an immune related disease in the mammal from which the test tissue cells were obtained.
2. The method of claim 1(2), further comprising (a) contacting said antibody with a control sample of known normal tissue cells of the same cell type and (b) detecting the formation of a complex between the antibody and the polypeptide in the control sample, wherein a larger quantity of complexes formed in the test sample is indicative of the presence of an immune related disease in the mammal from which the test tissue cells were obtained.
3. The method of claim 1 or 2, wherein the gene encoding a PRO51161 polypeptide (i) has at least 90% or at least 95% nucleic acid sequence identity to the nucleic acid sequence shown as Figure 697 (SEQ ID NO: 697) or (ii) encodes a polypeptide having at least 90% or at least 95% amino acid sequence identity to the amino acid sequence shown as Figure 698 (SEQ ID NO: 698).
4. The method of claim 1 or 2, wherein the gene encoding a PRO51161 polypeptide (i) has the nucleic acid sequence shown as Figure 697 (SEQ ID NO: 697) or (ii) encodes a polypeptide having the amino acid sequence shown as Figure 698 (SEQ ID NO: 698).
5. The method of claim 1 or 2, wherein the anti-PRO51161 antibody is to a polypeptide (i) having the sequence shown as Figure 698 (SEQ ID NO: 698) or (ii) encoded by the full length coding region of the nucleotide sequence shown as Figure 697 (SEQ ID NO: 697).
6. The method of any one of claims 1 to 5, wherein the immune related disease is a T cell mediated disease.
7. The method of any one of claims 1 to 6, wherein the immune related disease is inflammatory bowel disease (ulcerative colitis, Crohn's disease), systemic lupus erythematosis, rheumatoid arthritis, osteoarthritis, juvenile chronic arthritis, a spondyloarthropathy, systemic sclerosis, an idiopathic inflammatory myopathy, Sjögren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barré syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.
8. The method of claim 1(1), wherein nucleic acid levels are determined by hybridization of nucleic acid obtained from the test and control samples to one or more probes specific for the nucleic acid encoding said PRO polypeptide.
9. The method of claim 8, wherein hybridization is performed under stringent conditions using 50% formamide, 5x SSC, 50 mM sodium phosphate (pH 6.8), 0.1% sodium pyrophosphate, 5x Denhardt's solution, sonicated salmon sperm DNA (50 µg/ml), 0.1 % SDS, and 10% dextran sulfate at 42°C, with washes at 42°C in 0.2x SSC and 50% formamide at 55°C, followed by a wash consisting of 0.1x SSC containing EDTA at 55°C.
10. The method of claim 8, wherein the nucleic acids obtained from the test and normal biological samples are mRNAs.
11. The method of claim 1(1), wherein polypeptide levels are detected with an anti-PRO antibody to a polypeptide (i) having the sequence shown as Figure 698 (SEQ ID NO: 698) or (ii) encoded by the full length coding region of the nucleotide sequence shown as Figure 697 (SEQ ID NO: 697).
12. The method of claim 1, 2, 5 or 11, wherein the anti-PR051161 antibody is a monoclonal antibody, a humanized antibody, an antibody fragment or is detectably labelled.
